# EUROPEAN PATENT SPECIFICATION

(11) **EP 2 869 850 B1**
(45) Date of publication and mention of the grant of the patent: **23.08.2017**
(21) Application number: 13739332.8
(22) Date of filing: 08.07.2013
(51) Int. Cl.: A61K 47/68, A61K 31/551, A61P 35/00

(54) **IMMUNOCONJUGATES COMPRISING ANTI-CD79B ANTIBODIES**
IMMUNKONJUGATE MIT ANTI-CD79B-ANTIKÖRPERN
IMMUNOCONJUGUÉS COMPRENANT DES ANTICORPS ANTI-CD79B

(30) Priority: 09.07.2012 US 201261669270 P
(43) Date of publication of application: 13.05.2015
(73) Proprietor: Genentech, Inc., South San Francisco, CA 94080 (US)
(72) Inventor: POLSON, Andrew, South San Francisco, California 94080 (US); SPENCER, Susan Diane, South San Francisco, California 94080 (US); YU, Shang-Fan, South San Francisco, California 94080 (US); POLAKIS, Paul, South San Francisco, California 94080 (US)
(74) Representative: Woolley, Lindsey Claire
(86) International application number: PCT/US2013/049517
(87) International publication number: WO 2014/011519

(56) References cited:
- US-A1- 2011 256 157
- US-B2- 8 088 378
- D. DORNAN ET AL: "Therapeutic potential of an anti-CD79b antibody-drug conjugate, anti-CD79b-vc-MMAE, for the treatment of non-Hodgkin lymphoma", BLOOD, vol. 114, no. 13, 24 September 2009 (2009-09-24), pages 2721-2729, XP055075268, ISSN: 0006-4971, DOI: 10.1182/blood-2009-02-205500

### SEQUENCE LISTING

<110> Genentech, Inc.
<120> ANTI-CD79B ANTIBODIES AND IMMUNOCONJUGATES
<130> GENE-32633/WO-1/ORD
<150> US 61/669,270
   <151> 2012-07-09
<160> 56
<170> PatentIn version 3.5
<210> 1
   <211> 113
   <212> PRT
   <213> Homo sapiens
<400> 1
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Val Ile Ser Gly Asp Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Phe Asp Tyr Trp Gly Gln Gly Thr Leu Val Thr Val Ser
               100                 105                 110
   Ser
<210> 2
   <211> 108
   <212> PRT
   <213> Homo sapiens
<400> 2
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Glu Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser Leu Pro Trp
                   85                  90                  95
   Thr Phe Gly Gln Gly Thr Lys Val Glu Ile Lys Arg
               100                 105
<210> 3
   <211> 117
   <212> PRT
   <213> Artificial sequence
<220>
   <223> Synthetic
<400> 3
   Glu Val Gln Leu Gln Gln Ser Gly Ala Glu Leu Met Lys Pro Gly Ala
   1               5                   10                  15
   Ser Val Lys Ile Ser Cys Lys Ala Thr Gly Tyr Thr Phe Ser Ser Tyr
               20                  25                  30
   Trp Ile Glu Trp Val Lys Gln Arg Pro Gly His Gly Leu Glu Trp Ile
           35                  40                  45
   Gly Glu Ile Leu Pro Gly Gly Gly Asp Thr Asn Tyr Asn Glu Ile Phe
       50                  55                  60
   Lys Gly Lys Ala Thr Phe Thr Ala Asp Thr Ser Ser Asn Thr Ala Tyr
   65                  70                  75                  80
   Met Gln Leu Ser Ser Leu Thr Ser Glu Asp Ser Ala Val Tyr Tyr Cys
                   85                  90                  95
   Thr Arg Arg Val Pro Val Tyr Phe Asp Tyr Trp Gly Gln Gly Thr Ser
               100                 105                 110
   Val Thr Val Ser Ser
   115
<210> 4
   <211> 112
   <212> PRT
   <213> Artificial sequence
<220>
   <223> Synthetic
<400> 4
   Asp Ile Val Leu Thr Gln Ser Pro Ala Ser Leu Ala Val Ser Leu Gly
   1 5 10 15
   Gln Arg Ala Thr Ile Ser Cys Lys Ala Ser Gln Ser Val Asp Tyr Asp
   20 25 30
   Gly Asp Ser Phe Leu Asn Trp Tyr Gln Gln Lys Pro Gly Gln Pro Pro
   35 40 45
   Lys Leu Phe Ile Tyr Ala Ala Ser Asn Leu Glu Ser Gly Ile Pro Ala
   50 55 60
   Arg Phe Ser Gly Ser Gly Ser Gly Thr Asp Phe Thr Leu Asn Ile His
   65 70 75 80
   Pro Val Glu Glu Glu Asp Ala Ala Thr Tyr Tyr Cys Gln Gln Ser Asn
   85 90 95
   Glu Asp Pro Leu Thr Phe Gly Ala Gly Thr Glu Leu Glu Leu Lys Arg
   100 105 110
<210> 5
   <211> 117
   <212> PRT
   <213> Artificial sequence
<220>
   <223> Synthetic
<400> 5
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Ser Ser Tyr
               20                  25                  30
   Trp Ile Glu Trp Val Arg Gln Ala Pro Gly Lys Gly Leu Glu Trp Val
           35                  40                  45
   Gly Glu Ile Leu Pro Gly Gly Gly Asp Thr Asn Tyr Asn Glu Ile Phe
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Thr Arg Arg Val Pro Val Tyr Phe Asp Tyr Trp Gly Gln Gly Thr Leu
               100                 105                 110
   Val Thr Val Ser Ser
           115
<210> 6
   <211> 112
   <212> PRT
   <213> Artificial sequence
<220>
   <223> Synthetic
<400> 6
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Ser Val Asp Tyr Asp
               20                  25                  30
   Gly Asp Ser Phe Leu Asn Trp Tyr Gln Gln Lys Pro Gly Lys Ala Pro
           35                  40                  45
   Lys Leu Leu Ile Tyr Ala Ala Ser Asn Leu Glu Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln Gln Ser Asn
                   85                  90                  95
   Glu Asp Pro Leu Thr Phe Gly Gln Gly Thr Lys Val Glu Ile Lys Arg
               100                 105                 110
<210> 7
   <211> 117
   <212> PRT
   <213> Artificial sequence
<220>
   <223> Synthetic
<400> 7
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Ser Ser Tyr
               20                  25                  30
   Trp Ile Glu Trp Val Arg Gln Ala Pro Gly Lys Gly Leu Glu Trp Ile
           35                  40                  45
   Gly Glu Ile Leu Pro Gly Gly Gly Asp Thr Asn Tyr Asn Glu Ile Phe
       50                  55                  60
   Lys Gly Arg Ala Thr Phe Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Thr Arg Arg Val Pro Val Tyr Phe Asp Tyr Trp Gly Gln Gly Thr Leu
               100                 105                 110
   Val Thr Val Ser Ser
           115
<210> 8
   <211> 112
   <212> PRT
   <213> Artificial sequence
<220>
   <223> Synthetic
<400> 8
   Asp Ile Gln Leu Thr Gln Ser Pro Ser Ser Leu Ser Ala Ser Val Gly
   1               5                   10                  15
   Asp Arg Val Thr Ile Thr Cys Lys Ala Ser Gln Ser Val Asp Tyr Asp
               20                  25                  30
   Gly Asp Ser Phe Leu Asn Trp Tyr Gln Gln Lys Pro Gly Lys Ala Pro
           35                  40                  45
   Lys Leu Leu Ile Tyr Ala Ala Ser Asn Leu Glu Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln Gln Ser Asn
                   85                  90                  95
   Glu Asp Pro Leu Thr Phe Gly Gln Gly Thr Lys Val Glu Ile Lys Arg
               100                 105                 110
<210> 9
   <211> 117
   <212> PRT
   <213> Artificial sequence
<220>
   <223> Synthetic
<400> 9
<210> 10
   <211> 112
   <212> PRT
   <213> Artificial sequence
<220>
   <223> Synthetic
<400> 10
   Asp Ile Gln Leu Thr Gln Ser Pro Ser Ser Leu Ser Ala Ser Val Gly
   1               5                   10                  15
   Asp Arg Val Thr Ile Thr Cys Lys Ala Ser Gln Ser Val Asp Tyr Asp
               20                  25                  30
   Gly Asp Ser Phe Leu Asn Trp Tyr Gln Gln Lys Pro Gly Lys Ala Pro
           35                  40                  45
   Lys Leu Leu Ile Tyr Ala Ala Ser Asn Leu Glu Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln Gln Ser Asn
                   85                  90                  95
   Glu Asp Pro Leu Thr Phe Gly Gln Gly Thr Lys Val Glu Ile Lys Arg
               100                 105                 110
<210> 11
   <211> 117
   <212> PRT
   <213> Artificial sequence
<220>
   <223> Synthetic
<400> 11
<210> 12
   <211> 112
   <212> PRT
   <213> Artificial sequence
<220>
   <223> Synthetic
<400> 12
   Asp Ile Gln Leu Thr Gln Ser Pro Ser Ser Leu Ser Ala Ser Val Gly
   1               5                   10                  15
   Asp Arg Val Thr Ile Thr Cys Lys Ala Ser Gln Ser Val Asp Tyr Glu
               20                  25                  30
   Gly Asp Ser Phe Leu Asn Trp Tyr Gln Gln Lys Pro Gly Lys Ala Pro
           35                  40                  45
   Lys Leu Leu Ile Tyr Ala Ala Ser Asn Leu Glu Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln Gln Ser Asn
                   85                  90                  95
   Glu Asp Pro Leu Thr Phe Gly Gln Gly Thr Lys Val Glu Ile Lys Arg
               100                 105                 110
<210> 13
   <211> 117
   <212> PRT
   <213> Artificial sequence
<220>
   <223> Synthetic
<400> 13
<210> 14
   <211> 112
   <212> PRT
   <213> Artificial sequence
<220>
   <223> Synthetic
<400> 14
   Asp Ile Gln Leu Thr Gln Ser Pro Ser Ser Leu Ser Ala Ser Val Gly
   1 5 10 15
   Asp Arg Val Thr Ile Thr Cys Lys Ala Ser Gln Ser Val Asp Tyr Ser
   20 25 30
   Gly Asp Ser Phe Leu Asn Trp Tyr Gln Gln Lys Pro Gly Lys Ala Pro
   35 40 45
   Lys Leu Phe Ile Tyr Ala Ala Ser Asn Leu Glu Ser Gly Val Pro Ser
   50 55 60
   Arg Phe Ser Gly Ser Gly Ser Gly Thr Asp Phe Thr Leu Thr Ile Ser
   65 70 75 80
   Ser Leu Gln Pro Glu Asp Phe Ala Thr Tyr Tyr Cys Gln Gln Ser Asn
   85 90 95
   Glu Asp Pro Leu Thr Phe Gly Gln Gly Thr Lys Val Glu Ile Lys Arg
   100 105 110
<210> 15
   <211> 10
   <212> PRT
   <213> Artificial sequence
<220>
   <223> Synthetic
<400> 15
   Gly Tyr Thr Phe Ser Ser Tyr Trp Ile Glu
   1 5 10
<210> 16
   <211> 18
   <212> PRT
   <213> Artificial sequence
<220>
   <223> Synthetic
<400> 16
   Gly Glu Ile Leu Pro Gly Gly Gly Asp Thr Asn Tyr Asn Glu Ile Phe
   1 5 10 15
   Lys Gly
<210> 17
   <211> 10
   <212> PRT
   <213> Artificial sequence
<220>
   <223> Synthetic
<400> 17
   Thr Arg Arg Val Pro Val Tyr Phe Asp Tyr
   1 5 10
<210> 18
   <211> 15
   <212> PRT
   <213> Artificial sequence
<220>
   <223> Synthetic
<400> 18
   Lys Ala Ser Gln Ser Val Asp Tyr Asp Gly Asp Ser Phe Leu Asn
   1 5 10 15
<210> 19
   <211> 7
   <212> PRT
   <213> Artificial sequence
<220>
   <223> Synthetic
<400> 19
   Ala Ala Ser Asn Leu Glu Ser
   1 5
<210> 20
   <211> 9
   <212> PRT
   <213> Artificial sequence
<220>
   <223> Synthetic
<400> 20
   Gln Gln Ser Asn Glu Asp Pro Leu Thr
   1 5
<210> 21
   <211> 10
   <212> PRT
   <213> Artificial sequence
<220>
   <223> Synthetic
<400> 21
   Gly Tyr Thr Phe Ser Ser Tyr Trp Ile Glu
   1 5 10
<210> 22
   <211> 18
   <212> PRT
   <213> Artificial sequence
<220>
   <223> Synthetic
<400> 22
   Gly Glu Ile Leu Pro Gly Gly Gly Asp Thr Asn Tyr Asn Glu Ile Phe
   1 5 10 15
   Lys Gly
<210> 23
   <211> 10
   <212> PRT
   <213> Artificial sequence
<220>
   <223> Synthetic
<400> 23
   Thr Arg Arg Val Pro Ile Arg Leu Asp Tyr
   1 5 10
<210> 24
   <211> 15
   <212> PRT
   <213> Artificial sequence
<220>
   <223> Synthetic
<400> 24
   Lys Ala Ser Gln Ser Val Asp Tyr Glu Gly Asp Ser Phe Leu Asn
   1 5 10 15
<210> 25
   <211> 7
   <212> PRT
   <213> Artificial sequence
<220>
   <223> Synthetic
<400> 25
   Ala Ala Ser Asn Leu Glu Ser
   1 5
<210> 26
   <211> 9
   <212> PRT
   <213> Artificial sequence
<220>
   <223> Synthetic
<400> 26
   Gln Gln Ser Asn Glu Asp Pro Leu Thr
   1 5
<210> 27
   <211> 25
   <212> PRT
   <213> Artificial sequence
<220>
   <223> Synthetic
<400> 27
<210> 28
   <211> 13
   <212> PRT
   <213> Artificial sequence
<220>
   <223> Synthetic
<400> 28
   Trp Val Arg Gln Ala Pro Gly Lys Gly Leu Glu Trp Ile
   1 5 10
<210> 29
   <211> 30
   <212> PRT
   <213> Artificial sequence
<220>
   <223> Synthetic
<400> 29
   Arg Ala Thr Phe Ser Ala Asp Thr Ser Lys Asn Thr Ala Tyr Leu Gln
   1               5                   10                  15
   Met Asn Ser Leu Arg Ala Glu Asp Thr Ala Val Tyr Tyr Cys
               20                  25                  30
<210> 30
   <211> 11
   <212> PRT
   <213> Artificial sequence
<220>
   <223> Synthetic
<400> 30
   Trp Gly Gln Gly Thr Leu Val Thr Val Ser Ser
   1               5                   10
<210> 31
   <211> 23
   <212> PRT
   <213> Artificial sequence
<220>
   <223> Synthetic
<400> 31
   Asp Ile Gln Leu Thr Gln Ser Pro Ser Ser Leu Ser Ala Ser Val Gly
   1               5                   10                  15
   Asp Arg Val Thr Ile Thr Cys
               20
<210> 32
   <211> 15
   <212> PRT
   <213> Artificial sequence
<220>
   <223> Synthetic
<400> 32
   Trp Tyr Gln Gln Lys Pro Gly Lys Ala Pro Lys Leu Leu Ile Tyr
   1               5                   10                  15
<210> 33
   <211> 32
   <212> PRT
   <213> Artificial sequence
<220>
   <223> Synthetic
<400> 33
   Gly Val Pro Ser Arg Phe Ser Gly Ser Gly Ser Gly Thr Asp Phe Thr
   1               5                   10                  15
   Leu Thr Ile Ser Ser Leu Gln Pro Glu Asp Phe Ala Thr Tyr Tyr Cys
               20                  25                  30
<210> 34
   <211> 11
   <212> PRT
   <213> Artificial sequence
<220>
   <223> Synthetic
<400> 34
   Phe Gly Gln Gly Thr Lys Val Glu Ile Lys Arg
   1               5                   10
<210> 35
   <211> 15
   <212> PRT
   <213> Artificial sequence
<220>
   <223> Synthetic
<400> 35
   Lys Ala Ser Gln Ser Val Asp Tyr Ser Gly Asp Ser Phe Leu Asn
   1               5                   10                  15
<210> 36
   <211> 15
   <212> PRT
   <213> Artificial sequence
<220>
   <223> Synthetic
<400> 36
   Trp Tyr Gln Gln Lys Pro Gly Lys Ala Pro Lys Leu Leu Phe Tyr
   1               5                   10                  15
<210> 37
   <211> 218
   <212> PRT
   <213> Artificial sequence
<220>
   <223> Synthetic
<400> 37
   Asp Ile Gln Leu Thr Gln Ser Pro Ser Ser Leu Ser Ala Ser Val Gly
   1               5                   10                  15
   Asp Arg Val Thr Ile Thr Cys Lys Ala Ser Gln Ser Val Asp Tyr Glu
               20                  25                  30
   Gly Asp Ser Phe Leu Asn Trp Tyr Gln Gln Lys Pro Gly Lys Ala Pro
           35                  40                  45
   Lys Leu Leu Ile Tyr Ala Ala Ser Asn Leu Glu Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln Gln Ser Asn
                   85                  90                  95
   Glu Asp Pro Leu Thr Phe Gly Gln Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 38
   <211> 446
   <212> PRT
   <213> Artificial sequence
<220>
   <223> Synthetic
<400> 38
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Ser Ser Tyr
               20                  25                  30
   Trp Ile Glu Trp Val Arg Gln Ala Pro Gly Lys Gly Leu Glu Trp Ile
           35                  40                  45
   Gly Glu Ile Leu Pro Gly Gly Gly Asp Thr Asn Tyr Asn Glu Ile Phe
       50                  55                  60
   Lys Gly Arg Ala Thr Phe Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Thr Arg Arg Val Pro Ile Arg Leu Asp Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly
   435 440 445
<210> 39
   <211> 446
   <212> PRT
   <213> Artificial sequence
<220>
   <223> Synthetic
<400> 39
   Glu Val Gln Leu Val Glu Ser Gly Gly Gly Leu Val Gln Pro Gly Gly
   1 5 10 15
   Ser Leu Arg Leu Ser Cys Ala Ala Ser Gly Tyr Thr Phe Ser Ser Tyr
   20 25 30
   Trp Ile Glu Trp Val Arg Gln Ala Pro Gly Lys Gly Leu Glu Trp Ile
   35 40 45
   Gly Glu Ile Leu Pro Gly Gly Gly Asp Thr Asn Tyr Asn Glu Ile Phe
   50 55 60
   Lys Gly Arg Ala Thr Phe Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Thr Arg Arg Val Pro Ile Arg Leu Asp Tyr Trp Gly Gln Gly Thr Leu
   100 105 110
   Val Thr Val Ser Ser Cys Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly
   435 440 445
<210> 40
   <211> 229
   <212> PRT
   <213> Homo sapiens
<400> 40
   Met Ala Arg Leu Ala Leu Ser Pro Val Pro Ser His Trp Met Val Ala
   1 5 10 15
   Leu Leu Leu Leu Leu Ser Ala Glu Pro Val Pro Ala Ala Arg Ser Glu
   20 25 30
   Asp Arg Tyr Arg Asn Pro Lys Gly Ser Ala Cys Ser Arg Ile Trp Gln
   35 40 45
   Ser Pro Arg Phe Ile Ala Arg Lys Arg Gly Phe Thr Val Lys Met His
   50 55 60
   Cys Tyr Met Asn Ser Ala Ser Gly Asn Val Ser Trp Leu Trp Lys Gln
   65 70 75 80
   Glu Met Asp Glu Asn Pro Gln Gln Leu Lys Leu Glu Lys Gly Arg Met
   85 90 95
   Glu Glu Ser Gln Asn Glu Ser Leu Ala Thr Leu Thr Ile Gln Gly Ile
   100 105 110
   Arg Phe Glu Asp Asn Gly Ile Tyr Phe Cys Gln Gln Lys Cys Asn Asn
   115 120 125
   Thr Ser Glu Val Tyr Gln Gly Cys Gly Thr Glu Leu Arg Val Met Gly
   130 135 140
   Phe Ser Thr Leu Ala Gln Leu Lys Gln Arg Asn Thr Leu Lys Asp Gly
   145 150 155 160
   Ile Ile Met Ile Gln Thr Leu Leu Ile Ile Leu Phe Ile Ile Val Pro
   165 170 175
   Ile Phe Leu Leu Leu Asp Lys Asp Asp Ser Lys Ala Gly Met Glu Glu
   180 185 190
   Asp His Thr Tyr Glu Gly Leu Asp Ile Asp Gln Thr Ala Thr Tyr Glu
   195 200 205
   Asp Ile Val Thr Leu Arg Thr Gly Glu Val Lys Trp Ser Val Gly Glu
   210 215 220
   His Pro Gly Gln Glu
   225
<210> 41
   <211> 201
   <212> PRT
   <213> Homo sapiens
<400> 41
   Ala Arg Ser Glu Asp Arg Tyr Arg Asn Pro Lys Gly Ser Ala Cys Ser
   1 5 10 15
   Arg Ile Trp Gln Ser Pro Arg Phe Ile Ala Arg Lys Arg Gly Phe Thr
   20 25 30
   Val Lys Met His Cys Tyr Met Asn Ser Ala Ser Gly Asn Val Ser Trp
   35 40 45
   Leu Trp Lys Gln Glu Met Asp Glu Asn Pro Gln Gln Leu Lys Leu Glu
   50 55 60
   Lys Gly Arg Met Glu Glu Ser Gln Asn Glu Ser Leu Ala Thr Leu Thr
   65 70 75 80
   Ile Gln Gly Ile Arg Phe Glu Asp Asn Gly Ile Tyr Phe Cys Gln Gln
   85 90 95
   Lys Cys Asn Asn Thr Ser Glu Val Tyr Gln Gly Cys Gly Thr Glu Leu
   100 105 110
   Arg Val Met Gly Phe Ser Thr Leu Ala Gln Leu Lys Gln Arg Asn Thr
   115 120 125
   Leu Lys Asp Gly Ile Ile Met Ile Gln Thr Leu Leu Ile Ile Leu Phe
   130 135 140
   Ile Ile Val Pro Ile Phe Leu Leu Leu Asp Lys Asp Asp Ser Lys Ala
   145 150 155 160
   Gly Met Glu Glu Asp His Thr Tyr Glu Gly Leu Asp Ile Asp Gln Thr
   165 170 175
   Ala Thr Tyr Glu Asp Ile Val Thr Leu Arg Thr Gly Glu Val Lys Trp
   180 185 190
   Ser Val Gly Glu His Pro Gly Gln Glu
   195 200
<210> 42
   <211> 10
   <212> PRT
   <213> Artificial sequence
<220>
   <223> Synthetic
<400> 42
   Gly Tyr Glu Phe Ser Arg Ser Trp Met Asn
   1 5 10
<210> 43
   <211> 18
   <212> PRT
   <213> Artificial sequence
<220>
   <223> Synthetic
<400> 43
   Gly Arg Ile Tyr Pro Gly Asp Gly Asp Thr Asn Tyr Ser Gly Lys Phe
   1 5 10 15
   Lys Gly
<210> 44
   <211> 11
   <212> PRT
   <213> Artificial sequence
<220>
   <223> Synthetic
<400> 44
   Asp Gly Ser Ser Trp Asp Trp Tyr Phe Asp Val
   1 5 10
<210> 45
   <211> 16
   <212> PRT
   <213> Artificial sequence
<220>
   <223> Synthetic
<400> 45
   Arg Ser Ser Gln Ser Ile Val His Ser Val Gly Asn Thr Phe Leu Glu
   1               5                   10                  15
<210> 46
   <211> 7
   <212> PRT
   <213> Artificial sequence
<220>
   <223> Synthetic
<400> 46
   Lys Val Ser Asn Arg Phe Ser
   1               5
<210> 47
   <211> 9
   <212> PRT
   <213> Artificial sequence
<220>
   <223> Synthetic
<400> 47
   Phe Gln Gly Ser Gln Phe Pro Tyr Thr
   1               5
<210> 48
   <211> 120
   <212> PRT
   <213> Artificial sequence
<220>
   <223> Synthetic
<400> 48
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Glu Phe Ser Arg Ser
               20                  25                  30
   Trp Met Asn Trp Val Arg Gln Ala Pro Gly Lys Gly Leu Glu Trp Val
           35                  40                  45
   Gly Arg Ile Tyr Pro Gly Asp Gly Asp Thr Asn Tyr Ser Gly Lys Phe
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ser Ser Trp Asp Trp Tyr Phe Asp Val Trp Gly Gln
               100                 105                 110
   Gly Thr Leu Val Thr Val Ser Ser
           115                 120
<210> 49
   <211> 112
   <212> PRT
   <213> Artificial sequence
<220>
   <223> Synthetic
<400> 49
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Ser Ile Val His Ser
               20                  25                  30
   Val Gly Asn Thr Phe Leu Glu Trp Tyr Gln Gln Lys Pro Gly Lys Ala
           35                  40                  45
   Pro Lys Leu Leu Ile Tyr Lys Val Ser Asn Arg Phe Ser Gly Val Pro
       50                  55                  60
   Ser Arg Phe Ser Gly Ser Gly Ser Gly Thr Asp Phe Thr Leu Thr Ile
   65                  70                  75                  80
   Ser Ser Leu Gln Pro Glu Asp Phe Ala Thr Tyr Tyr Cys Phe Gln Gly
                   85                  90                  95
   Ser Gln Phe Pro Tyr Thr Phe Gly Gln Gly Thr Lys Val Glu Ile Lys
               100                 105                 110
<210> 50
   <211> 450
   <212> PRT
   <213> Artificial sequence
<220>
   <223> Synthetic
<400> 50
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Glu Phe Ser Arg Ser
               20                  25                  30
   Trp Met Asn Trp Val Arg Gln Ala Pro Gly Lys Gly Leu Glu Trp Val
           35                  40                  45
   Gly Arg Ile Tyr Pro Gly Asp Gly Asp Thr Asn Tyr Ser Gly Lys Phe
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ser Ser Trp Asp Trp Tyr Phe Asp Val Trp Gly Gln
               100                 105                 110
   Gly Thr Leu Val Thr Val Ser Ser Cys Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 51
   <211> 219
   <212> PRT
   <213> Artificial sequence
<220>
   <223> Synthetic
<400> 51
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Ser Ile Val His Ser
               20                  25                  30
   Val Gly Asn Thr Phe Leu Glu Trp Tyr Gln Gln Lys Pro Gly Lys Ala
           35                  40                  45
   Pro Lys Leu Leu Ile Tyr Lys Val Ser Asn Arg Phe Ser Gly Val Pro
       50                  55                  60
   Ser Arg Phe Ser Gly Ser Gly Ser Gly Thr Asp Phe Thr Leu Thr Ile
   65                  70                  75                  80
   Ser Ser Leu Gln Pro Glu Asp Phe Ala Thr Tyr Tyr Cys Phe Gln Gly
                   85                  90                  95
   Ser Gln Phe Pro Tyr Thr Phe Gly Gln Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 52
   <211> 450
   <212> PRT
   <213> Artificial sequence
<220>
   <223> Synthetic
<400> 52
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Glu Phe Ser Arg Ser
               20                  25                  30
   Trp Met Asn Trp Val Arg Gln Ala Pro Gly Lys Gly Leu Glu Trp Val
           35                  40                  45
   Gly Arg Ile Tyr Pro Gly Asp Gly Asp Thr Asn Tyr Ser Gly Lys Phe
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ser Ser Trp Asp Trp Tyr Phe Asp Val Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Cys Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 53
   <211> 219
   <212> PRT
   <213> Artificial sequence
<220>
   <223> Synthetic
<400> 53
   Asp Ile Gln Met Thr Gln Ser Pro Ser Ser Leu Ser Ala Ser Val Gly
   1               5                   10                  15
   Asp Arg Val Thr Ile Thr Cys Arg Ser Ser Gln Ser Ile Val His Ser
               20                  25                  30
   Val Gly Asn Thr Phe Leu Glu Trp Tyr Gln Gln Lys Pro Gly Lys Ala
           35                  40                  45
   Pro Lys Leu Leu Ile Tyr Lys Val Ser Asn Arg Phe Ser Gly Val Pro
       50                  55                  60
   Ser Arg Phe Ser Gly Ser Gly Ser Gly Thr Asp Phe Thr Leu Thr Ile
   65                  70                  75                  80
   Ser Ser Leu Gln Pro Glu Asp Phe Ala Thr Tyr Tyr Cys Phe Gln Gly
                   85                  90                  95
   Ser Gln Phe Pro Tyr Thr Phe Gly Gln Gly Thr Lys Val Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Cys Thr Lys Ser Phe Asn Arg Gly Glu Cys
       210                 215
<210> 54
   <211> 218
   <212> PRT
   <213> Artificial sequence
<220>
   <223> Synthetic
<400> 54
   Asp Ile Gln Leu Thr Gln Ser Pro Ser Ser Leu Ser Ala Ser Val Gly
   1               5                   10                  15
   Asp Arg Val Thr Ile Thr Cys Lys Ala Ser Gln Ser Val Asp Tyr Glu
               20                  25                  30
   Gly Asp Ser Phe Leu Asn Trp Tyr Gln Gln Lys Pro Gly Lys Ala Pro
           35                  40                  45
   Lys Leu Leu Ile Tyr Ala Ala Ser Asn Leu Glu Ser Gly Val Pro Ser
       50                  55                  60
   Arg Phe Ser Gly Ser Gly Ser Gly Thr Asp Phe Thr Leu Thr Ile Ser
   65                  70                  75                  80
   Ser Leu Gln Pro Glu Asp Phe Ala Thr Tyr Tyr Cys Gln Gln Ser Asn
                   85                  90                  95
   Glu Asp Pro Leu Thr Phe Gly Gln Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Cys Thr Lys Ser Phe Asn Arg Gly Glu Cys
       210                 215
<210> 55
   <211> 447
   <212> PRT
   <213> Artificial sequence
<220>
   <223> Synthetic
<400> 55
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Thr Phe Ser Ser Tyr
               20                  25                  30
   Trp Ile Glu Trp Val Arg Gln Ala Pro Gly Lys Gly Leu Glu Trp Ile
           35                  40                  45
   Gly Glu Ile Leu Pro Gly Gly Gly Asp Thr Asn Tyr Asn Glu Ile Phe
       50                  55                  60
   Lys Gly Arg Ala Thr Phe Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Thr Arg Arg Val Pro Ile Arg Leu Asp Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Lys Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Asn Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Cys
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
               420                 425                 430
   His Asn His Tyr Thr Gln Lys Ser Leu Ser Leu Ser Pro Gly Lys
           435                 440                 445
<210> 56
   <211> 450
   <212> PRT
   <213> Artificial sequence
<220>
   <223> Synthetic
<400> 56
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Glu Phe Ser Arg Ser
               20                  25                  30
   Trp Met Asn Trp Val Arg Gln Ala Pro Gly Lys Gly Leu Glu Trp Val
           35                  40                  45
   Gly Arg Ile Tyr Pro Gly Asp Gly Asp Thr Asn Tyr Ser Gly Lys Phe
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Asp Gly Ser Ser Trp Asp Trp Tyr Phe Asp Val Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450

## Description

### FIELD OF THE INVENTION

The present invention relates to immunoconjugates comprising anti-CD79b antibodies and methods of using the same.

### BACKGROUND

CD79 is the signaling component of the B-cell receptor consisting of a covalent heterodimer containing CD79a (Igα, mb-1) and CD79b (Igβ, B29). CD79a and CD79b each contain an extracellular immunoglobulin (Ig) domain, a transmembrane domain, and an intracellular signaling domain, an immunoreceptor tyrosine-based activation motif (ITAM) domain. CD79 is expressed on B cells and, for example, in Non-Hodgkin's Lymphoma cells (NHLs) (Cabezudo et al., Haematologica, 84:413-418 (1999); D'Arena et al., Am. J. Hematol., 64: 275-281 (2000); Olejniczak et al., Immunol. Invest., 35: 93-114 (2006)). CD79a and CD79b and sIg are all required for surface expression of the CD79 (Matsuuchi et al., Curr. Opin. Immunol., 13(3): 270-7)). The average surface expression of CD79b on NHLs is similar to that on normal B-cells, but with a greater range (Matsuuchi et al., Curr. Opin. Immunol., 13(3): 270-7 (2001)).

There is a need in the art for agents that target CD79b for the diagnosis and treatment of CD79b-associated conditions, such as cancer. The invention fulfills that need and provides other benefits.

### SUMMARY

The invention provides anti-CD79b antibodies and immunoconjugates and methods of using the same. Subject-matter which is not encompassed by the scope of the claims does not form part of the presently claimed invention.

An immunoconjugate comprising an antibody that binds CD79b covalently attached to a cytotoxic agent is provided, wherein the cytotoxic agent is a pyrrolobenzodiazepine, and wherein the antibody that binds CD79b comprises (i) HVR-H1 comprising the amino acid sequence of SEQ ID NO: 21, (ii) HVR-H2 comprising the amino acid sequence of SEQ ID NO: 22, and (iii) HVR-H3 comprising the amino acid sequence of SEQ ID NO: 23, (iv) HVR-L1 comprising an amino acid sequence selected from SEQ ID NOs: 18, 24, and 35, (v) HVR-L2 comprising the amino acid sequence of SEQ ID NO: 25, and (vi) HVR-L3 comprising the amino acid sequence of SEQ ID NO: 26. In some embodiments, the antibody comprises HVR-L1 comprising the amino acid sequence of SEQ ID NO: 24.

In some embodiments, the antibody comprises: a) a VH sequence having at least 95% sequence identity to the amino acid sequence of SEQ ID NO: 11; or b) a VL sequence having at least 95% sequence identity to the amino acid sequence of SEQ ID NO: 12; or c) a VH sequence as in (a) and a VL sequence as in (b). In some embodiments, the antibody comprises a VH sequence having an amino acid sequence selected from SEQ ID NOs: 9, 11, and 13. In some embodiments, the antibody comprises a VH sequence having the amino acid sequence of SEQ ID NO: 11. In some embodiments, the antibody comprises a VL sequence having an amino acid sequence selected from SEQ ID NOs: 8, 10, 12, and 14. In some embodiments, the antibody comprises a VL sequence having the amino acid sequence of SEQ ID NO: 12. In some embodiments, the antibody is an IgG1, IgG2a or IgG2b antibody.

In some embodiments, an immunoconjugate comprising an antibody that binds CD79b covalently attached to a cytotoxic agent is provided, wherein the antibody comprises (a) a VH sequence having the amino acid sequence of SEQ ID NO: 11 and a VL sequence having the amino acid sequence of SEQ ID NO: 12, and wherein the cytotoxic agent is a pyrrolobenzodiazepine.

In some embodiments, the immunoconjugate has the formula Ab-(L-D)p, wherein: (a) Ab is the antibody; (b) L is a linker; (c) D is the cytotoxic agent; and (d) p ranges from 1-8.

In some such embodiments, D is a pyrrolobenzodiazepine of Formula A: 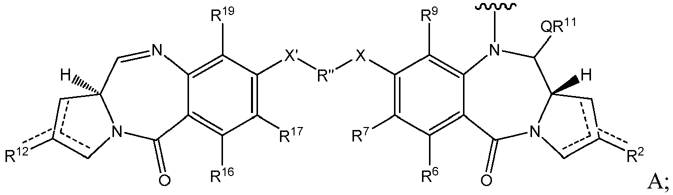 wherein the dotted lines indicate the optional presence of a double bond between C1 and C2 or C2 and C3;
R² is independently selected from H, OH, =O, =CH₂, CN, R, OR, =CH-R^{D}, =C(R^{D})₂, O-SO₂-R, CO₂R and COR, and optionally further selected from halo or dihalo, wherein R^{D} is independently selected from R, CO₂R, COR, CHO, CO₂H, and halo;
R⁶ and R⁹ are independently selected from H, R, OH, OR, SH, SR, NH₂, NHR, NRR', NO₂, Me₃Sn and halo;
R⁷ is independently selected from H, R, OH, OR, SH, SR, NH₂, NHR, NRR', NO₂, Me₃Sn and halo;
Q is independently selected from O, S and NH;
R¹¹ is either H, or R or, where Q is O, SO₃M, where M is a metal cation;
R and R' are each independently selected from optionally substituted C₁₋₈ alkyl, C₃₋₈ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
R¹², R¹⁶, R¹⁹ and R¹⁷ are as defined for R², R⁶, R⁹ and R⁷ respectively;
R" is a C₃₋₁₂ alkylene group, which chain may be interrupted by one or more heteroatoms and/or aromatic rings that are optionally substituted; and
X and X' are independently selected from O, S and N(H).

In some embodiments, D has the structure: 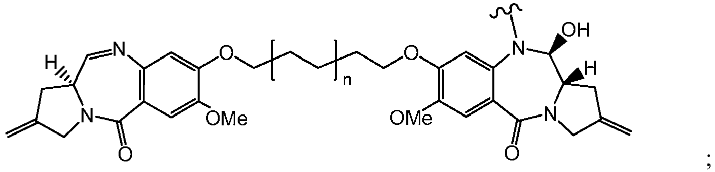 wherein n is 0 or 1.

In some embodiments, D has a structure selected from: 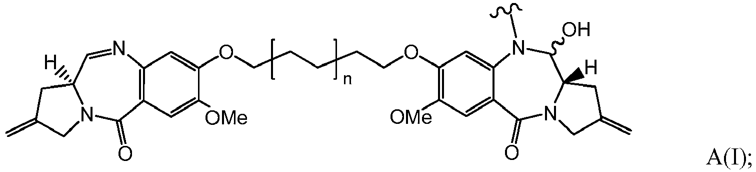 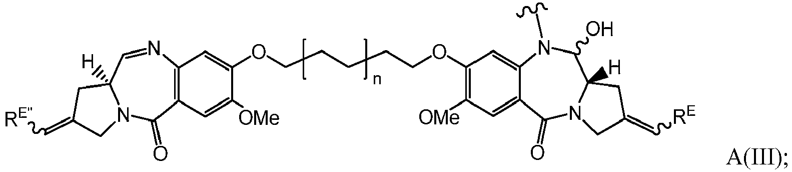 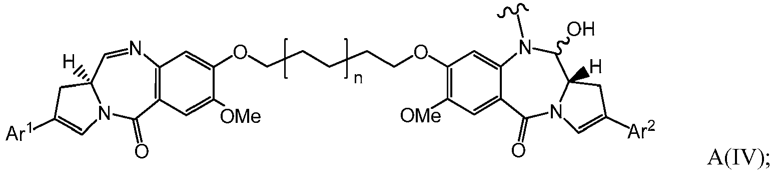 and 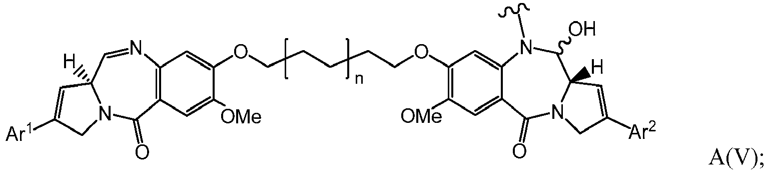
wherein R^{E} and R^{E"} are each independently selected from H or R^{D}, wherein R^{D} is independently selected from R, CO₂R COR, CHO, CO₂H, and halo;
wherein Ar¹ and Ar² are each independently optionally substituted C₅₋₂₀ aryl; and wherein n is 0 or 1.

In some embodiments, D is a pyrrolobenzodiazepine of Formula B: 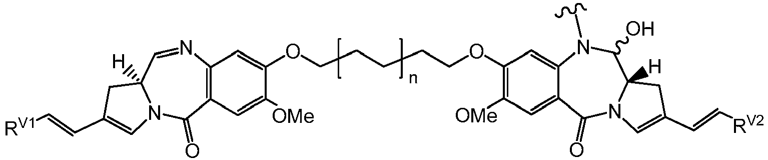 wherein the horizontal wavy line indicates the covalent attachement site to the linker;
R^{V1} and R^{V2} are independently selected from H, methyl, ethyl, phenyl, fluoro-substituted phenyl, and C₅₋₆ heterocyclyl; and
n is 0 or 1.

In some embodiments, the immunoconjugate comprises a linker that is cleavable by a protease. In some such embodiments, the linker comprises a val-cit dipeptide. The linker may comprise a Phe-homoLys dipeptide. In some embodiments, the immunoconjuge has the formula: 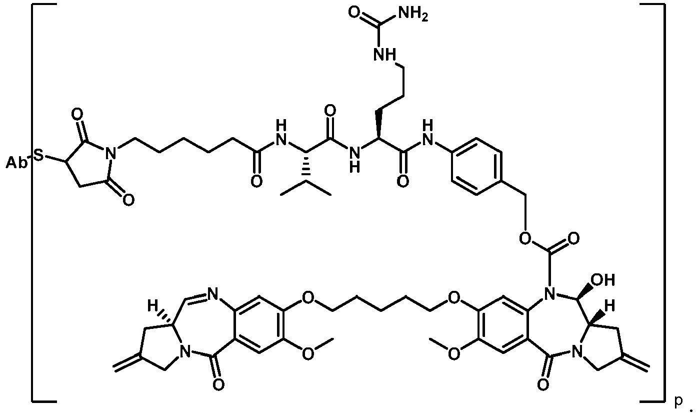 In some embodiments, p ranges from 1-3.

In some embodiments, an immunoconjugate is provided, wherein the immunoconjugate has the formula: 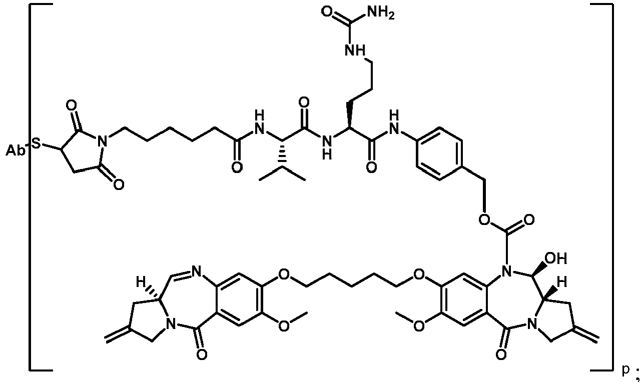 wherein Ab is an antibody comprising (i) HVR-H1 comprising the amino acid sequence of SEQ ID NO: 21, (ii) HVR-H2 comprising the amino acid sequence of SEQ ID NO: 22, (iii) HVR-H3 comprising the amino acid sequence of SEQ ID NO: 23, (iv) HVR-L1 comprising the amino acid sequence of SEQ ID NO: 24, (v) HVR-L2 comprising the amino acid sequence of SEQ ID NO: 25, and (vi) HVR-L3 comprising the amino acid sequence of SEQ ID NO: 26; and wherein p ranges from 1 to 3. In some embodiments, the antibody comprises a VH sequence of SEQ ID NO: 11 and a VL sequence of SEQ ID NO: 12. In some embodiments, the antibody comprises a heavy chain of SEQ ID NO: 39 and a light chain of SEQ ID NO: 37.

In any of the embodiments discussed herein, the antibody may be a monoclonal antibody. In some embodiments, the antibody may be a human, humanized, or chimeric antibody. In some embodiments, the antibody is an antibody fragment that binds CD79b. in some embodiments, the antibody binds human CD79b. In some such embodiments, human CD79b has the sequence of SEQ ID NO: 40 or SEQ ID NO: 41.

In some embodiments, pharmaceutical formulations are provided, wherein the formulation comprises an immunoconjugate described herein and a pharmaceutically acceptable carrier. In some embodiments, the pharmaceutical formulation comprises an additional therapeutic agent.

In some embodiments, the immunoconjugates according to the claims are for use in methods of treating an individual with a CD79b-positive cancer are provided. In some embodiments, the method may comprise administering to the individual an effective amount of the immunoconjugate described herein. In some embodiments, the CD79b-positive cancer is selected from lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma. In some embodiments, the method may further comprise administering an additional therapeutic agent to the individual. In some such embodiments, the additional therapeutic agent comprises an antibody that binds CD22. In some embodiments, the additional therapeutic agent is an immunoconjugate comprising an antibody that binds CD22 covalently attached to a cytotoxic agent.

In some embodiments, the immunoconjugates according to the claims are for use in a method of inhibiting proliferation of a CD79b-positive cell is provided. In some such embodiments, the method comprises exposing the cell to the immunoconjugate described herein under conditions permissive for binding of the immunoconjugate to CD79b on the surface of the cell, thereby inhibiting proliferation of the cell. In some embodiments, the cell is a neoplastic B cell. In some embodiments, the cell is a lymphoma cell.

### BRIEF DESCRIPTION OF THE FIGURES

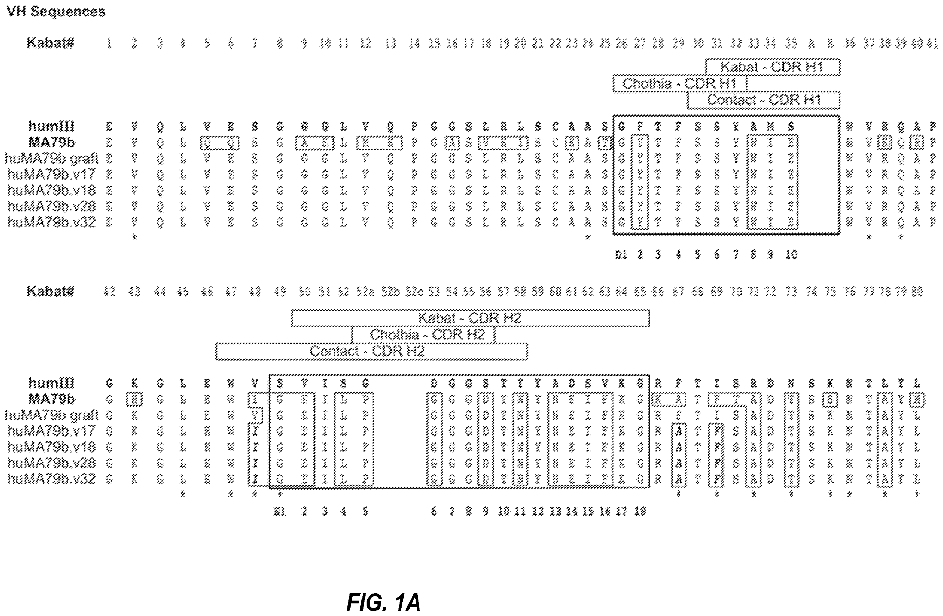

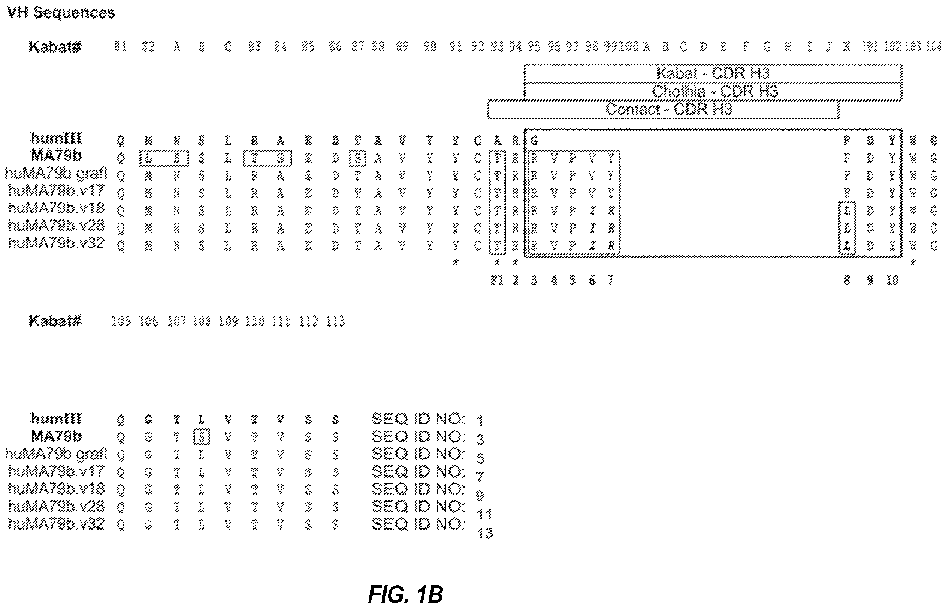

**Figures 1A-1B** show the amino acid sequence of the heavy chain variable region of murine anti-CD79b antibody MA79b aligned with the humanized MA79b graft and humanized versions 17, 18, 28, and 32 (huMA79b graft, huMA79bv17, huMA79bv18, huMA79bv28, and huMA79bv32, respectively), and aligned with the human subgroup III sequence. The HVRs are boxed (HVR-H1, HVR-H2, HVR-H3). The sequences bracketing the HVRs are the framework sequences (FR-H1 to FR-H4). The sequences are numbered according to Kabat numbering. The Kabat, Chothia, and contact CDRs are indicated about the boxed HVRs.

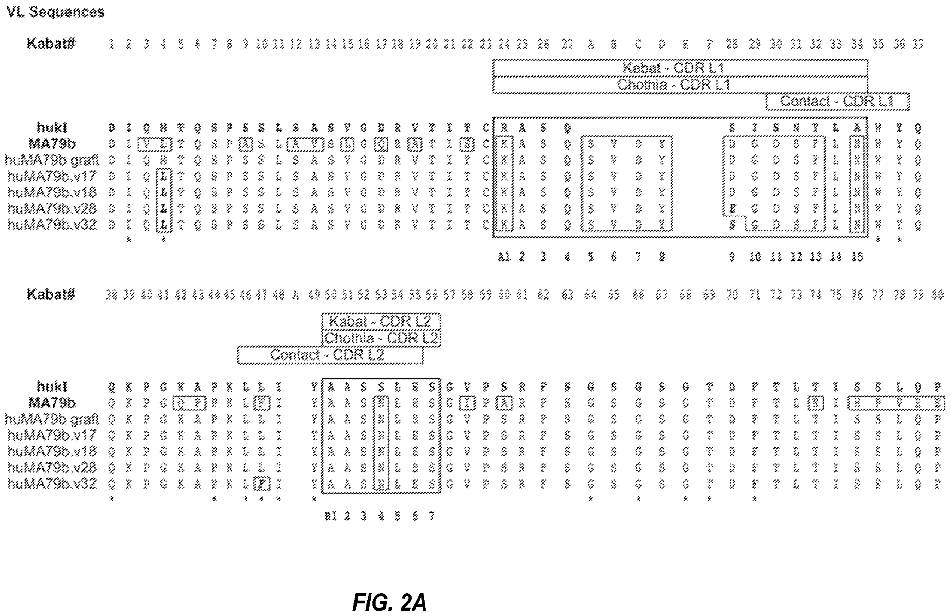

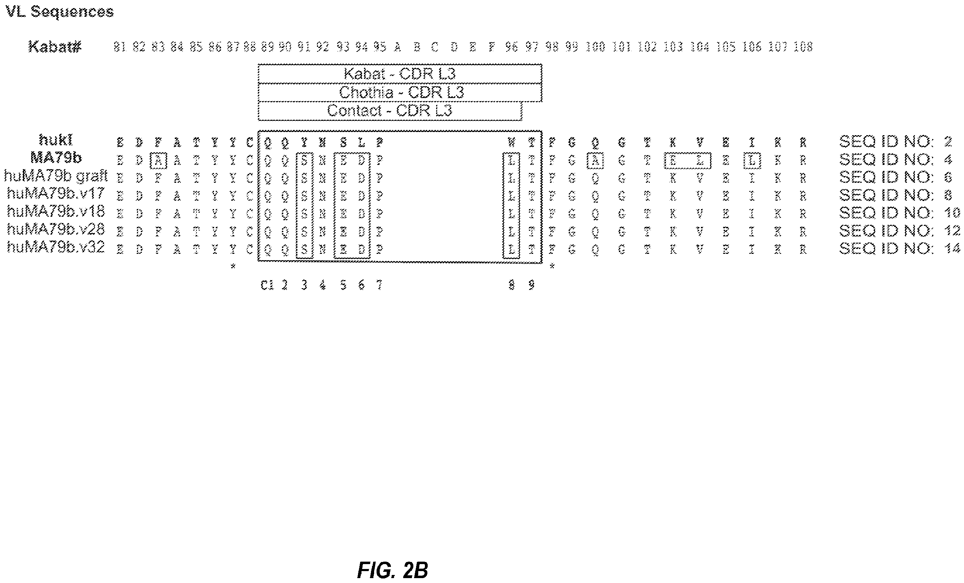

**Figures 2A-2B** show the amino acid sequence of the light chain variable region of murine anti-CD79b antibody MA79b aligned with the humanized MA79b graft and humanized versions 17, 18, 28, and 32 (huMA79b graft, huMA79bv17, huMA79bv18, huMA79bv28, and huMA79bv32, respectively), and aligned with the human subgroup kappa I sequence. The HVRs are boxed. The FR-L1, FR-L2, FR-L3, and FR-L4 sequences bracket the HVRs (HVR-L1, HVR-L2, HVR-L3). The sequences are numbered according to Kabat numbering. The Kabat, Chothia, and contact CDRs are indicated about the boxed HVRs.
**Figure 3** shows the full length amino acid sequences (variable and constant regions) of the light and heavy chains of humanized anti-CD79b antibody huMA79bv28, isotype IgG1. The underlined portions are the constant domains.

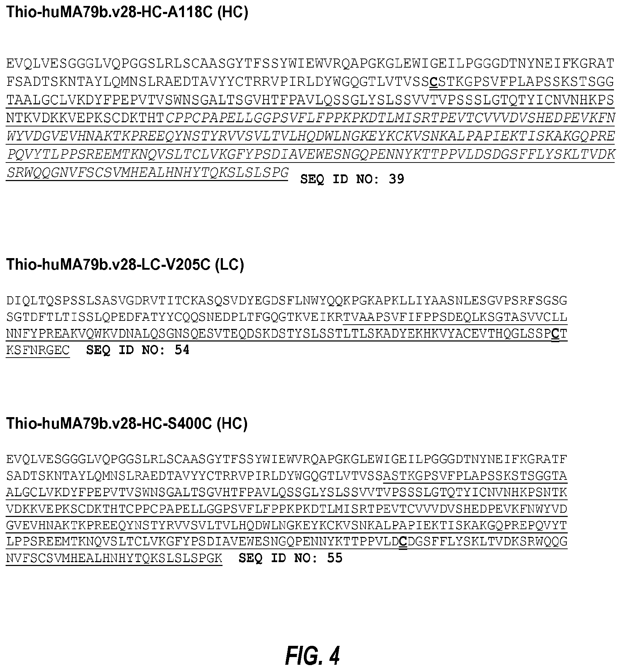

**Figure 4** shows the amino acid sequences of the anti-CD79b cysteine engineered antibodies in which the light chain or heavy chain or Fc region is altered to replace an amino acid with a cysteine at selected amino acid positions. The cysteine engineered antibodies shown include Thio-huMA79bv28-HC-A118C heavy chain, in which the alanine at EU position 118 (sequential position alanine 118) is altered to a cysteine; Thio-huMA79b.v28-LC-V205C light chain in which a valine at Kabat position 205 (sequential position valine 209) is altered to a cysteine; and Thio-huMA79b.v28-HC-S400C heavy chain in which a serine at EU position 400 (sequential position serine 400) is altered to a cysteine. In each figure, the altered amino acid is shown in bold text with double underlining. Single underlining indicates constant regions. Variable regions are not underlined.
**Figure 5** shows the linker and drug structure of huMA79bv28-PBD, which is described in Example A.
**Figure 6** shows efficacy of various antibody-drug conjugates in a WSU-DLCL2 mouse xenograft model, as described in Example B.
**Figure 7** shows efficacy of various antibody-drug conjugates in a Granta-519 mouse xenograft model, as described in Example C.
**Figure 8** shows efficacy of various antibody-drug conjugates in a SuDHL4-luc mouse xenograft model, as described in Example D.
**Figure 9** shows dose-dependent inhibition of tumor growth by huMA79bv28-PBD in a SuDHL4-luc mouse xenograft model, as described in Example E.
**Figure 10** shows dose-dependent inhibition of tumor growth by huMA79bv28-PBD in a BJAB-luc mouse xenograft model, as described in Example F.
**Figure 11** shows inhibition of tumor growth by huMA79bv28-MMAE in a BJAB-luc mouse xenograft model, as described in Example G.

### DETAILED DESCRIPTION

### I. DEFINITIONS

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

"Affinity" refers to the strength of the sum total of noncovalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-CD79b antibody" and "an antibody that binds to CD79b" refer to an antibody that is capable of binding CD79b with sufficient affinity such that the antibody is useful as a diagnostic and/or therapeutic agent in targeting CD79b. In one embodiment, the extent of binding of an anti-CD79b antibody to an unrelated, non-CD79b protein is less than about 10% of the binding of the antibody to CD79b as measured, e.g., by a radioimmunoassay (RIA). In certain embodiments, an antibody that binds to CD79b has a dissociation constant (Kd) of ≤ 1µM, ≤ 100nM, ≤ 10 nM, , ≤ 5 Nm, , ≤ 4 nM, , ≤ 3 nM, , ≤ 2 nM, ≤ 1 nM, ≤ 0.1 nM, ≤ 0.01 nM, or ≤ 0.001 nM (*e.g*., 10⁻⁸ M or less, *e.g*. from 10⁻⁸ M to 10⁻¹³ M, *e.g*., from 10⁻⁹ M to 10⁻¹³ M). In certain embodiments, an anti-CD79b antibody binds to an epitope of CD79b that is conserved among CD79b from different species.

The term "antibody" is used herein in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody and that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The terms "cancer" and "cancerous" refer to or describe the physiological condition in mammals that is typically characterized by unregulated cell growth/proliferation. Examples of cancer include, but are not limited to, melanoma, carcinoma, lymphoma (e.g., Hodgkin's and non-Hodgkin's lymphoma), blastoma, sarcoma, and leukemia. More particular examples of cancer include B-cell associated cancers, including for example, high, intermediate and low grade lymphomas (including B cell lymphomas such as, for example, mucosa-associated-lymphoid tissue B cell lymphoma and non-Hodgkin's lymphoma (NHL), mantle cell lymphoma, Burkitt's lymphoma, small lymphocytic lymphoma, marginal zone lymphoma, diffuse large cell lymphoma, follicular lymphoma, and Hodgkin's lymphoma and T cell lymphomas) and leukemias (including secondary leukemia, chronic lymphocytic leukemia (CLL), such as B cell leukemia (CD5+ B lymphocytes), myeloid leukemia, such as acute myeloid leukemia, chronic myeloid leukemia, lymphoid leukemia, such as acute lymphoblastic leukemia (ALL) and myelodysplasia), and other hematological and/or B cell- or T-cell-associated cancers. Also included are cancers of additional hematopoietic cells, including polymorphonuclear leukocytes, such as basophils, eosinophils, neutrophils and monocytes, dendritic cells, platelets, erythrocytes and natural killer cells. Also included are cancerous B cell proliferative disorders selected from the following: lymphoma, non-Hodgkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), and mantle cell lymphoma. The origins of B-cell cancers include as follows: marginal zone B-cell lymphoma origins in memory B-cells in marginal zone, follicular lymphoma and diffuse large B-cell lymphoma originates in centrocytes in the light zone of germinal centers, chronic lymphocytic leukemia and small lymphocytic leukemia originates in B1 cells (CD5+), mantle cell lymphoma originates in naive B-cells in the mantle zone and Burkitt's lymphoma originates in centroblasts in the dark zone of germinal centers. Tissues which include hematopoietic cells referred herein to as "hematopoietic cell tissues" include thymus and bone marrow and peripheral lymphoid tissues, such as spleen, lymph nodes, lymphoid tissues associated with mucosa, such as the gut-associated lymphoid tissues, tonsils, Peyer's patches and appendix and lymphoid tissues associated with other mucosa, for example, the bronchial linings. Further particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

A "B-cell malignancy" herein includes non-Hodgkin's lymphoma (NHL), including low grade/follicular NHL, small lymphocytic (SL) NHL, intermediate grade/follicular NHL, intermediate grade diffuse NHL, high grade immunoblastic NHL, high grade lymphoblastic NHL, high grade small non-cleaved cell NHL, bulky disease NHL, mantle cell lymphoma, AIDS-related lymphoma, and Waldenstrom's Macroglobulinemia, non-Hodgkin's lymphoma (NHL), lymphocyte predominant Hodgkin's disease (LPHD), small lymphocytic lymphoma (SLL), chronic lymphocytic leukemia (CLL), indolent NHL including relapsed indolent NHL and rituximab-refractory indolent NHL; leukemia, including acute lymphoblastic leukemia (ALL), chronic lymphocytic leukemia (CLL), Hairy cell leukemia, chronic myeloblastic leukemia; Burkitt's lymphoma; mantle cell lymphoma; and other hematologic malignancies. Such malignancies may be treated with antibodies directed against B-cell surface markers, such as CD79b. Such diseases are contemplated herein to be treated by the administration of an antibody directed against a B cell surface marker, such as CD79b, and includes the administration of an unconjugated ("naked") antibody or an antibody conjugated to a cytotoxic agent as disclosed herein. Such diseases are also contemplated herein to be treated by combination therapy including an anti-CD79b antibody or anti-CD79b antibody drug conjugate of the invention in combination with another antibody or antibody drug conjugate, another cytoxic agent, radiation or other treatment administered simultaneously or in series. In an exemplary treatment method, an anti-CD79b immunoconjugate is administered in combination with an anti-CD22 antibody, immunoglobulin, or CD22 binding fragment thereof, either together or sequentially. The anti-CD22 antibody may be a naked antibody or an antibody drug conjugate. In another exemplary treatment method, an anti-CD79b immunoconjugate is administered in combination with an anti-CD20 antibody, immunoglobulin, or CD20 binding fragment thereof, either together or sequentially. The anti-CD20 antibody may be a naked antibody or an antibody drug conjugate. In some embodiments of the combination therapy, the anti-CD79b immunoconjugate is administered with Rituxan® (rituximab).

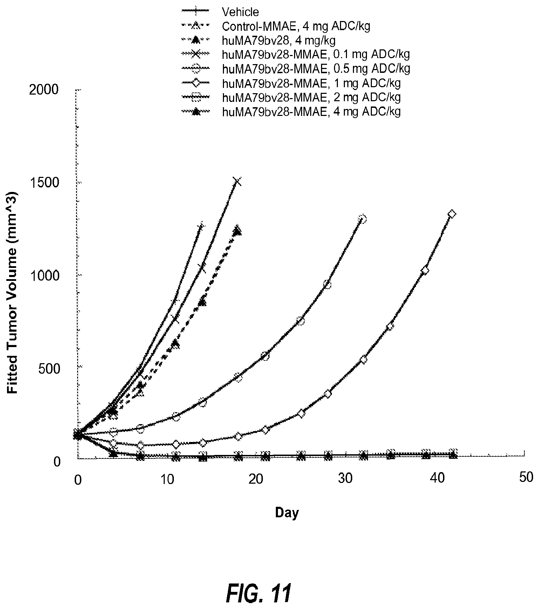

The term "non-Hodgkin's lymphoma" or "NHL", as used herein, refers to a cancer of the lymphatic system other than Hodgkin's lymphomas. Hodgkin's lymphomas can generally be distinguished from non-Hodgkin's lymphomas by the presence of Reed-Sternberg cells in Hodgkin's lymphomas and the absence of said cells in non-Hodgkin's lymphomas. Examples of non-Hodgkin's lymphomas encompassed by the term as used herein include any that would be identified as such by one skilled in the art (e.g., an oncologist or pathologist) in accordance with classification schemes known in the art, such as the Revised European-American Lymphoma (REAL) scheme as described in Color Atlas of Clinical Hematology (3rd edition), A. Victor Hoffbrand and John E. Pettit (eds.) (Harcourt Publishers Ltd., 2000). See, in particular, the lists in Fig. 11.57, 11.58 and 11.59. More specific examples include, but are not limited to, relapsed or refractory NHL, front line low grade NHL, Stage III/IV NHL, chemotherapy resistant NHL, precursor B lymphoblastic leukemia and/or lymphoma, small lymphocytic lymphoma, B cell chronic lymphocytic leukemia and/or prolymphocytic leukemia and/or small lymphocytic lymphoma, B-cell prolymphocytic lymphoma, immunocytoma and/or lymphoplasmacytic lymphoma, lymphoplasmacytic lymphoma, marginal zone B cell lymphoma, splenic marginal zone lymphoma, extranodal marginal zone - MALT lymphoma, nodal marginal zone lymphoma, hairy cell leukemia, plasmacytoma and/or plasma cell myeloma, low grade/follicular lymphoma, intermediate grade/follicular NHL, mantle cell lymphoma, follicle center lymphoma (follicular), intermediate grade diffuse NHL, diffuse large B-cell lymphoma, aggressive NHL (including aggressive front-line NHL and aggressive relapsed NHL), NHL relapsing after or refractory to autologous stem cell transplantation, primary mediastinal large B-cell lymphoma, primary effusion lymphoma, high grade immunoblastic NHL, high grade lymphoblastic NHL, high grade small non-cleaved cell NHL, bulky disease NHL, Burkitt's lymphoma, precursor (peripheral) large granular lymphocytic leukemia, mycosis fungoides and/or Sezary syndrome, skin (cutaneous) lymphomas, anaplastic large cell lymphoma, angiocentric lymphoma.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG₁, IgG₂, IgG₃, IgG₄, IgA₁, and IgA₂. The heavy chain constant domains that correspond to the different classes of immunoglobulins are called α, δ ε, γ, and µ, respectively.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "chemotherapeutic agent" is a chemical compound useful in the treatment of cancer. Examples of chemotherapeutic agents include alkylating agents such as thiotepa and cyclosphosphamide (CYTOXAN^{®}); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); delta-9-tetrahydrocannabinol (dronabinol, MARINOL^{®}); beta-lapachone; lapachol; colchicines; betulinic acid; a camptothecin (including the synthetic analogue topotecan (HYCAMTIN^{®}), CPT-11 (irinotecan, CAMPTOSAR^{®}), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE^{®}, FILDESIN^{®}); dacarbazine; mannomustine; mitobronitol; mitolactol; pipobroman; gacytosine; arabinoside ("Ara-C"); thiotepa; taxoids, e.g., paclitaxel (TAXOL^{®}; Bristol-Myers Squibb Oncology, Princeton, N.J.), ABRAXANE™ Cremophor-free, albumin-engineered nanoparticle formulation of paclitaxel (American Pharmaceutical Partners, Schaumberg, Illinois), and docetaxel (TAXOTERE^{®}; Rhône-Poulenc Rorer, Antony, France); chloranbucil; gemcitabine (GEMZAR®); 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluoromethylornithine (DMFO); retinoids such as retinoic acid; capecitabine (XELODA®); pharmaceutically acceptable salts, acids or derivatives of any of the above; as well as combinations of two or more of the above such as CHOP, an abbreviation for a combined therapy of cyclophosphamide, doxorubicin, vincristine, and prednisolone; CVP, an abbreviation for a combined therapy of cyclophosphamide, vincristine, and prednisolone; and FOLFOX, an abbreviation for a treatment regimen with oxaliplatin (ELOXATIN™) combined with 5-FU and leucovorin.

"Effector functions" refer to those biological activities attributable to the Fc region of an antibody, which vary with the antibody isotype. Examples of antibody effector functions include: Clq binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of an agent, e.g., a pharmaceutical formulation, refers to an amount effective, at dosages and for periods of time necessary, to achieve the desired therapeutic or prophylactic result.

The term "epitope" refers to the particular site on an antigen molecule to which an antibody binds.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The term "glycosylated forms of CD79b" refers to naturally occurring forms of CD79b that are post-translationally modified by the addition of carbohydrate residues.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., *supra.* In one embodiment, for the VH, the subgroup is subgroup III as in Kabat et al., *supra.*

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR," as used herein, refers to each of the regions of an antibody variable domain which are hypervariable in sequence and/or form structurally defined loops ("hypervariable loops"). Generally, native four-chain antibodies comprise six HVRs; three in the VH (H1, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (See Almagro and Fransson, Front. Biosci. 13:1619-1633 (2008).) Unless otherwise indicated, HVR residues and other residues in the variable domain (e.g., FR residues) are numbered herein according to Kabat et al., *supra.*

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated antibody" is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated nucleic acid" refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-CD79b antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "CD79b," as used herein, refers to any native CD79b from any vertebrate source, including mammals such as primates (e.g. humans, cynomolgus monkey (cyno)) and rodents (e.g., mice and rats), unless otherwise indicated. The term encompasses "full-length," unprocessed CD79b as well as any form of CD79b that results from processing in the cell. The term also encompasses naturally occurring variants of CD79b, e.g., splice variants, allelic variants, and isoforms. The amino acid sequence of an exemplary human CD79b precursor (with signal sequence) is shown in SEQ ID NO: 40. The amino acid sequence of an exemplary human mature CD79b (without signal sequence) is shown in SEQ ID NO: 41.

The term "CD79b-positive cancer" refers to a cancer comprising cells that express CD79b on their surface.

The term "CD79b-positive cell" refers to a cell that expresses CD79b on its surface.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, immunoconjugates of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The phrase "optionally substituted" as used herein, pertains to a parent group that may be unsubstituted or that may be substituted.

Unless otherwise specified, the term "substituted" as used herein, pertains to a parent group that bears one or more substituents. The term "substituent" is used herein in the conventional sense and refers to a chemical moiety that is covalently attached to, or if appropriate, fused to, a parent group. A wide variety of substituents are known, and methods for their formation and introduction into a variety of parent groups are also known.

In some embodiments, the substituents described herein (which include optional substituents) are limited to those groups that are not reactive to the antibody. In some embodiments, the link to the antibody is formed from the N10 position of the PBD compound through the linker (L). In some instances, reactive functional groups located at other parts of the PBD structure may be capable of forming additional bonds to the antibody (this may be referred to as crosslinking). Such additional bonds, in some instances, may alter transport and biological activity of the conjugate. Therefore, in some embodiments, the additional substituents are limited to those lacking reactive functionality.

In some embodiments, the substituents are selected from R, OR, SR, NRR', NO₂, halo, CO₂R, COR, CONH₂, CONHR, and CONRR'. In some embodiments, the substituents are selected from R, OR, SR, NRR', NO₂, CO₂R, COR, CONH₂, CONHR, and CONRR'. In some embodiments, the substituents are selected from R, OR, SR, NRR', NO₂, and halo. In some embodiments, the substituents are selected from the group consisting of R, OR, SR, NRR', and NO₂.

Any of the embodiments discussed above may be applied to any of the substituents described herein. Alternatively, the substituents may be selected from one or more of the groups discussed below.

The term "C₁₋₁₂ alkyl" as used herein, pertains to a monovalent moiety obtained by removing a hydrogen atom from a carbon atom of a hydrocarbon compound having from 1 to 12 carbon atoms, which is aliphatic, and which may be cyclic or acyclic, and which may be saturated or unsaturated (e.g. partially unsaturated, fully unsaturated). Thus, the term "alkyl" includes the sub-classes alkenyl, alkynyl, cycloalkyl, etc., discussed below.

Examples of saturated alkyl groups include, but are not limited to, methyl (C₁), ethyl (C₂), propyl (C₃), butyl (C₄), pentyl (C₅), hexyl (C₆) and heptyl (C₇).

Examples of saturated linear alkyl groups include, but are not limited to, methyl (C₁), ethyl (C₂), n-propyl (C₃), n-butyl (C₄), n-pentyl (amyl) (C₅), n-hexyl (C₆) and n-heptyl (C7).

Examples of saturated branched alkyl groups include, iso-propyl (C₃), iso-butyl (C₄), sec-butyl (C₄), tert-butyl (C₄), iso-pentyl (C₅), and neo-pentyl (C₅).

An alkyl group may optionally be interrupted by one or more heteroatoms selected from O, N(H) and S. Such groups may be referred to as "heteroalkyl".

The term "C₂₋₁₂ heteroalkyl" as used herein, pertains to a monovalent moiety obtained by removing a hydrogen atom from a carbon atom of a hydrocarbon compound having from 2 to 12 carbon atoms, and one or more heteroatoms selected from O, N(H) and S, preferably O and S.

Examples of heteroalkyl groups include, those comprising one or more ethylene glycol units of the type -(OCH₂CH₂)-. The terminus of a heteroalkyl group may be the primary form of a heteroatom, e.g. -OH, -SH or -NH₂. In a preferred embodiment, the terminus is -CH₃.

The term "C₂₋₁₂ alkenyl" as used herein, pertains to an alkyl group having one or more carbon-carbon double bonds.

Examples of unsaturated alkenyl groups include, ethenyl (vinyl, -CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

The term "C₂₋₁₂ alkynyl" as used herein, pertains to an alkyl group having one or more carbon-carbon triple bonds.

Examples of unsaturated alkynyl groups include, ethynyl (-C≡CH) and 2-propynyl (propargyl, -CH₂-C≡CH).

The term "C₃₋₁₂ cycloalkyl" as used herein, pertains to an alkyl group which is also a cyclyl group; that is, a monovalent moiety obtained by removing a hydrogen atom from an alicyclic ring atom of a cyclic hydrocarbon (carbocyclic) compound, which moiety has from 3 to 7 carbon atoms, including from 3 to 7 ring atoms.

Examples of cycloalkyl groups include, those derived from:
(i) saturated monocyclic hydrocarbon compounds: cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) and methylcyclohexane (C₇);
(ii) unsaturated monocyclic hydrocarbon compounds:
   cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
(iii) saturated polycyclic hydrocarbon compounds:
   norcarane (C₇), norpinane (C₇), norbornane (C₇).

The term "C₃₋₂₀ heterocyclyl" as used herein, pertains to a monovalent moiety obtained by removing a hydrogen atom from a ring atom of a heterocyclic compound, which moiety has from 3 to 20 ring atoms, of which from 1 to 10 are ring heteroatoms. In some embodiments, each ring has from 3 to 7 ring atoms, of which from 1 to 4 are ring heteroatoms.

As used herein, the prefixes (e.g. C₃₋₂₀, C₃₋₇, C₅₋₆, etc.) denote the number of ring atoms, or range of number of ring atoms, whether carbon atoms or heteroatoms. For example, the term "C₅₋₆heterocyclyl", as used herein, pertains to a heterocyclyl group having 5 or 6 ring atoms.

Examples of monocyclic heterocyclyl groups include, those derived from:
(i) N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
(ii) O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
(iii) S₁: thiirane (C₃), thietane (C₄), thiolane (tetrahydrothiophene) (C₅), thiane (tetrahydrothiopyran) (C₆), thiepane (C₇);
(iv) O₂: dioxolane (C₅), dioxane (C₆), and dioxepane (C₇);
(v) O₃: trioxane (C₆);
(vi) N₂: imidazolidine (C₅), pyrazolidine (diazolidine) (C₅), imidazoline (C₅), pyrazoline (dihydropyrazole) (C₅), piperazine (C₆);
(vii) N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
(viii) N₁S₁: thiazoline (C₅), thiazolidine (C₅), thiomorpholine (C₆);
(ix) N₂O₁: oxadiazine (C₆);
(x) O₁S₁: oxathiole (C₅) and oxathiane (thioxane) (C₆); and,
(xi) N₁O₁S₁: oxathiazine (C₆).

Examples of substituted monocyclic heterocyclyl groups include, those derived from saccharides, in cyclic form, for example, furanoses (C₅), such as arabinofuranose, lyxofuranose, ribofuranose, and xylofuranse, and pyranoses (C₆), such as allopyranose, altropyranose, glucopyranose, mannopyranose, gulopyranose, idopyranose, galactopyranose, and talopyranose.

The term "C₅₋₂₀ aryl", as used herein, pertains to a monovalent moiety obtained by removing a hydrogen atom from an aromatic ring atom of an aromatic compound, which moiety has from 3 to 20 ring atoms. In some embodiments, each ring has from 5 to 7 ring atoms.

In some embodiments, the ring atoms are all carbon atoms, as in "carboaryl groups". Examples of carboaryl groups include, but are not limited to, those derived from benzene (i.e. phenyl) (C₆), naphthalene (C₁₀), azulene (C₁₀), anthracene (C₁₄), phenanthrene (C₁₄), naphthacene (C₁₈), and pyrene (C₁₆).

Examples of aryl groups which comprise fused rings, at least one of which is an aromatic ring, include, groups derived from indane (e.g. 2,3-dihydro-1H-indene) (C₉), indene (C₉), isoindene (C₉), tetraline (1,2,3,4-tetrahydronaphthalene (C₁₀), acenaphthene (C₁₂), fluorene (C₁₃), phenalene (C₁₃), acephenanthrene (C₁₅), and aceanthrene (C₁₆).

In some embodiments, the ring atoms may include one or more heteroatoms, as in "heteroaryl groups". Examples of monocyclic heteroaryl groups include, those derived from:
(i) N₁: pyrrole (azole) (C₅), pyridine (azine) (C₆);
(ii) O₁: furan (oxole) (C₅);
(iii) S₁: thiophene (thiole) (C₅);
(iv) N₁O₁: oxazole (C₅), isoxazole (C₅), isoxazine (C₆);
(v) N₂O₁: oxadiazole (furazan) (C₅);
(vi) N₃O₁: oxatriazole (C₅);
(vii) N₁S₁: thiazole (C₅), isothiazole (C₅);
(viii) N₂: imidazole (1,3-diazole) (C₅), pyrazole (1,2-diazole) (C₅), pyridazine (1,2-diazine) (C₆), pyrimidine (1,3-diazine) (C₆) (e.g., cytosine, thymine, uracil), pyrazine (1,4-diazine) (C₆);
(ix) N₃: triazole (C₅), triazine (C₆); and,
(x) N₄: tetrazole (C₅).

Examples of heteroaryl which comprise fused rings, include:
(i) C₉ (with 2 fused rings) derived from benzofuran (O₁), isobenzofuran (O₁), indole (N₁), isoindole (N₁), indolizine (N₁), indoline (N₁), isoindoline (N₁), purine (N₄) (e.g., adenine, guanine), benzimidazole (N₂), indazole (N₂), benzoxazole (N₁O₁), benzisoxazole (N₁O₁), benzodioxole (O₂), benzofurazan (N₂O₁), benzotriazole (N₃), benzothiofuran (S₁), benzothiazole (N₁S₁), benzothiadiazole (N₂S);
(ii) C₁₀ (with 2 fused rings) derived from chromene (O₁), isochromene (O₁), chroman (O₁), isochroman (O₁), benzodioxan (O₂), quinoline (N₁), isoquinoline (N₁), quinolizine (N₁), benzoxazine (N₁O₁), benzodiazine (N₂), pyridopyridine (N₂), quinoxaline (N₂), quinazoline (N₂), cinnoline (N₂), phthalazine (N₂), naphthyridine (N₂), pteridine (N₄);
(iii) C₁₁ (with 2 fused rings) derived from benzodiazepine (N₂);
(iv) C₁₃ (with 3 fused rings) derived from carbazole (N₁), dibenzofuran (O₁), dibenzothiophene (S₁), carboline (N₂), perimidine (N₂), pyridoindole (N₂); and,
(v) C₁₄ (with 3 fused rings) derived from acridine (N₁), xanthene (O₁), thioxanthene (S₁), oxanthrene (O₂), phenoxathiin (O₁S₁), phenazine (N₂), phenoxazine (N₁O₁), phenothiazine (N₁S₁), thianthrene (S₂), phenanthridine (N₁), phenanthroline (N₂), phenazine (N₂).

The above groups, whether alone or part of another substituent, may themselves optionally be substituted with one or more groups selected from themselves and the additional substituents listed below.

Halo: -F, -Cl, -Br, and -I.

Hydroxy: -OH.

Ether: -OR, wherein R is an ether substituent, for example, a C₁₋₇ alkyl group (also referred to as a C₁₋₇ alkoxy group, discussed below), a C₃₋₂₀ heterocyclyl group (also referred to as a C₃₋₂₀ heterocyclyloxy group), or a C₅₋₂₀ aryl group (also referred to as a C₅₋₂₀ aryloxy group). In some embodiments, R is a C₁₋₇ alkyl group.

Alkoxy: -OR, wherein R is an alkyl group, for example, a C₁₋₇ alkyl group. Examples of C₁₋₇ alkoxy groups include, -OMe (methoxy), -OEt (ethoxy), -O(nPr) (n-propoxy), -O(iPr) (isopropoxy), -O(nBu) (n-butoxy), -O(sBu) (sec-butoxy), -O(iBu) (isobutoxy), and -O(tBu) (tert-butoxy).

Acetal: -CH(OR¹)(OR²), wherein R¹ and R² are independently acetal substituents, for example, a C₁₋₇ alkyl group, a C₃₋₂₀ heterocyclyl group, or a C₅₋₂₀ aryl group. In some embodiments, R¹ and/or R² are independently a C₁₋₇ alkyl group. In some embodiments, in the case of a "cyclic" acetal group, R¹ and R², taken together with the two oxygen atoms to which they are attached, and the carbon atom to which they are attached, form a heterocyclic ring having from 4 to 8 ring atoms. Examples of acetal groups include, but are not limited to, -CH(OMe)₂, -CH(OEt)₂, and -CH(OMe)(OEt).

Hemiacetal: -CH(OH)(OR¹), wherein R¹ is a hemiacetal substituent, for example, a C₁₋₇ alkyl group, a C₃₋₂₀ heterocyclyl group, or a C₅₋₂₀ aryl group. In some embodiments, R¹ is a C₁₋₇ alkyl group. Examples of hemiacetal groups include, -CH(OH)(OMe) and -CH(OH)(OEt).

Ketal: -CR(OR¹)(OR²), where R¹ and R² are as defined for acetals, and R is a ketal substituent other than hydrogen, for example, a C₁₋₇ alkyl group, a C₃₋₂₀ heterocyclyl group, or a C₅₋₂₀ aryl group. In some embodiments, R is a C₁₋₇ alkyl group. Examples ketal groups include, but are not limited to, -C(Me)(OMe)₂, -C(Me)(OEt)₂, -C(Me)(OMe)(OEt), - C(Et)(OMe)₂, -C(Et)(OEt)₂, and -C(Et)(OMe)(OEt).

Hemiketal: -CR(OH)(OR¹), where R¹ is as defined for hemiacetals, and R is a hemiketal substituent other than hydrogen, for example, a C₁₋₇ alkyl group, a C₃₋₂₀ heterocyclyl group, or a C₅₋₂₀ aryl group. In some embodiments, R is a C₁₋₇ alkyl group. Examples of hemiketal groups include, but are not limited to, -C(Me)(OH)(OMe), - C(Et)(OH)(OMe), -C(Me)(OH)(OEt), and -C(Et)(OH)(OEt).

Oxo (keto, -one): =O.

Thione (thioketone): =S.

Imino (imine): =NR, wherein R is an imino substituent, for example, hydrogen, C₁₋₇ alkyl group, a C₃₋₂₀ heterocyclyl group, or a C₅₋₂₀ aryl group. In some embodiments, R is hydrogen or a C₁₋₇ alkyl group. Examples of imino groups include, =NH, =NMe, =NEt, and =NPh.

Formyl (carbaldehyde, carboxaldehyde): -C(=O)H.

Acyl (keto): -C(=O)R, wherein R is an acyl substituent, for example, a C₁₋₇ alkyl group (also referred to as C₁₋₇ alkylacyl or C₁₋₇ alkanoyl), a C₃₋₂₀heterocyclyl group (also referred to as C₃₋₂₀ heterocyclylacyl), or a C₅₋₂₀ aryl group (also referred to as C₅₋₂₀ arylacyl). In some embodiments, R is a C₁₋₇ alkyl group. Examples of acyl groups include, -C(=O)CH₃ (acetyl), -C(=O)CH₂CH₃ (propionyl), -C(=O)C(CH₃)₃ (t-butyryl), and -C(=O)Ph (benzoyl, phenone).

Carboxy (carboxylic acid): -C(=O)OH.

Thiocarboxy (thiocarboxylic acid): -C(=S)SH.

Thiolocarboxy (thiolocarboxylic acid): -C(=O)SH.

Thionocarboxy (thionocarboxylic acid): -C(=S)OH.

Imidic acid: -C(=NH)OH.

Hydroxamic acid: -C(=NOH)OH.

Ester (carboxylate, carboxylic acid ester, oxycarbonyl): -C(=O)OR, wherein R is an ester substituent, for example, a C₁₋₇ alkyl group, a C₃₋₂₀ heterocyclyl group, or a C₅₋₂₀ aryl group. In some embodiments, R is a C₁₋₇ alkyl group. Examples of ester groups include, -C(=O)OCH₃, -C(=O)OCH₂CH₃, -C(=O)OC(CH₃)₃, and -C(=O)OPh.

Acyloxy (reverse ester): -OC(=O)R, wherein R is an acyloxy substituent, for example, a C₁₋₇ alkyl group, a C₃₋₂₀ heterocyclyl group, or a C₅₋₂₀ aryl group. In some embodiments, R is a C₁₋₇ alkyl group. Examples of acyloxy groups include, -OC(=O)CH₃ (acetoxy), -OC(=O)CH₂CH₃, -OC(=O)C(CH₃)₃, -OC(=O)Ph, and -OC(=O)CH₂Ph.

Oxycarbonyloxy: -OC(=O)OR, wherein R is an ester substituent, for example, a C₁₋₇ alkyl group, a C₃₋₂₀ heterocyclyl group, or a C₅₋₂₀ aryl group. In some embodiments, R is a C₁₋₇ alkyl group. Examples of oxycarbonyloxy groups include, -OC(=O)OCH₃, -OC(=O)OCH₂CH₃, -OC(=O)OC(CH₃)₃, and -OC(=O)OPh.

Amino: -NR¹R², wherein R¹ and R² are independently amino substituents, for example, hydrogen, a C₁₋₇ alkyl group (also referred to as C₁₋₇ alkylamino or di-C₁₋₇ alkylamino), a C₃₋₂₀ heterocyclyl group, or a C₅₋₂₀ aryl group. In some embodiments, R¹ and R² are independently H or a C₁₋₇ alkyl group. In some embodiments, in the case of a "cyclic" amino group, R¹ and R², taken together with the nitrogen atom to which they are attached, form a heterocyclic ring having from 4 to 8 ring atoms. Amino groups may be primary (-NH₂), secondary (-NHR¹), or tertiary (-NHR¹R²), and in cationic form, may be quaternary (-⁺NR¹R²R³). Examples of amino groups include, -NH₂, -NHCH₃, -NHC(CH₃)₂, -N(CH₃)₂, -N(CH₂CH₃)₂, and -NHPh. Examples of cyclic amino groups include, aziridino, azetidino, pyrrolidino, piperidino, piperazino, morpholino, and thiomorpholino.

Amido (carbamoyl, carbamyl, aminocarbonyl, carboxamide): -C(=O)NR¹R², wherein R¹ and R² are independently amino substituents, as defined for amino groups. Examples of amido groups include, -C(=O)NH₂, -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinocarbonyl.

Thioamido (thiocarbamyl): -C(=S)NR¹R², wherein R¹ and R² are independently amino substituents, as defined for amino groups. Examples of thioamido groups include, -C(=S)NH₂, -C(=S)NHCH₃, -C(=S)N(CH₃)₂, and -C(=S)NHCH₂CH₃.

Acylamido (acylamino): -NR¹C(=O)R², wherein R¹ is an amide substituent, for example, hydrogen, a C₁₋₇ alkyl group, a C₃₋₂₀ heterocyclyl group, or a C₅₋₂₀ aryl group, and R² is an acyl substituent, for example, a C₁₋₇ alkyl group, a C₃₋₂₀ heterocyclyl group, or a C₅₋₂₀aryl group. In some embodiments, R¹ and/or R² is hydrogen or a C₁₋₇ alkyl group. Examples of acylamide groups include, -NHC(=O)CH₃, -NHC(=O)CH₂CH₃, and -NHC(=O)Ph. R¹ and R² may together form a cyclic structure, as in, for example, succinimidyl, maleimidyl, and phthalimidyl:

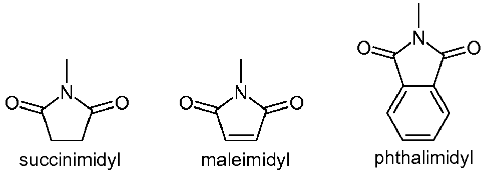

Aminocarbonyloxy: -OC(=O)NR¹R², wherein R¹ and R² are independently amino substituents, as defined for amino groups. Examples of aminocarbonyloxy groups include, -OC(=O)NH₂, -OC(=O)NHMe, -OC(=O)NMe₂, and -OC(=O)NEt₂.

Ureido: -N(R¹)CONR²R³ wherein R² and R³ are independently amino substituents, as defined for amino groups, and R¹ is a ureido substituent, for example, hydrogen, a C₁₋₇ alkyl group, a C₃₋₂₀ heterocyclyl group, or a C₅₋₂₀ aryl group. In some embodiments, R¹ is hydrogen or a C₁₋₇ alkyl group. Examples of ureido groups include, -NHCONH₂, -NHCONHMe, -NHCONHEt, -NHCONMe₂, -NHCONEt₂,-NMeCONH₂, -NMeCONHMe, -NMeCONHEt, -NMeCONMe₂, and -NMeCONEt₂.

Guanidino: -NH-C(=NH)NH₂.

Tetrazolyl: a five membered aromatic ring having four nitrogen atoms and one carbon atom,

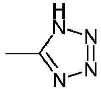

Amidine (amidino): -C(=NR)NR₂, wherein each R is an amidine substituent, for example, hydrogen, a C₁₋₇ alkyl group, a C₃₋₂₀ heterocyclyl group, or a C₅₋₂₀ aryl group. In some embodiments, each R is H or a C₁₋₇ alkyl group. Examples of amidine groups include, -C(=NH)NH₂, -C(=NH)NMe₂, and -C(=NMe)NMe₂.

Nitro: -NO₂.

Nitroso: -NO.

Azido: -N₃.

Cyano (nitrile, carbonitrile): -CN.

Isocyano: -NC.

Cyanato: -OCN.

Isocyanato: -NCO.

Thiocyano (thiocyanato): -SCN.

Isothiocyano (isothiocyanato): -NCS.

Sulfhydryl (thiol, mercapto): -SH.

Thioether (sulfide): -SR, wherein R is a thioether substituent, for example, a C₁₋₇ alkyl group (also referred to as a C₁₋₇alkylthio group), a C₃₋₂₀ heterocyclyl group, or a C₅₋₂₀ aryl group. In some embodiments, R is a C₁₋₇ alkyl group. Examples of thioether groups include, -SCH₃ and -SCH₂CH₃.

Disulfide: -SS-R, wherein R is a disulfide substituent, for example, a C₁₋₇ alkyl group, a C₃₋₂₀ heterocyclyl group, or a C₅₋₂₀ aryl group. In some embodiments, R is a C₁₋₇ alkyl group (also referred to herein as C₁₋₇ alkyl disulfide). Examples of disulfide groups include, -SSCH₃ and -SSCH₂CH₃.

Sulfine (sulfinyl, sulfoxide): -S(=O)R, wherein R is a sulfine substituent, for example, a C₁₋₇ alkyl group, a C₃₋₂₀ heterocyclyl group, or a C₅₋₂₀ aryl group. In some embodiments, R is a C₁₋₇ alkyl group. Examples of sulfine groups include, -S(=O)CH₃ and -S(=O)CH₂CH₃.

Sulfone (sulfonyl): -S(=O)₂R, wherein R is a sulfone substituent, for example, a C₁₋₇ alkyl group, a C₃₋₂₀ heterocyclyl group, or a C₅₋₂₀ aryl group. In some embodiments, R is a C₁₋₇ alkyl group, including, for example, a fluorinated or perfluorinated C₁₋₇ alkyl group. Examples of sulfone groups include, -S(=O)₂CH₃ (methanesulfonyl, mesyl), -S(=O)₂CF₃ (triflyl), -S(=O)₂CH₂CH₃ (esyl), -S(=O)₂C₄F₉ (nonaflyl), -S(=O)₂CH₂CF₃ (tresyl), -S(=O)₂CH₂CH₂NH₂ (tauryl), -S(=O)₂Ph (phenylsulfonyl, besyl), 4-methylphenylsulfonyl (tosyl), 4-chlorophenylsulfonyl (closyl), 4-bromophenylsulfonyl (brosyl), 4-nitrophenyl (nosyl), 2-naphthalenesulfonate (napsyl), and 5-dimethylamino-naphthalen-1-ylsulfonate (dansyl).

Sulfinic acid (sulfino): -S(=O)OH, -SO₂H.

Sulfonic acid (sulfo): -S(=O)₂OH, -SO₃H.

Sulfinate (sulfinic acid ester): -S(=O)OR; wherein R is a sulfinate substituent, for example, a C₁₋₇ alkyl group, a C₃₋₂₀ heterocyclyl group, or a C₅₋₂₀ aryl group. In some embodiments, R is a C₁₋₇ alkyl group. Examples of sulfinate groups include, -S(=O)OCH₃ (methoxysulfinyl; methyl sulfinate) and -S(=O)OCH₂CH₃ (ethoxysulfinyl; ethyl sulfinate).

Sulfonate (sulfonic acid ester): -S(=O)₂OR, wherein R is a sulfonate substituent, for example, a C₁₋₇ alkyl group, a C₃₋₂₀ heterocyclyl group, or a C₅₋₂₀ aryl group. In some embodiments, R is a C₁₋₇ alkyl group. Examples of sulfonate groups include, -S(=O)₂OCH₃ (methoxysulfonyl; methyl sulfonate) and -S(=O)₂OCH₂CH₃ (ethoxysulfonyl; ethyl sulfonate).

Sulfinyloxy: -OS(=O)R, wherein R is a sulfinyloxy substituent, for example, a C₁₋₇ alkyl group, a C₃₋₂₀ heterocyclyl group, or a C₅₋₂₀ aryl group. In some embodiments, R is a C₁₋₇ alkyl group. Examples of sulfinyloxy groups include, -OS(=O)CH₃ and -OS(=O)CH₂CH₃.

Sulfonyloxy: -OS(=O)₂R, wherein R is a sulfonyloxy substituent, for example, a C₁₋₇ alkyl group, a C₃₋₂₀ heterocyclyl group, or a C₅₋₂₀ aryl group. In some embodiments, R is a C₁₋₇ alkyl group. Examples of sulfonyloxy groups include, -OS(=O)₂CH₃ (mesylate) and -OS(=O)₂CH₂CH₃ (esylate).

Sulfate: -OS(=O)₂OR; wherein R is a sulfate substituent, for example, a C₁₋₇ alkyl group, a C₃₋₂₀ heterocyclyl group, or a C₅₋₂₀ aryl group. In some embodiments, R is a C₁₋₇ alkyl group. Examples of sulfate groups include, -OS(=O)₂OCH₃ and -SO(=O)₂OCH₂CH₃.

Sulfamyl (sulfamoyl; sulfinic acid amide; sulfinamide): -S(=O)NR¹R², wherein R¹ and R² are independently amino substituents, as defined for amino groups. Examples of sulfamyl groups include, -S(=O)NH₂, -S(=O)NH(CH₃), -S(=O)N(CH₃)₂, -S(=O)NH(CH₂CH₃), -S(=O)N(CH₂CH₃)₂, and -S(=O)NHPh.

Sulfonamido (sulfinamoyl; sulfonic acid amide; sulfonamide): -S(=O)₂NR¹R² wherein R¹ and R² are independently amino substituents, as defined for amino groups. Examples of sulfonamido groups include, -S(=O)₂NH₂, -S(=O)₂NH(CH₃), -S(=O)₂N(CH₃)₂, -S(=O)₂NH(CH₂CH₃), -S(=O)₂N(CH₂CH ₃)₂, and -S(=O)₂NHPh.

Sulfamino: -NR¹S(=O)₂OH, wherein R¹ is an amino substituent, as defined for amino groups. Examples of sulfamino groups include, -NHS(=O)₂OH and -N(CH₃)S(=O)₂OH.

Sulfonamino: -NR¹S(=O)₂R, wherein R¹ is an amino substituent, as defined for amino groups, and R is a sulfonamino substituent, for example, a C₁₋₇ alkyl group, a C₃₋₂₀ heterocyclyl group, or a C₅₋₂₀ aryl group. In some embodiments, R is a C₁₋₇ alkyl group. Examples of sulfonamino groups include, -NHS(=O)₂CH₃ and -N(CH₃)S(=O)₂C₆H₅.

Sulfinamino: -NR¹S(=O)R, wherein R¹ is an amino substituent, as defined for amino groups, and R is a sulfinamino substituent, for example, a C₁₋₇ alkyl group, a C₃₋₂₀ heterocyclyl group, or a C₅₋₂₀ aryl group. In some embodiments, R is a C₁₋₇ alkyl group. Examples of sulfinamino groups include, -NHS(=O)CH₃ and -N(CH₃)S(=O)C₆H₅.

Phosphino (phosphine): -PR₂, wherein R is a phosphino substituent, for example, -H, a C₁₋₇ alkyl group, a C₃₋₂₀ heterocyclyl group, or a C₅₋₂₀ aryl group. In some embodiments, R is -H, a C₁₋₇ alkyl group, or a C₅₋₂₀ aryl group. Examples of phosphino groups include, -PH₂, -P(CH₃)₂, -P(CH₂CH₃)₂, -P(t-Bu)₂, and -P(Ph)₂.

Phospho: -P(=O)₂.

Phosphinyl (phosphine oxide): -P(=O)R₂, wherein R is a phosphinyl substituent, for example, a C₁₋₇ alkyl group, a C₃₋₂₀ heterocyclyl group, or a C₅₋₂₀ aryl group. In some embodiments, R is a C₁₋₇ alkyl group or a C₅₋₂₀ aryl group. Examples of phosphinyl groups include, -P(=O)(CH₃)₂, -P(=O)(CH₂CH₃)₂, -P(=O)(t-Bu)₂, and -P(=O)(Ph)₂.

Phosphonic acid (phosphono): -P(=O)(OH)₂.

Phosphonate (phosphono ester): -P(=O)(OR)₂, where R is a phosphonate substituent, for example, -H, a C₁₋₇ alkyl group, a C₃₋₂₀ heterocyclyl group, or a C₅₋₂₀ aryl group. In some embodiments, R is -H, a C₁₋₇ alkyl group, or a C₅₋₂₀ aryl group. Examples of phosphonate groups include, -P(=O)(OCH₃)₂, -P(=O)(OCH₂CH₃)₂, -P(=O)(O-t-Bu)₂, and -P(=O)(OPh)₂.

Phosphoric acid (phosphonooxy): -OP(=O)(OH)₂.

Phosphate (phosphonooxy ester): -OP(=O)(OR)₂, where R is a phosphate substituent, for example, -H, a C₁₋₇ alkyl group, a C₃₋₂₀ heterocyclyl group, or a C₅₋₂₀ aryl group. In some embodiments, R is -H, a C₁₋₇ alkyl group, or a C₅₋₂₀ aryl group. Examples of phosphate groups include, -OP(=O)(OCH₃)₂, -OP(=O)(OCH₂CH₃)₂, -OP(=O)(O-t-Bu)₂, and -OP(=O)(OPh)₂.

Phosphorous acid: -OP(OH)₂

Phosphite: -OP(OR)₂, where R is a phosphite substituent, for example, -H, a C₁₋₇ alkyl group, a C₃₋₂₀ heterocyclyl group, or a C₅₋₂₀ aryl group. In some embodiments, R is - H, a C₁₋₇ alkyl group, or a C₅₋₂₀ aryl group. Examples of phosphite groups include, -OP(OCH₃)₂, -OP(OCH₂CH₃)₂, -OP(O-t-Bu)₂, and -OP(OPh)₂.

Phosphoramidite: -OP(OR¹)-NR²₂, where R¹ and R² are phosphoramidite substituents, for example, -H, a (optionally substituted) C₁₋₇ alkyl group, a C₃₋₂₀ heterocyclyl group, or a C₅₋₂₀ aryl group. In some embodiments, R is -H, a C₁₋₇ alkyl group, or a C₅₋₂₀ aryl group. Examples of phosphoramidite groups include, -OP(OCH₂CH₃)-N(CH₃)₂, -OP(OCH₂CH₃)-N(i-Pr)₂, and -OP(OCH₂CH₂CN)-N(i-Pr)₂.

Phosphoramidate: -OP(=O)(OR¹)-NR²₂, where R¹ and R² are phosphoramidate substituents, for example, -H, a (optionally substituted) C₁₋₇ alkyl group, a C₃₋₂₀ heterocyclyl group, or a C₅₋₂₀ aryl group. In some embodiments, R¹ and R² are -H, a C₁₋₇ alkyl group, or a C₅₋₂₀ aryl group. Examples of phosphoramidate groups include, -OP(=O)(OCH₂CH₃)-N(CH₃)₂, -OP(=O)(OCH₂CH₃)-N(i-Pr)₂, and -OP(=O)(OCH₂CH₂CN)-N(i-Pr)₂.

The term "C₃₋₁₂ alkylene", as used herein, pertains to a bidentate moiety obtained by removing two hydrogen atoms, either both from the same carbon atom, or one from each of two different carbon atoms, of a hydrocarbon compound having from 3 to 12 carbon atoms (unless otherwise specified), which is aliphatic, and which may be cyclic or acyclic, and which may be saturated, partially unsaturated, or fully unsaturated. Thus, the term "alkylene" includes the sub-classes alkenylene, alkynylene, cycloalkylene, etc., discussed below.

Examples of linear saturated C₃₋₁₂ alkylene groups include, but are not limited to, -(CH₂)ₙ- where n is an integer from 3 to 12, for example, -CH₂CH₂CH₂-(propylene), -CH₂CH₂CH₂CH₂- (butylene), -CH₂CH₂CH₂CH₂CH₂- (pentylene) and -CH₂CH₂CH₂CH₂CH₂CH₂CH₂- (heptylene).

Examples of branched saturated C₃₋₁₂ alkylene groups include, -CH(CH₃)CH₂-, -CH(CH₃)CH₂CH₂-, -CH(CH₃)CH₂CH₂CH₂-, -CH₂CH(CH₃)CH₂-, -CH₂C H(CH₃)CH₂CH₂-, -CH(CH₂CH₃)-, -CH(CH₂CH₃)CH₂-, and -CH₂CH(CH₂CH₃)CH₂-.

Examples of linear partially unsaturated C₃₋₁₂ alkylene groups (C₃₋₁₂ alkenylene, and alkynylene groups) include, -CH=CH-CH₂-, -CH₂-CH=CH₂-, -CH=CH-CH₂-CH₂-, -CH=CH-CH₂-CH₂-CH₂-, -CH=CH-CH=CH-, -CH=CH-CH=CH-CH₂-, -CH=CH-CH=CH-CH₂-CH₂-, -CH=CH-CH₂-CH=CH-, -CH=CH-CH₂-CH₂-CH=CH-, and -CH₂-C≡C-CH₂-.

Examples of branched partially unsaturated C₃₋₁₂ alkylene groups (C₃₋₁₂ alkenylene and alkynylene groups) include, -C(CH₃)=CH-, -C(CH₃)=CH-CH₂-, -CH=CH-CH(CH₃)- and -C≡C-CH(CH₃)-.

Examples of alicyclic saturated C₃₋₁₂ alkylene groups (C₃₋₁₂ cycloalkylenes) include, cyclopentylene (e.g. cyclopent-1,3-ylene), and cyclohexylene (e.g. cyclohex-1,4-ylene).

Examples of alicyclic partially unsaturated C₃₋₁₂ alkylene groups (C₃₋₁₂ cycloalkylenes) include, cyclopentenylene (e.g. 4-cyclopenten-1,3-ylene), cyclohexenylene (e.g. 2-cyclohexen-1,4-ylene; 3-cyclohexen-1,2-ylene; 2,5-cyclohexadien-1,4-ylene).

"Linker" refers to a chemical moiety comprising a covalent bond or a chain of atoms that covalently attaches an antibody to a drug moiety. Exemplary linkers are described herein.

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., Stereochemistry of Organic Compounds (1994) John Wiley & Sons, Inc., New York. Many organic compounds exist in optically active forms, i.e., they have the ability to rotate the plane of plane-polarized light. In describing an optically active compound, the prefixes D and L, or *R* and *S,* are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

"Leaving group" refers to a functional group that can be substituted by another functional group. Certain leaving groups are well known in the art, and examples include, a halide (*e.g.,* chloride, bromide, iodide), methanesulfonyl (mesyl), p-toluenesulfonyl (tosyl), trifluoromethylsulfonyl (triflate), and trifluoromethylsulfonate.

The term "protecting group" refers to a substituent that is commonly employed to block or protect a particular functionality while reacting other functional groups on the compound. For example, an "amino-protecting group" is a substituent attached to an amino group that blocks or protects the amino functionality in the compound. Suitable amino-protecting groups include, but are not limited to, acetyl, trifluoroacetyl, t-butoxycarbonyl (BOC), benzyloxycarbonyl (CBZ) and 9-fluorenylmethylenoxycarbonyl (Fmoc). For a general description of protecting groups and their use, see T. W. Greene, Protective Groups in Organic Synthesis, John Wiley & Sons, New York, 1991, or a later edition.

### II. COMPOSITIONS AND METHODS

In one aspect, the invention is based, in part, on antibodies that bind to CD79b and immunoconjugates comprising such antibodies. Antibodies and immunoconjugates of the invention are useful, e.g., for the diagnosis or treatment of CD79b-positive cancers.

### A. Exemplary Anti-CD79b Antibodies

Isolated antibodies that bind to CD79b are provided. CD79b heterodimerizes with CD79a to form CD79, a B-cell-restricted receptor. CD79b is expressed in various B-cell related disorders and cancers, including various lymphomas, such as Non-Hodgkin's lymphoma.

An exemplary naturally occurring human CD79b precursor sequence, with signal sequence (amino acids 1-28) is provided in SEQ ID NO: 40, and the corresponding mature CD79b sequence is shown in SEQ ID NO: 41 (corresponding to amino acids 29 to 229 of SEQ ID NO: 40).

In some embodiments, an anti-CD79b antibody binds human CD79b. In some embodiments, an anti-CD79b antibody binds human CD79b with an affinity of ≤ 10 nM, or ≤ 5 nM, or ≤ 4 nM, or ≤3 nM, or ≤ 2 nM and optionally ≥ 0.0001 nM, or ≥ 0.001 nM, or ≥ 0.01 nM. Exemplary such antibodies include huMA79bv28 and huMA79bv32, which bind to human CD79b with an affinity of 0.44 nM and 0.24 nM, respectively. See, e.g., US 8,088,378 B2.

### Assays

Whether an anti-CD79b antibody "binds with an affinity of" ≤ 10 nM, or ≤ 5 nM, or ≤ 4 nM, or ≤ 3 nM, or ≤ 2 nM, is determined using Scatchard analysis, as described, e.g., US 8,088,378 B2. Briefly, I¹²⁵ labeled antibody is competed against serial dilutions of unlabeled antibody in the presence of BJAB cells expressing human CD79b. Following the incubation, cells are washed and cell pellet counts read by a gamma counter. *See, e.g.,* US 8,088,378 B2. Binding affinity, K_{D}, of the antibodies may be determined in accordance with standard Scatchard analysis performed utilizing a non-linear curve fitting program (see, for example, Munson et al., Anal Biochem, 107: 220-239, 1980).

### Antibody MA79b and other embodiments

The invention discloses an anti-CD79b antibody or immunoconjugate comprising at least one, two, three, four, five, or six HVRs selected from (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 17 or SEQ ID NO: 23; (d) HVR-L1 comprising an amino acid sequence selected from SEQ ID NOs: 18, 24, and 35; (e) HVR-L2 comprising the amino acid sequence of SEQ ID NO: 25; and (f) HVR-L3 comprising the amino acid sequence of SEQ ID NO: 26. In some such embodiments, the antibody or immunoconjugate comprises at least one of: (i) HVR-H3 comprising the amino acid sequence of SEQ ID NO: 23, and/or (ii) HVR-L1 comprising an amino acid sequence selected from SEQ ID NOs: 24 and 35.

The invention discloses an anti-CD79b antibody or immunoconjugate comprising at least one, two, three, four, five, or six HVRs selected from (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 23; (d) HVR-L1 comprising an amino acid sequence of SEQ ID NO: 24; (e) HVR-L2 comprising the amino acid sequence of SEQ ID NO: 25; and (f) HVR-L3 comprising the amino acid sequence of SEQ ID NO: 26. In some such embodiments, the antibody or immunoconjugate comprises at least one of: (i) HVR-H3 comprising the amino acid sequence of SEQ ID NO: 23, and/or (ii) HVR-L1 comprising the amino acid sequence of SEQ ID NO: 24.

The invention discloses an antibody or immunoconjugate comprising at least one, at least two, or all three VH HVR sequences selected from (a) HVR-H1 comprising the amino acid sequence of SEQ ID NO: 21; (b) HVR-H2 comprising the amino acid sequence of SEQ ID NO: 22; and (c) HVR-H3 comprising the amino acid sequence of SEQ ID NO: 23. In some embodiments, the antibody comprises HVR-H3 comprising the amino acid sequence of SEQ ID NO: 23. In another embodiment, the antibody comprises HVR-H3 comprising the amino acid sequence of SEQ ID NO: 23 and HVR-L3 comprising the amino acid sequence of SEQ ID NO: 26. In a further embodiment, the antibody comprises HVR-H3 comprising the amino acid sequence of SEQ ID NO: 23, HVR-L3 comprising the amino acid sequence of SEQ ID NO: 26, and HVR-H2 comprising the amino acid sequence of SEQ ID NO: 22. In a further embodiment, the antibody comprises (a) HVR-H1 comprising the amino acid sequence of SEQ ID NO: 21; (b) HVR-H2 comprising the amino acid sequence of SEQ ID NO: 22; and (c) HVR-H3 comprising the amino acid sequence of SEQ ID NO: 23.

In another aspect, the invention provides an antibody or immunoconjugate comprising at least one, at least two, or all three VL HVR sequences selected from (a) HVR-L1 comprising an amino acid sequence selected from SEQ ID NOs: 18, 24, and 35; (b) HVR-L2 comprising the amino acid sequence of SEQ ID NO: 25; and (c) HVR-L3 comprising the amino acid sequence of SEQ ID NO: 26. In another aspect, the invention provides an antibody or immunoconjugate comprising at least one, at least two, or all three VL HVR sequences selected from (a) HVR-L1 comprising the amino acid sequence of SEQ ID NO: 24; (b) HVR-L2 comprising the amino acid sequence of SEQ ID NO: 25; and (c) HVR-L3 comprising the amino acid sequence of SEQ ID NO: 26. In one embodiment, the antibody comprises (a) HVR-L1 comprising an amino acid sequence selected from SEQ ID NOs: 24 and 35; (b) HVR-L2 comprising the amino acid sequence of SEQ ID NO: 25; and (c) HVR-L3 comprising the amino acid sequence of SEQ ID NO: 26. In some embodiments, the antibody comprises an HVR-L1 comprising the amino acid sequence of SEQ ID NO: 24 or SEQ ID NO: 35. In some embodiments, the antibody comprises an HVR-L1 comprising the amino acid sequence of SEQ ID NO: 24. In some embodiments, the antibody comprises (a) HVR-L1 comprising the amino acid sequence of SEQ ID NO: 24; (b) HVR-L2 comprising the amino acid sequence of SEQ ID NO: 25; and (c) HVR-L3 comprising the amino acid sequence of SEQ ID NO: 26. In some embodiments, the antibody comprises (a) HVR-L1 comprising the amino acid sequence of SEQ ID NO: 35; (b) HVR-L2 comprising the amino acid sequence of SEQ ID NO: 25; and (c) HVR-L3 comprising the amino acid sequence of SEQ ID NO: 26.

In another aspect, an antibody or immunoconjugate comprises (a) a VH domain comprising at least one, at least two, or all three VH HVR sequences selected from (i) HVR-H1 comprising the amino acid sequence of SEQ ID NO: 21, (ii) HVR-H2 comprising the amino acid sequence of SEQ ID NO: 22, and (iii) HVR-H3 comprising an amino acid sequence selected from SEQ ID NOs: 17 and 23; and (b) a VL domain comprising at least one, at least two, or all three VL HVR sequences selected from (i) HVR-L1 comprising an amino acid sequence selected from SEQ ID NOs: 18, 24, and 35, (ii) HVR-L2 comprising the amino acid sequence of SEQ ID NO: 25, and (iii) HVR-L3 comprising the amino acid sequence of SEQ ID NO: 26. In some such embodiments, the antibody or immunoconjugate comprises at least one of: (i) HVR-H3 comprising the amino acid sequence of SEQ ID NO: 23, and/or (ii) HVR-L1 comprising the amino acid sequence of SEQ ID NO: 24 or SEQ ID NO: 35.

In another aspect, the invention provides an antibody or immunoconjugate comprising (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 17 or SEQ ID NO: 23; (d) HVR-L1 comprising an amino acid sequence selected from SEQ ID NOs: 18, 24, and 35; (e) HVR-L2 comprising the amino acid sequence of SEQ ID NO: 25; and (f) HVR-L3 comprising the amino acid sequence of SEQ ID NO: 26. In some such embodiments, the antibody or immunoconjugate comprises at least one of: HVR-H3 comprising the amino acid sequence of SEQ ID NO: 23 and/or HVR-L1 comprising an amino acid sequence selected from SEQ ID NOs: 24 and 35. In another aspect, the invention provides an antibody or immunoconjugate comprising (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 23; (d) HVR-L1 comprising the amino acid sequence of SEQ ID NO: 24; (e) HVR-L2 comprising the amino acid sequence of SEQ ID NO: 25; and (f) HVR-L3 comprising the amino acid sequence of SEQ ID NO: 26.

In any of the above embodiments, an anti-CD79b antibody is humanized. In one embodiment, an anti-CD79b antibody comprises HVRs as in any of the above embodiments, and further comprises a human acceptor framework, e.g. a human immunoglobulin framework or a human consensus framework. In certain embodiments, the human acceptor framework is the human VL kappa 1 (VL_{KI}) framework and/or the VH framework VH_{III}. In some embodiments, a humanized anti-CD79b antibody comprises (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 17 or SEQ ID NO: 23; (d) HVR-L1 comprising an amino acid sequence selected from SEQ ID NOs: 18, 24, and 35; (e) HVR-L2 comprising the amino acid sequence of SEQ ID NO: 25; and (f) HVR-L3 comprising the amino acid sequence of SEQ ID NO: 26. In some embodiments, a humanized anti-CD79b antibody comprises (a) HVR-H1 comprising the amino acid sequence of SEQ ID NO: 21; (b) HVR-H2 comprising the amino acid sequence of SEQ ID NO: 22; (c) HVR-H3 comprising the amino acid sequence of SEQ ID NO: 23; (d) HVR-L1 comprising the amino acid sequence of SEQ ID NO: 24; (e) HVR-L2 comprising the amino acid sequence of SEQ ID NO: 25; and (f) HVR-L3 comprising the amino acid sequence of SEQ ID NO: 26.

In another aspect, an anti-CD79b antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO: 11. In certain embodiments, a VH sequence having at least 90%, 91%, 92%, 93%, 94%, 95%, 96%, 97%, 98%, or 99% identity to the amino acid sequence of SEQ ID NO: 11 contains substitutions (e.g., conservative substitutions), insertions, or deletions relative to the reference sequence, but an anti-CD79b antibody comprising that sequence retains the ability to bind to CD79b. In certain embodiments, a total of 1 to 10 amino acids have been substituted, inserted and/or deleted in SEQ ID NO: 11. In certain embodiments, a total of 1 to 5 amino acids have been substituted, inserted and/or deleted in SEQ ID NO: 11. In certain embodiments, substitutions, insertions, or deletions occur in regions outside the HVRs (i.e., in the FRs).

Optionally, the anti-CD79b antibody comprises the VH sequence of any one of SEQ ID NOs: 5, 7, 9, 11, and 13, including post-translational modifications of that sequence. In some embodiments, the anti-CD79b antibody comprises the VH sequence of SEQ ID NO: 11, including post-translational modifications of that sequence. In a particular embodiment, the VH comprises one, two or three HVRs selected from: (a) HVR-H1 comprising the amino acid sequence of SEQ ID NO: 21, (b) HVR-H2 comprising the amino acid sequence of SEQ ID NO: 22, and (c) HVR-H3 comprising the amino acid sequence of SEQ ID NO: 17 or SEQ ID NO: 23.

In some embodiments, an anti-CD79b antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO: 12. In certain embodiments, a VL sequence having at least 90%, 91%, 92%, 93%, 94%, 95%, 96%, 97%, 98%, or 99% identity to the amino acid sequence of SEQ ID NO: 12 contains substitutions (e.g., conservative substitutions), insertions, or deletions relative to the reference sequence, but an anti-CD79b antibody comprising that sequence retains the ability to bind to CD79b. In certain embodiments, a total of 1 to 10 amino acids have been substituted, inserted and/or deleted in SEQ ID NO: 12. In certain embodiments, a total of 1 to 5 amino acids have been substituted, inserted and/or deleted in SEQ ID NO: 12. In certain embodiments, the substitutions, insertions, or deletions occur in regions outside the HVRs (i.e., in the FRs). Optionally, the anti-CD79b antibody comprises the VL sequence of any one of SEQ ID NOs: 6, 8, 10, 12, and 14, including post-translational modifications of that sequence. In some embodiments, the anti-CD79b antibody comprises the VL sequence of SEQ ID NO: 12, including post-translational modifications of that sequence. In a particular embodiment, the VL comprises one, two or three HVRs selected from (a) HVR-L1 comprising an amino acid sequence selected from SEQ ID NOs: 18, 24, and 35; (b) HVR-L2 comprising the amino acid sequence of SEQ ID NO: 25; and (c) HVR-L3 comprising the amino acid sequence of SEQ ID NO: 26. In some embodiments, the VL comprises one, two or three HVRs selected from (a) HVR-L1 comprising the amino acid sequence of SEQ ID NO: 24 or SEQ ID NO: 35; (b) HVR-L2 comprising the amino acid sequence of SEQ ID NO: 25; and (c) HVR-L3 comprising the amino acid sequence of SEQ ID NO: 26.

In another aspect, an anti-CD79b antibody is provided, wherein the antibody comprises a VH as in any of the embodiments provided above, and a VL as in any of the embodiments provided above. In some embodiments, the antibody comprises the VH and VL sequences in SEQ ID NO: 11 and SEQ ID NO: 12, respectively, including post-translational modifications of those sequences. In some embodiments, the antibody comprises the VH and VL sequences in SEQ ID NO: 13 and SEQ ID NO: 14, respectively, including post-translational modifications of those sequences. In some embodiments, the antibody comprises the VH and VL sequences in SEQ ID NO: 9 and SEQ ID NO: 10, respectively, including post-translational modifications of those sequences. In some embodiments, the antibody comprises the VH and VL sequences in SEQ ID NO: 7 and SEQ ID NO: 8, respectively, including post-translational modifications of those sequences. In some embodiments, the antibody comprises the heavy chain and light chain sequences in SEQ ID NO: 38 and SEQ ID NO: 37, respectively, including post-translational modifications of those sequences. In some embodiments, the antibody comprises the heavy chain and light chain sequences in SEQ ID NO: 39 and SEQ ID NO: 37, respectively, including post-translational modifications of those sequences. In some embodiments, the antibody comprises the heavy chain and light chain sequences in SEQ ID NO: 38 and SEQ ID NO: 54, respectively, including post-translational modifications of those sequences. In some embodiments, the antibody comprises the heavy chain and light chain sequences in SEQ ID NO: 55 and SEQ ID NO: 37, respectively, including post-translational modifications of those sequences.

In a further aspect, the invention provides an antibody or immunoconjugate that binds to the same epitope as an anti-CD79b antibody provided herein. For example, in certain embodiments, an antibody or immunoconjugate is provided that binds to the same epitope as an anti-CD79b antibody comprising a VH sequence of SEQ ID NO: 11 and a VL sequence of SEQ ID NO: 12.

In a further aspect of the invention, an anti-CD79b antibody according to any of the above embodiments is a monoclonal antibody, including a chimeric, humanized or human antibody. In one embodiment, an anti-CD79b antibody is an antibody fragment, e.g., a Fv, Fab, Fab', scFv, diabody, or F(ab')₂ fragment. In another embodiment, the antibody is a substantially full length antibody, e.g., an IgG1 antibody or other antibody class or isotype as defined herein.

In any of the immunoconjugates described above, the antibody may be conjugated to a drug moiety. In some embodiments, the antibody is conjugated to a cytotoxic agent. In some such embodiments, the cytotoxic agent is a pyrrolobenzodiazepine (PBD), such as a PBD dimer. Various exemplary PBD dimers are discussed herein.

In a further aspect, an anti-CD79b antibody or immunoconjugate according to any of the above embodiments may incorporate any of the features, singly or in combination, as described in Sections 1-7 below.

### 1. Antibody Affinity

In certain embodiments, an antibody provided herein has a dissociation constant (Kd) of ≤ 1µM, ≤ 100 nM, ≤ 10 nM, ≤ 1 nM, ≤ 0.1 nM, ≤ 0.01 nM, or ≤ 0.001 nM, and optionally is ≥ 10⁻¹³ M. (e.g. 10⁻⁸ M or less, e.g. from 10⁻⁸ M to 10⁻¹³ M, e.g., from 10⁻⁹ M to 10⁻¹³ M).

In one embodiment, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER^{®} multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20^{®}) in PBS. When the plates have dried, 150 µl/well of scintillant (MICROSCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE^{®}-2000 or a BIACORE^{®}-3000 (BIAcore, Inc., Piscataway, NJ) at 25°C with immobilized antigen CM5 chips at ∼10 response units (RU). Briefly, carboxymethylated dextran biosensor chips (CM5, BIACORE, Inc.) are activated with *N*-ethyl-*N'*-(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 µl/min. Association rates (kₒₙ) and dissociation rates (k_{off}) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio k_{off}/kₒₙ. See, e.g., Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ™ spectrophotometer (ThermoSpectronic) with a stirred cuvette.

### 2. Antibody Fragments

In certain embodiments, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for CD79b and the other is for any other antigen. In certain embodiments, bispecific antibodies may bind to two different epitopes of CD79b. Bispecific antibodies may also be used to localize cytotoxic agents to cells which express CD79b. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to CD79b as well as another, different antigen (see, US 2008/0069820, for example).

### 7. Antibody Variants

Amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

Antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE 1**

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g.* a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex is used to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c) Fc region variants

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the invention contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96^{®} non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). Clq binding assays may also be carried out to confirm that the antibody is unable to bind Clq and hence lacks CDC activity. See, e.g., Clq and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either improved or diminished) Clq binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Nonlimiting exemplary cysteine engineered heavy chains and light chains of a huMA79bv28 antibody are shown in Figure 4 (SEQ ID NOs: 39, 54, and 55). Cysteine engineered antibodies may be generated as described, e.g., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Antibodies may be produced using recombinant methods and compositions, e.g., as described in U.S. Patent No. 4,816,567. In one embodiment, isolated nucleic acid encoding an anti-CD79b antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp20 cell). In one embodiment, a method of making an anti-CD79b antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an anti-CD79b antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

### C. Assays

Anti-CD79b antibodies provided herein may be identified, screened for, or characterized for their physical/chemical properties and/or biological activities by various assays known in the art.

In one aspect, an antibody is tested for its antigen binding activity, e.g., by known methods such as ELISA, BIACore^{®}, FACS, or Western blot.

In another aspect, competition assays may be used to identify an antibody that competes with any of the antibodies described herein for binding to CD79b. In certain embodiments, such a competing antibody binds to the same epitope (e.g., a linear or a conformational epitope) that is bound by an antibody described herein. Detailed exemplary methods for mapping an epitope to which an antibody binds are provided in Morris (1996) "Epitope Mapping Protocols," in Methods in Molecular Biology vol. 66 (Humana Press, Totowa, NJ).

In an exemplary competition assay, immobilized CD79b is incubated in a solution comprising a first labeled antibody that binds to CD79b (e.g., murine MA79b antibody, humanized MA79b.v17 antibody and/or humanized MA79b.v18 antibody and/or humanized MA79b.v28 and/or humanized MA79b.v32) and a second unlabeled antibody that is being tested for its ability to compete with the first antibody for binding to CD79b. The second antibody may be present in a hybridoma supernatant. As a control, immobilized CD79b is incubated in a solution comprising the first labeled antibody but not the second unlabeled antibody. After incubation under conditions permissive for binding of the first antibody to CD79b, excess unbound antibody is removed, and the amount of label associated with immobilized CD79b is measured. If the amount of label associated with immobilized CD79b is substantially reduced in the test sample relative to the control sample, then that indicates that the second antibody is competing with the first antibody for binding to CD79b. In certain embodiments, immobilized CD79b is present on the surface of a cell or in a membrane preparation obtained from a cell expressing CD79b on its surface. *See* Harlow and Lane (1988) Antibodies: A Laboratory Manual ch.14 (Cold Spring Harbor Laboratory, Cold Spring Harbor, NY).

### D. Immunoconjugates

The invention also discloses immunoconjugates comprising an anti-CD79b antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes (i.e., a radioconjugate).

Immunoconjugates allow for the targeted delivery of a drug moiety to a tumor, and, in some embodiments intracellular accumulation therein, where systemic administration of unconjugated drugs may result in unacceptable levels of toxicity to normal cells (Polakis P. (2005) Current Opinion in Pharmacology 5:382-387).

Antibody-drug conjugates (ADC) are targeted chemotherapeutic molecules which combine properties of both antibodies and cytotoxic drugs by targeting potent cytotoxic drugs to antigen-expressing tumor cells (Teicher, B.A. (2009) Current Cancer Drug Targets 9:982-1004), thereby enhancing the therapeutic index by maximizing efficacy and minimizing off-target toxicity (Carter, P.J. and Senter P.D. (2008) The Cancer Jour. 14(3):154-169; Chari, R.V. (2008) Acc. Chem. Res. 41:98-107.

The ADC compounds of the invention include those with anticancer activity. In some embodiments, the ADC compounds include an antibody conjugated, i.e. covalently attached, to the drug moiety. In some embodiments, the antibody is covalently attached to the drug moiety through a linker. The antibody-drug conjugates (ADC) of the invention selectively deliver an effective dose of a drug to tumor tissue whereby greater selectivity, i.e. a lower efficacious dose, may be achieved while increasing the therapeutic index ("therapeutic window").

The drug moiety (D) of the antibody-drug conjugates (ADC) may include any compound, moiety or group that has a cytotoxic or cytostatic effect. Exemplary drug moieties include, but are not limited to, pyrrolobenzodiazepine (PBD) and derivatives thereof that have cytotoxic activity. Nonlimiting examples of such immunoconjugates are discussed in further detail below.

### 1. Exemplary Antibody-drug Conjugates

An exemplary embodiment of an antibody-drug conjugate (ADC) compound comprises an antibody (Ab) which targets a tumor cell, a drug moiety (D), and a linker moiety (L) that attaches Ab to D. In some embodiments, the antibody is attached to the linker moiety (L) through one or more amino acid residues, such as lysine and/or cysteine.

An exemplary ADC has Formula I:

Ab-(L-D)ₚ **I**

where p is 1 to about 20. In some embodiments, the number of drug moieties that can be conjugated to an antibody is limited by the number of free cysteine residues. In some embodiments, free cysteine residues are introduced into the antibody amino acid sequence by the methods described herein. Exemplary ADC of Formula I include, but are not limited to, antibodies that have 1, 2, 3, or 4 engineered cysteine amino acids (Lyon, R. et al (2012) Methods in Enzym. 502:123-138). In some embodiments, one or more free cysteine residues are already present in an antibody, without the use of engineering, in which case the existing free cysteine residues may be used to conjugate the antibody to a drug. In some embodiments, an antibody is exposed to reducing conditions prior to conjugation of the antibody in order to generate one or more free cysteine residues.

### a) Exemplary Linkers

A "Linker" (L) is a bifunctional or multifunctional moiety that can be used to link one or more drug moieties (D) to an antibody (Ab) to form an antibody-drug conjugate (ADC) of Formula I. In some embodiments, antibody-drug conjugates (ADC) can be prepared using a Linker having reactive functionalities for covalently attaching to the drug and to the antibody. For example, in some embodiments, a cysteine thiol of an antibody (Ab) can form a bond with a reactive functional group of a linker or a drug-linker intermediate to make an ADC.

In one aspect, a linker has a functionality that is capable of reacting with a free cysteine present on an antibody to form a covalent bond. Nonlimiting exemplary such reactive functionalities include maleimide, haloacetamides, α-haloacetyl, activated esters such as succinimide esters, 4-nitrophenyl esters, pentafluorophenyl esters, tetrafluorophenyl esters, anhydrides, acid chlorides, sulfonyl chlorides, isocyanates, and isothiocyanates. *See, e.g.,* the conjugation method at page 766 of Klussman, et al (2004), Bioconjugate Chemistry 15(4):765-773, and the Examples herein.

In some embodiments, a linker has a functionality that is capable of reacting with an electrophilic group present on an antibody. Exemplary such electrophilic groups include, but are not limited to, aldehyde and ketone carbonyl groups. In some embodiments, a heteroatom of the reactive functionality of the linker can react with an electrophilic group on an antibody and form a covalent bond to an antibody unit. Nonlimiting exemplary such reactive functionalities include, but are not limited to, hydrazide, oxime, amino, hydrazine, thiosemicarbazone, hydrazine carboxylate, and arylhydrazide.

A linker may comprise one or more linker components. Exemplary linker components include 6-maleimidocaproyl ("MC"), maleimidopropanoyl ("MP"), valine-citrulline ("val-cit" or "vc"), alanine-phenylalanine ("ala-phe"), p-aminobenzyloxycarbonyl (a "PAB"), N-Succinimidyl 4-(2-pyridylthio) pentanoate ("SPP"), and 4-(N-maleimidomethyl) cyclohexane-1 carboxylate ("MCC"). Various linker components are known in the art, some of which are described below.

A linker may be a "cleavable linker," facilitating release of a drug. Nonlimiting exemplary cleavable linkers include acid-labile linkers (e.g., comprising hydrazone), protease-sensitive (e.g., peptidase-sensitive) linkers, photolabile linkers, or disulfide-containing linkers (Chari et al., Cancer Research 52:127-131 (1992); US 5208020).

In certain embodiments, a linker has the following Formula II:

-Aₐ-W_{w}-Y_{y}- **II**

wherein A is a "stretcher unit", and a is an integer from 0 to 1; W is an "amino acid unit", and w is an integer from 0 to 12; Y is a "spacer unit", and y is 0, 1, or 2. An ADC comprising the linker of Formula II has the Formula I(A): Ab-(Aₐ-W_{w}-Y_{y}-D)ₚ, wherein Ab, D, and p are defined as above for Formula I. Exemplary embodiments of such linkers are described in U.S. Patent No. 7,498,298, which is expressly incorporated herein by reference.

In some embodiments, a linker component comprises a "stretcher unit" (A) that links an antibody to another linker component or to a drug moiety. Nonlimiting exemplary stretcher units are shown below (wherein the wavy line indicates sites of covalent attachment to an antibody, drug, or additional linker components):

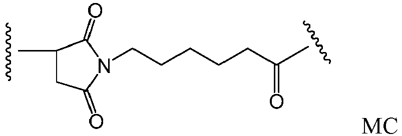

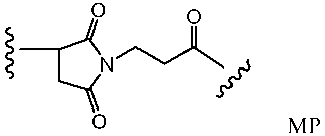

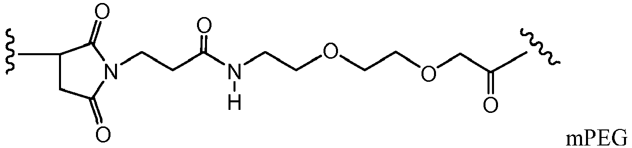

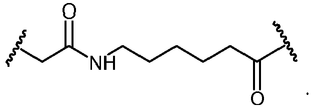

In some embodiments, a linker component comprises an "amino acid unit" (W). In some such embodiments, the amino acid unit allows for cleavage of the linker by a protease, thereby facilitating release of the drug from the immunoconjugate upon exposure to intracellular proteases, such as lysosomal enzymes (Doronina et al. (2003) Nat. Biotechnol. 21:778-784). Exemplary amino acid units include, dipeptides, tripeptides, tetrapeptides, and pentapeptides. Exemplary dipeptides include, valine-citrulline (vc or val-cit), alanine-phenylalanine (af or ala-phe); phenylalanine-lysine (fk or phe-lys); phenylalanine-homolysine (phe-homolys); and N-methyl-valine-citrulline (Me-val-cit). Exemplary tripeptides include, glycine-valine-citrulline (gly-val-cit) and glycine-glycine-glycine (gly-gly-gly). An amino acid unit may comprise amino acid residues that occur naturally and/or minor amino acids and/or non-naturally occurring amino acid analogs, such as citrulline. Amino acid units can be designed and optimized for enzymatic cleavage by a particular enzyme, for example, a tumor-associated protease, cathepsin B, C and D, or a plasmin protease.

Typically, peptide-type linkers can be prepared by forming a peptide bond between two or more amino acids and/or peptide fragments. Such peptide bonds can be prepared, for example, according to a liquid phase synthesis method (e.g., E. Schröder and K. Lübke (1965) "The Peptides", volume 1, pp 76-136, Academic Press).

In some embodiments, a linker component comprises a "spacer" unit that links the antibody to a drug moiety, either directly or through a stretcher unit and/or an amino acid unit. A spacer unit may be "self-immolative" or a "non-self-immolative." A "non-self-immolative" spacer unit is one in which part or all of the spacer unit remains bound to the drug moiety upon cleavage of the ADC. Examples of non-self-immolative spacer units include, but are not limited to, a glycine spacer unit and a glycine-glycine spacer unit. In some embodiments, enzymatic cleavage of an ADC containing a glycine-glycine spacer unit by a tumor-cell associated protease results in release of a glycine-glycine-drug moiety from the remainder of the ADC. In some such embodiments, the glycine-glycine-drug moiety is subjected to a hydrolysis step in the tumor cell, thus cleaving the glycine-glycine spacer unit from the drug moiety.

A "self-immolative" spacer unit allows for release of the drug moiety. In certain embodiments, a spacer unit of a linker comprises a p-aminobenzyl unit. In some such embodiments, a p-aminobenzyl alcohol is attached to an amino acid unit via an amide bond, and a carbamate, methylcarbamate, or carbonate is made between the benzyl alcohol and the drug (Hamann et al. (2005) Expert Opin. Ther. Patents (2005) 15:1087-1103). In some embodiments, the spacer unit comprises p-aminobenzyloxycarbonyl (PAB). In some embodiments, an ADC comprising a self-immolative linker has the structure: 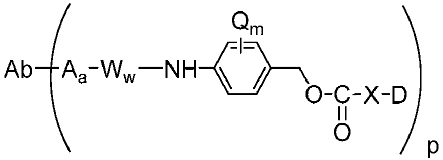 wherein Q is -C₁-C₈ alkyl, -O-(C₁-C₈ alkyl), -halogen, -nitro, or -cyano; m is an integer ranging from 0 to 4; X may be one or more additional spacer units or may be absent; and p ranges from 1 to about 20. In some embodiments, p ranges from 1 to 10, 1 to 7, 1 to 5, or 1 to 4. Exemplary X spacer units include: 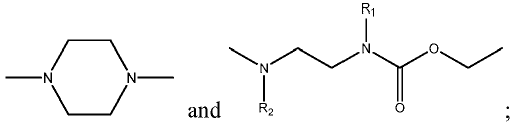 wherein R₁ and R₂ are independently selected from H and C₁-C₆ alkyl. In some embodiments, R1 and R2 are each - CH₃.

Other examples of self-immolative spacers include, aromatic compounds that are electronically similar to the PAB group, such as 2-aminoimidazol-5-methanol derivatives (U.S. Patent No. 7,375,078; Hay et al. (1999) Bioorg. Med. Chem. Lett. 9:2237) and ortho- or para-aminobenzylacetals. In some embodiments, spacers can be used that undergo cyclization upon amide bond hydrolysis, such as substituted and unsubstituted 4-aminobutyric acid amides (Rodrigues et al (1995) Chemistry Biology 2:223), appropriately substituted bicyclo[2.2.1] and bicyclo[2.2.2] ring systems (Storm et al (1972) J. Amer. Chem. Soc. 94:5815) and 2-aminophenylpropionic acid amides (Amsberry, et al (1990) J. Org. Chem. 55:5867). Linkage of a drug to the α-carbon of a glycine residue is another example of a self-immolative spacer that may be useful in ADC (Kingsbury et al (1984) J. Med. Chem. 27:1447).

In some embodiments, linker L may be a dendritic type linker for covalent attachment of more than one drug moiety to an antibody through a branching, multifunctional linker moiety (Sun et al (2002) Bioorganic & Medicinal Chemistry Letters 12:2213-2215; Sun et al (2003) Bioorganic & Medicinal Chemistry 11:1761-1768). Dendritic linkers can increase the molar ratio of drug to antibody, i.e. loading, which is related to the potency of the ADC. Thus, where an antibody bears only one reactive cysteine thiol group, a multitude of drug moieties may be attached through a dendritic linker.

Exemplary linkers are shown below in the context of an ADC of Formula I: 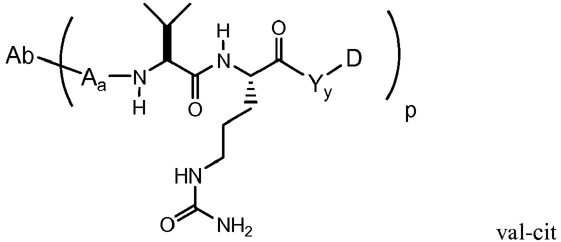 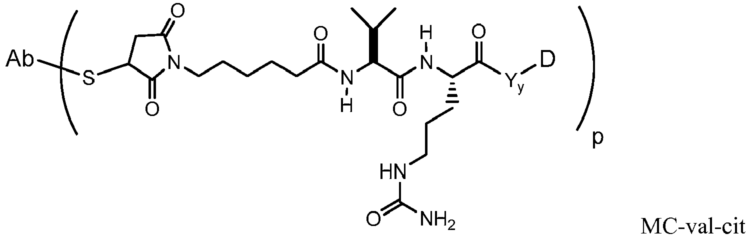 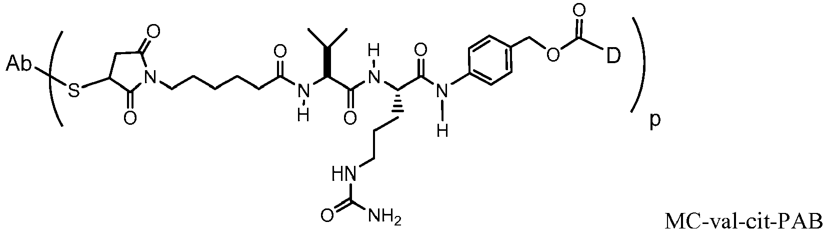 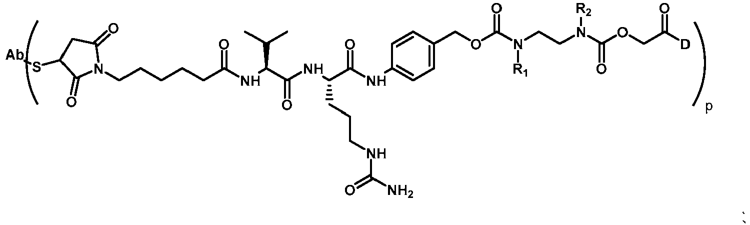 wherein R₁ and R₂ are independently selected from H and C₁-C₆ alkyl. In some embodiments, R1 and R2 are each -CH₃.

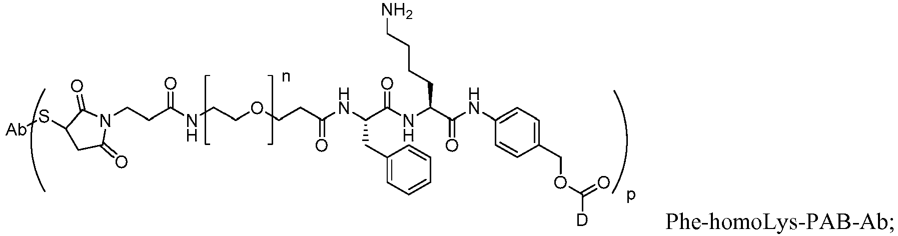

wherein n is 0 to 12. In some embodiments, n is 2 to 10. In some embodiments, n is 4 to 8.

Further exemplary ADCs include the structures: 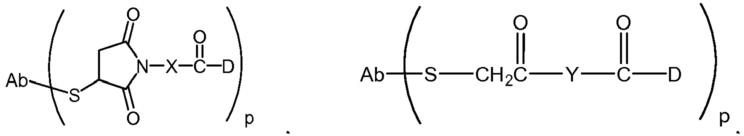 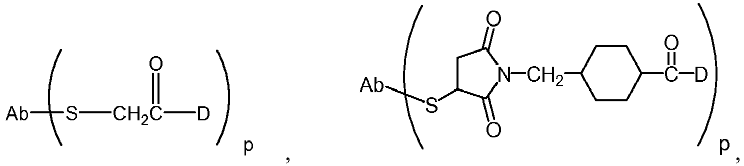 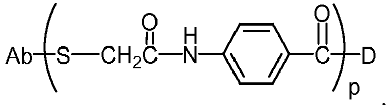 where X is: 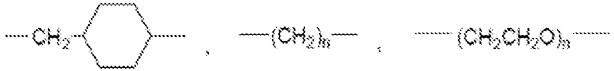 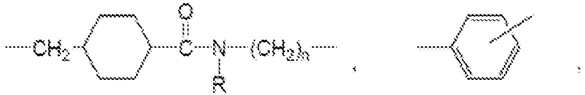 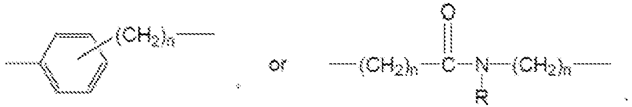 Y is: 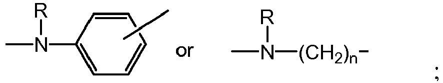 each R is independently H or C₁-C₆ alkyl; and n is 1 to 12.

In some embodiments, a linker is substituted with groups that modulate solubility and/or reactivity. As an example, a charged substituent such as sulfonate (-SO₃⁻) or ammonium may increase water solubility of the linker reagent and facilitate the coupling reaction of the linker reagent with the antibody and/or the drug moiety, or facilitate the coupling reaction of Ab-L (antibody-linker intermediate) with D, or D-L (drug-linker intermediate) with Ab, depending on the synthetic route employed to prepare the ADC. In some embodiments, a portion of the linker is coupled to the antibody and a portion of the linker is coupled to the drug, and then the Ab-(linker portion)^{a} is coupled to drug-(linker portion)^{b} to form the ADC of Formula I.

The compounds of the invention expressly contemplate ADC prepared with the following linker reagents: bis-maleimido-trioxyethylene glycol (BMPEO), N-(β-maleimidopropyloxy)-N-hydroxy succinimide ester (BMPS), N-(ε-maleimidocaproyloxy) succinimide ester (EMCS), N-[γ-maleimidobutyryloxy]succinimide ester (GMBS), 1,6-hexane-bis-vinylsulfone (HBVS), succinimidyl 4-(N-maleimidomethyl)cyclohexane-1-carboxy-(6-amidocaproate) (LC-SMCC), m-maleimidobenzoyl-N-hydroxysuccinimide ester (MBS), 4-(4-N-Maleimidophenyl)butyric acid hydrazide (MPBH), succinimidyl 3-(bromoacetamido)propionate (SBAP), succinimidyl iodoacetate (SIA), succinimidyl (4-iodoacetyl)aminobenzoate (SIAB), N-succinimidyl-3-(2-pyridyldithio) propionate (SPDP), N-succinimidyl-4-(2-pyridylthio)pentanoate (SPP), succinimidyl 4-(N-maleimidomethyl)cyclohexane-1-carboxylate (SMCC), succinimidyl 4-(p-maleimidophenyl)butyrate (SMPB), succinimidyl 6-[(beta-maleimidopropionamido)hexanoate] (SMPH), iminothiolane (IT), sulfo-EMCS, sulfo-GMBS, sulfo-KMUS, sulfo-MBS, sulfo-SIAB, sulfo-SMCC, and sulfo-SMPB, and succinimidyl-(4-vinylsulfone)benzoate (SVSB), and including bis-maleimide reagents: dithiobismaleimidoethane (DTME), 1,4-Bismaleimidobutane (BMB), 1,4 Bismaleimidyl-2,3-dihydroxybutane (BMDB), bismaleimidohexane (BMH), bismaleimidoethane (BMOE), BM(PEG)₂ (shown below), and BM(PEG)₃ (shown below); bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). In some embodiments, bis-maleimide reagents allow the attachment of the thiol group of a cysteine in the antibody to a thiol-containing drug moiety, linker, or linker-drug intermediate. Other functional groups that are reactive with thiol groups include, but are not limited to, iodoacetamide, bromoacetamide, vinyl pyridine, disulfide, pyridyl disulfide, isocyanate, and isothiocyanate.

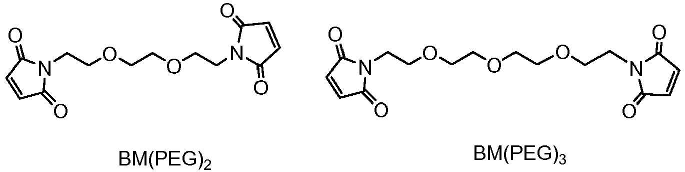

Certain useful linker reagents can be obtained from various commercial sources, such as Pierce Biotechnology, Inc. (Rockford, IL), Molecular Biosciences Inc.(Boulder, CO), or synthesized in accordance with procedures described in the art; for example, in Toki et al (2002) J. Org. Chem. 67:1866-1872; Dubowchik, et al. (1997) Tetrahedron Letters, 38:5257-60; Walker, M.A. (1995) J. Org. Chem. 60:5352-5355; Frisch et al (1996) Bioconjugate Chem. 7:180-186; US 6214345; WO 02/088172; US 2003130189; US2003096743; WO 03/026577; WO 03/043583; and WO 04/032828.

Carbon-14-labeled 1-isothiocyanatobenzyl-3-methyldiethylene triaminepentaacetic acid (MX-DTPA) is an exemplary chelating agent for conjugation of radionucleotide to the antibody. See, e.g., WO94/11026.

### b) Exemplary Drug Moieties

In some embodiments, an ADC comprises a pyrrolobenzodiazepine (PBD). In some embodiments, PBD dimers recognize and bind to specific DNA sequences. The natural product anthramycin, a PBD, was first reported in 1965 (Leimgruber, et al., (1965) J. Am. Chem. Soc., 87:5793-5795; Leimgruber, et al., (1965) J. Am. Chem. Soc., 87:5791-5793). Since then, a number of PBDs, both naturally-occurring and analogues, have been reported (Thurston, et al., (1994) Chem. Rev. 1994, 433-465 including dimers of the tricyclic PBD scaffold (US 6884799; US 7049311; US 7067511; US 7265105; US 7511032; US 7528126; US 7557099). Without intending to be bound by any particular theory, it is believed that the dimer structure imparts the appropriate three-dimensional shape for isohelicity with the minor groove of B-form DNA, leading to a snug fit at the binding site (Kohn, In Antibiotics III. Springer-Verlag, New York, pp. 3-11 (1975); Hurley and Needham-VanDevanter, (1986) Acc. Chem. Res., 19:230-237). Dimeric PBD compounds bearing C2 aryl substituents have been shown to be useful as cytotoxic agents (Hartley et al (2010) Cancer Res. 70(17):6849-6858; Antonow (2010) J. Med. Chem. 53(7):2927-2941; Howard et al (2009) Bioorganic and Med. Chem. Letters 19(22):6463-6466).

PBD dimers have been conjugated to antibodies and the resulting ADC shown to have anti-cancer properties. Nonlimiting exemplary linkage sites on the PBD dimer include the five-membered pyrrolo ring, the tether between the PBD units, and the N10-C11 imine group (WO 2009/016516; US 2009/304710; US 2010/047257; US 2009/036431; US 2011/0256157; WO 2011/130598).

Exemplary PBD dimer components of ADCs are of Formula A: 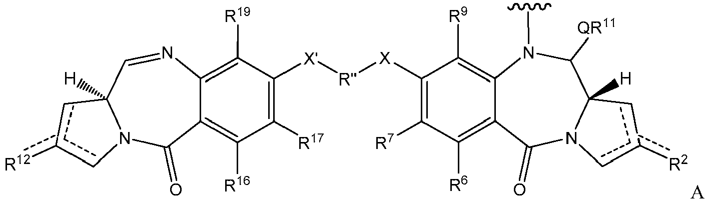 and salts and solvates thereof, wherein:
the wavy line indicates the covalent attachment site to the linker;
the dotted lines indicate the optional presence of a double bond between C1 and C2 or C2 and C3;
R² is independently selected from H, OH, =O, =CH₂, CN, R, OR, =CH-R^{D}, =C(R^{D})₂, O-SO₂-R, CO₂R and COR, and optionally further selected from halo or dihalo, wherein R^{D} is independently selected from R, CO₂R, COR, CHO, CO₂H, and halo;
R⁶ and R⁹ are independently selected from H, R, OH, OR, SH, SR, NH₂, NHR, NRR', NO₂, Me₃Sn and halo;
R⁷ is independently selected from H, R, OH, OR, SH, SR, NH₂, NHR, NRR', NO₂, Me₃Sn and halo;
Q is independently selected from O, S and NH;
R¹¹ is either H, or R or, where Q is O, SO₃M, where M is a metal cation;
R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
R¹², R¹⁶, R¹⁹ and R¹⁷ are as defined for R², R⁶, R⁹ and R⁷ respectively;
R" is a C₃₋₁₂ alkylene group, which chain may be interrupted by one or more heteroatoms, e.g. O, S, N(H), NMe and/or aromatic rings, e.g. benzene or pyridine, which rings are optionally substituted; and
X and X' are independently selected from O, S and N(H).

In some embodiments, R⁹ and R¹⁹ are H.

In some embodiments, R⁶ and R¹⁶ are H.

In some embodiments, R⁷ are R¹⁷ are both OR^{7A}, where R^{7A} is optionally substituted C₁₋₄ alkyl. In some embodiments, R^{7A} is Me. In some embodiments, R^{7A} is is CH₂Ph, where Ph is a phenyl group.

In some embodiments, X is O.

In some embodiments, R¹¹ is H.

In some embodiments, there is a double bond between C2 and C3 in each monomer unit.

In some embodiments, R² and R¹² are independently selected from H and R. In some embodiments, R² and R¹² are independently R. In some embodiments, R² and R¹² are independently optionally substituted C₅₋₂₀ aryl or C₅₋₇ aryl or C₈₋₁₀ aryl. In some embodiments, R² and R¹² are independently optionally substituted phenyl, thienyl, napthyl, pyridyl, quinolinyl, or isoquinolinyl. In some embodiments, R² and R¹² are independently selected from =O, =CH₂, =CH-R^{D}, and =C(R^{D})₂. In some embodiments, R² and R¹² are each =CH₂. In some embodiments, R² and R¹² are each H. In some embodiments, R² and R¹² are each =O. In some embodiments, R² and R¹² are each =CF₂. In some embodiments, R² and/or R¹² are independently =C(R^{D})₂. In some embodiments, R² and/or R¹² are independently =CH-R^{D}.

In some embodiments, when R² and/or R¹² is =CH-R^{D}, each group may independently have either configuration shown below: 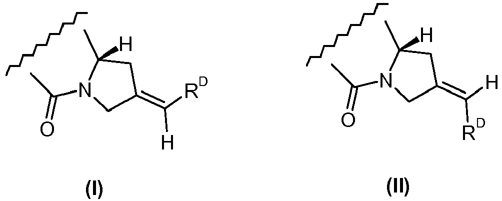 In some embodiments, a =CH-R^{D} is in configuration (I).

In some embodiments, R" is a C₃ alkylene group or a C₅ alkylene group.

In some embodiments, an exemplary PBD dimer component of an ADC has the structure of Formula A(I): 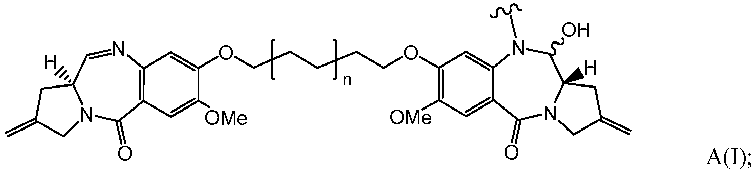 wherein n is 0 or 1.

In some embodiments, an exemplary PBD dimer component of an ADC has the structure of Formula A(II): 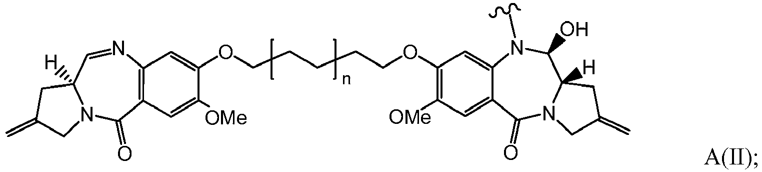 wherein n is 0 or 1.

In some embodiments, an exemplary PBD dimer component of an ADC has the structure of Formula A(III): 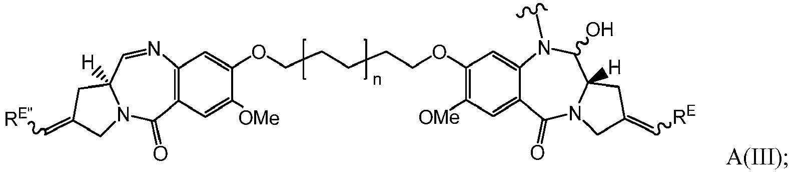
wherein R^{E} and R^{E"} are each independently selected from H or R^{D}, wherein R^{D} is defined as above; and
wherein n is 0 or 1.

In some embodiments, n is 0. In some embodiments, n is 1. In some embodiments, R^{E} and/or R^{E"} is H. In some embodiments, R^{E} and R^{E"} are H. In some embodiments, R^{E} and/or R^{E"} is R^{D}, wherein R^{D} is optionally substituted C₁₋₁₂ alkyl. In some embodiments, R^{E} and/or R^{E"} is R^{D}, wherein R^{D} is methyl.

In some embodiments, an exemplary PBD dimer component of an ADC has the structure of Formula A(IV): 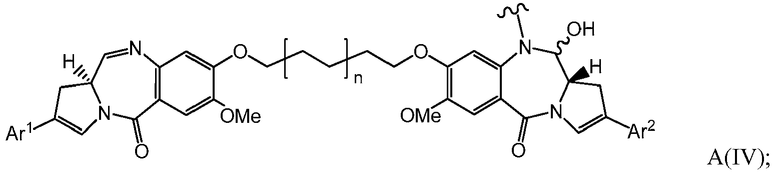
wherein Ar¹ and Ar² are each independently optionally substituted C₅₋₂₀ aryl; wherein Ar¹ and Ar² may be the same or different; and
wherein n is 0 or 1.

In some embodiments, an exemplary PBD dimer component of an ADC has the structure of Formula A(V): 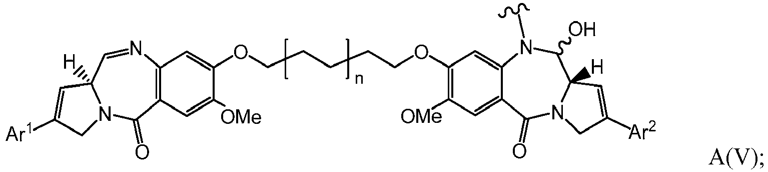
wherein Ar¹ and Ar² are each independently optionally substituted C₅₋₂₀ aryl; wherein Ar¹ and Ar² may be the same or different; and
wherein n is 0 or 1.

In some embodiments, Ar¹ and Ar² are each independently selected from optionally substituted phenyl, furanyl, thiophenyl and pyridyl. In some embodiments, Ar¹ and Ar² are each independently optionally substituted phenyl. In some embodiments, Ar¹ and Ar² are each independently optionally substituted thien-2-yl or thien-3-yl. In some embodiments, Ar¹ and Ar² are each independently optionally substituted quinolinyl or isoquinolinyl. The quinolinyl or isoquinolinyl group may be bound to the PBD core through any available ring position. For example, the quinolinyl may be quinolin-2-yl, quinolin-3-yl, quinolin-4yl, quinolin-5-yl, quinolin-6-yl, quinolin-7-yl and quinolin-8-yl. In some embodiments, the quinolinyl is selected from quinolin-3-yl and quinolin-6-yl. The isoquinolinyl may be isoquinolin-1-yl, isoquinolin-3-yl, isoquinolin-4yl, isoquinolin-5-yl, isoquinolin-6-yl, isoquinolin-7-yl and isoquinolin-8-yl. In some embodiments, the isoquinolinyl is selected from isoquinolin-3-yl and isoquinolin-6-yl.

Further exemplary PBD dimer components of ADCs are of Formula B: 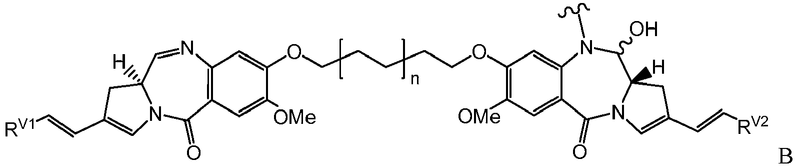 and salts and solvates thereof, wherein:
the wavy line indicates the covalent attachment site to the linker;
the wavy line connected to the OH indicates the S or R configuration;
R^{V1} and R^{V2} are independently selected from H, methyl, ethyl and phenyl (which phenyl may be optionally substituted with fluoro, particularly in the 4 position) and C₅₋₆ heterocyclyl; wherein R^{V1} and R^{V2} may be the same or different; and
n is 0 or 1.

In some embodiments, R^{V1} and R^{V2} are independently selected from H, phenyl, and 4-fluorophenyl.

In some embodiments, a linker may be attached at one of various sites of the PBD dimer drug moiety, including the N10 imine of the B ring, the C-2 endo/exo position of the C ring, or the tether unit linking the A rings (see structures C(I) and C(II) below).

Exemplary PBD dimer components of ADCs include Formulas C(I) and C(II):

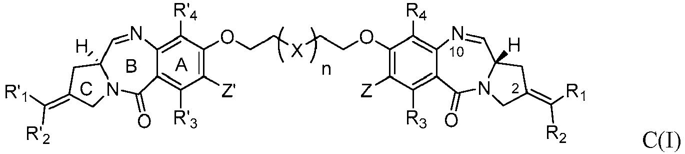

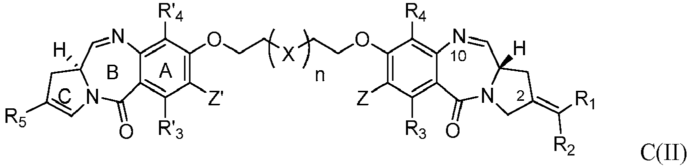

Formulas C(I) and C(II) are shown in their N10-C11 imine form. Exemplary PBD drug moieties also include the carbinolamine and protected carbinolamine forms as well, as shown in the table below:

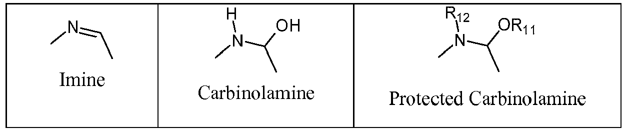

wherein:
X is CH₂ (n = 1 to 5), N, or O;
Z and Z' are independently selected from OR and NR₂, where R is a primary, secondary or tertiary alkyl chain containing 1 to 5 carbon atoms;
R₁, R'₁, R₂ and R'₂ are each independently selected from H, C₁-C₈ alkyl, C₂-C₈ alkenyl, C₂-C₈ alkynyl, C₅₋₂₀ aryl (including substituted aryls), C₅₋₂₀ heteroaryl groups, -NH₂, -NHMe, - OH, and -SH, where, in some embodiments, alkyl, alkenyl and alkynyl chains comprise up to 5 carbon atoms;
R₃ and R'₃ are independently selected from H, OR, NHR, and NR₂, where R is a primary, secondary or tertiary alkyl chain containing 1 to 5 carbon atoms;
R₄ and R'₄ are independently selected from H, Me, and OMe;
R₅ is selected from C₁-C₈ alkyl, C₂-C₈ alkenyl, C₂-C₈ alkynyl, C₅₋₂₀ aryl (including aryls substituted by halo, nitro, cyano, alkoxy, alkyl, heterocyclyl) and C₅₋₂₀ heteroaryl groups, where, in some embodiments, alkyl, alkenyl and alkynyl chains comprise up to 5 carbon atoms;
R₁₁ is H, C₁-C₈ alkyl, or a protecting group (such as acetyl, trifluoroacetyl, t-butoxycarbonyl (BOC), benzyloxycarbonyl (CBZ), 9-fluorenylmethylenoxycarbonyl (Fmoc), or a moiety comprising a self-immolating unit such as valine-citrulline-PAB);
R₁₂ is is H, C₁-C₈ alkyl, or a protecting group;
wherein a hydrogen of one of R₁, R'₁, R₂, R'₂, or R₁₂ or a hydrogen of the - OCH₂CH₂(X)ₙCH₂CH₂O- spacer between the A rings is replaced with a bond connected to the linker of the ADC.

Exemplary PBD dimer portions of ADC include, but are not limited to (the wavy line indicates the site of covalent attachment to the linker):

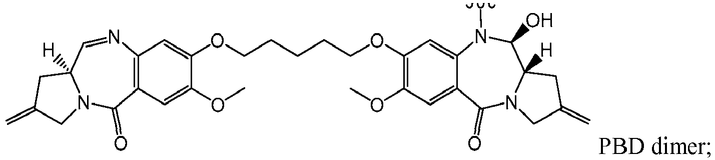

Exemplary embodiments of ADCs comprising PBD dimers have the following structures: 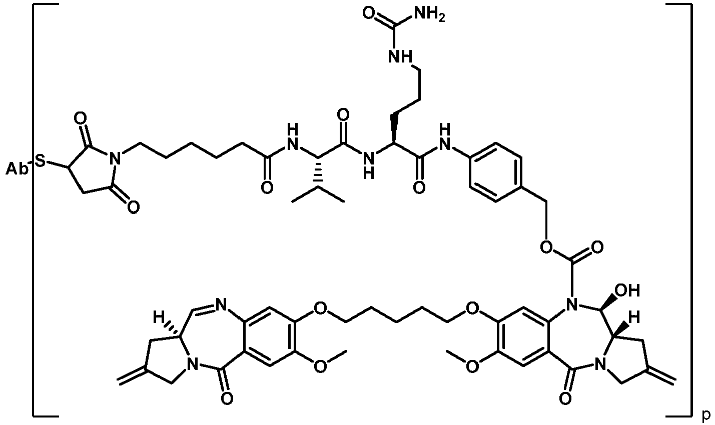 PBD dimer-val-cit-PAB-Ab; 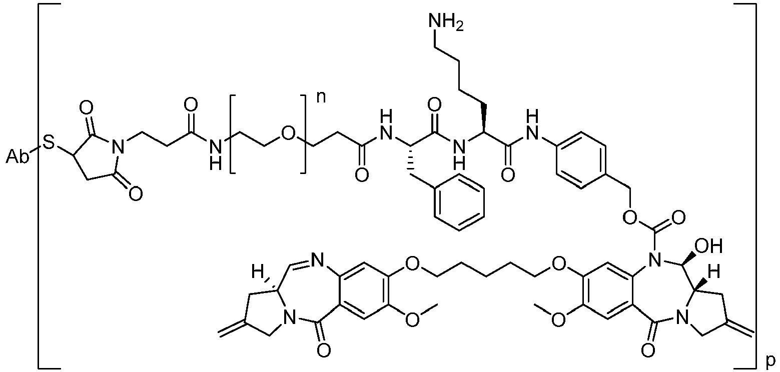 PBD dimer-Phe-homoLys-PAB-Ab, wherein:
n is 0 to 12. In some embodiments, n is 2 to 10. In some embodiments, n is 4 to 8. In some embodiments, n is selected from 4, 5, 6, 7, and 8.

The linkers of PBD dimer-val-cit-PAB-Ab and the PBD dimer-Phe-homoLys-PAB-Ab are protease cleavable.

PBD dimers and ADC comprising PBD dimers may be prepared according to methods known in the art. *See, e.g.*, WO 2009/016516; US 2009/304710; US 2010/047257; US 2009/036431; US 2011/0256157; WO 2011/130598.

### c) Drug Loading

Drug loading is represented by p, the average number of drug moieties per antibody in a molecule of Formula I. Drug loading may range from 1 to 20 drug moieties (D) per antibody. ADCs of Formula I include collections of antibodies conjugated with a range of drug moieties, from 1 to 20. The average number of drug moieties per antibody in preparations of ADC from conjugation reactions may be characterized by conventional means such as mass spectroscopy, ELISA assay, and HPLC. The quantitative distribution of ADC in terms of p may also be determined. In some instances, separation, purification, and characterization of homogeneous ADC where p is a certain value from ADC with other drug loadings may be achieved by means such as reverse phase HPLC or electrophoresis.

For some antibody-drug conjugates, p may be limited by the number of attachment sites on the antibody. For example, where the attachment is a cysteine thiol, as in certain exemplary embodiments above, an antibody may have only one or several cysteine thiol groups, or may have only one or several sufficiently reactive thiol groups through which a linker may be attached. In certain embodiments, higher drug loading, e.g. p >5, may cause aggregation, insolubility, toxicity, or loss of cellular permeability of certain antibody-drug conjugates. In certain embodiments, the average drug loading for an ADC ranges from 1 to about 8; from about 2 to about 6; or from about 3 to about 5. Indeed, it has been shown that for certain ADCs, the optimal ratio of drug moieties per antibody may be less than 8, and may be about 2 to about 5 (US 7498298).

In certain embodiments, fewer than the theoretical maximum of drug moieties are conjugated to an antibody during a conjugation reaction. An antibody may contain, for example, lysine residues that do not react with the drug-linker intermediate or linker reagent, as discussed below. Generally, antibodies do not contain many free and reactive cysteine thiol groups which may be linked to a drug moiety; indeed most cysteine thiol residues in antibodies exist as disulfide bridges. In certain embodiments, an antibody may be reduced with a reducing agent such as dithiothreitol (DTT) or tricarbonylethylphosphine (TCEP), under partial or total reducing conditions, to generate reactive cysteine thiol groups. In certain embodiments, an antibody is subjected to denaturing conditions to reveal reactive nucleophilic groups such as lysine or cysteine.

The loading (drug/antibody ratio) of an ADC may be controlled in different ways, and for example, by: (i) limiting the molar excess of drug-linker intermediate or linker reagent relative to antibody, (ii) limiting the conjugation reaction time or temperature, and (iii) partial or limiting reductive conditions for cysteine thiol modification.

It is to be understood that where more than one nucleophilic group reacts with a drug-linker intermediate or linker reagent, then the resulting product is a mixture of ADC compounds with a distribution of one or more drug moieties attached to an antibody. The average number of drugs per antibody may be calculated from the mixture by a dual ELISA antibody assay, which is specific for antibody and specific for the drug. Individual ADC molecules may be identified in the mixture by mass spectroscopy and separated by HPLC, e.g. hydrophobic interaction chromatography (*see, e.g.,* McDonagh et al (2006) Prot. Engr. Design & Selection 19(7):299-307; Hamblett et al (2004) Clin. Cancer Res. 10:7063-7070; Hamblett, K.J., et al. "Effect of drug loading on the pharmacology, pharmacokinetics, and toxicity of an anti-CD30 antibody-drug conjugate," Abstract No. 624, American Association for Cancer Research, 2004 Annual Meeting, March 27-31, 2004, Proceedings of the AACR, Volume 45, March 2004; Alley, S.C., et al. "Controlling the location of drug attachment in antibody-drug conjugates," Abstract No. 627, American Association for Cancer Research, 2004 Annual Meeting, March 27-31, 2004, Proceedings of the AACR, Volume 45, March 2004). In certain embodiments, a homogeneous ADC with a single loading value may be isolated from the conjugation mixture by electrophoresis or chromatography.

### d) Certain Methods of Preparing Immunoconjugates

An ADC of Formula I may be prepared by several routes employing organic chemistry reactions, conditions, and reagents known to those skilled in the art, including: (1) reaction of a nucleophilic group of an antibody with a bivalent linker reagent to form Ab-L via a covalent bond, followed by reaction with a drug moiety D; and (2) reaction of a nucleophilic group of a drug moiety with a bivalent linker reagent, to form D-L, via a covalent bond, followed by reaction with a nucleophilic group of an antibody. Exemplary methods for preparing an ADC of Formula I via the latter route are described in US 7498298.

Nucleophilic groups on antibodies include: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; and (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol) or tricarbonylethylphosphine (TCEP), such that the antibody is fully or partially reduced. Each cysteine bridge will thus form, theoretically, two reactive thiol nucleophiles. Additional nucleophilic groups can be introduced into antibodies through modification of lysine residues, e.g., by reacting lysine residues with 2-iminothiolane (Traut's reagent), resulting in conversion of an amine into a thiol. Reactive thiol groups may also be introduced into an antibody by introducing one, two, three, four, or more cysteine residues (e.g., by preparing variant antibodies comprising one or more non-native cysteine amino acid residues).

Antibody-drug conjugates of the invention may also be produced by reaction between an electrophilic group on an antibody, such as an aldehyde or ketone carbonyl group, with a nucleophilic group on a linker reagent or drug. Useful nucleophilic groups on a linker reagent include, hydrazide, oxime, amino, hydrazine, thiosemicarbazone, hydrazine carboxylate, and arylhydrazide. In one embodiment, an antibody is modified to introduce electrophilic moieties that are capable of reacting with nucleophilic substituents on the linker reagent or drug. In another embodiment, the sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In one embodiment, reaction of the carbohydrate portion of a glycosylated antibody with either galactose oxidase or sodium meta-periodate may yield carbonyl (aldehyde and ketone) groups in the antibody that can react with appropriate groups on the drug (Hermanson, Bioconjugate Techniques). In another embodiment, antibodies containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such an aldehyde can be reacted with a drug moiety or linker nucleophile.

Exemplary nucleophilic groups on a drug moiety include, amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

Exemplary cross-linker reagents that may be used to prepare ADC are described herein in the section titled "Exemplary Linkers." Methods of using such crosslinker reagents to link two moieties, including a proteinaceous moiety and a chemical moiety, are known in the art. In some embodiments, a fusion protein comprising an antibody and a cytotoxic agent may be made, e.g., by recombinant techniques or peptide synthesis. A recombinant DNA molecule may comprise regions encoding the antibody and cytotoxic portions of the conjugate either adjacent to one another or separated by a region encoding a linker peptide which does not destroy the desired properties of the conjugate.

In yet another embodiment, an antibody may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a drug or radionucleotide).

### E. Methods and Compositions for Diagnostics and Detection

Any of the antibodies provided herein may be useful for detecting the presence of CD79b in a biological sample. The term "detecting" as used herein encompasses quantitative or qualitative detection. A "biological sample" comprises, e.g., a cell or tissue (e.g., biopsy material, including cancerous or potentially cancerous lymph tissue, including tissue from subjects having or suspected of having a B cell disorder and/or a B cell proliferative disorder, including, but not limited to, lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma.

An anti-CD79b antibody for use in a method of diagnosis or detection is disclosed. In a further aspect, a method of detecting the presence of CD79b in a biological sample is provided. In certain embodiments, the method comprises contacting the biological sample with an anti-CD79b antibody as described herein under conditions permissive for binding of the anti-CD79b antibody to CD79b, and detecting whether a complex is formed between the anti-CD79b antibody and CD79b in the biological sample. Such method may be an *in vitro* or *in vivo* method. In one embodiment, an anti-CD79b antibody is used to select subjects eligible for therapy with an anti-CD79b antibody, e.g. where CD79b is a biomarker for selection of patients. In a further embodiment, the biological sample is a cell or tissue (e.g., cancerous or potentially cancerous lymph tissue, including tissue of subjects having or suspected of having a B cell disorder and/or a B cell proliferative disorder, including, but not limited to, lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma.

In a further embodiment, an anti-CD79b antibody is used in vivo to detect, e.g., by in vivo imaging, a CD79b-positive cancer in a subject, e.g., for the purposes of diagnosing, prognosing, or staging cancer, determining the appropriate course of therapy, or monitoring response of a cancer to therapy. One method known in the art for in vivo detection is immuno-positron emission tomography (immuno-PET), as described, e.g., in van Dongen et al., The Oncologist 12:1379-1389 (2007) and Verel et al., J. Nucl. Med. 44:1271-1281 (2003). In such embodiments, a method is provided for detecting a CD79b-positive cancer in a subject, the method comprising administering a labeled anti-CD79b antibody to a subject having or suspected of having a CD79b-positive cancer, and detecting the labeled anti-CD79b antibody in the subject, wherein detection of the labeled anti-CD79b antibody indicates a CD79b-positive cancer in the subject. In certain of such embodiments, the labeled anti-CD79b antibody comprises an anti-CD79b antibody conjugated to a positron emitter, such as ⁶⁸Ga, ¹⁸F, ^{6a}Cu, ⁸⁶Y, ⁷⁶Br, ⁸⁹Zr, and ¹²⁴I. In a particular embodiment, the positron emitter is ⁸⁹Zr.

A method of diagnosis or detection comprises contacting a first anti-CD79b antibody immobilized to a substrate with a biological sample to be tested for the presence of CD79b, exposing the substrate to a second anti-CD79b antibody, and detecting whether the second anti-CD79b is bound to a complex between the first anti-CD79b antibody and CD79b in the biological sample. A substrate may be any supportive medium, e.g., glass, metal, ceramic, polymeric beads, slides, chips, and other substrates. In certain embodiments, a biological sample comprises a cell or tissue (e.g., biopsy material, including cancerous or potentially cancerous lymph tissue, including tissue from subjects having or suspected of having a B cell disorder and/or a B cell proliferative disorder, including, but not limited to, lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma). In certain embodiments, the first or second anti-CD79b antibody is any of the antibodies described herein.

Exemplary disorders that may be diagnosed or detected include CD79b-positive cancers, such as CD79b-positive lymphoma, CD79b-positive non-Hogkins lymphoma (NHL; including, but not limited to CD79b-positive aggressive NHL, CD79b-positive relapsed aggressive NHL, CD79b-positive relapsed indolent NHL, CD79b-positive refractory NHL, and CD79b-positive refractory indolent NHL), CD79b-positive chronic lymphocytic leukemia (CLL), CD79b-positive small lymphocytic lymphoma, CD79b-positive leukemia, CD79b-positive hairy cell leukemia (HCL), CD79b-positive acute lymphocytic leukemia (ALL), CD79b-positive Burkitt's lymphoma, and CD79b-positive mantle cell lymphoma. In some embodiments, a CD79b-positive cancer is a cancer that receives an anti-CD79b immunohistochemistry (IHC) score greater than "0," which corresponds to very weak or no staining in >90% of tumor cells. In some embodiments, a CD79b-positive cancer expresses CD79b at a 1+, 2+ or 3+ level, wherein 1+ corresponds to weak staining in >50% of neoplastic cells, 2+ corresponds to moderate staining in >50% neoplastic cells, and 3+ corresponds to strong staining in >50% of neoplastic cells. In some embodiments, a CD79b-positive cancer is a cancer that expresses CD79b according to an in situ hybridization (ISH) assay. In some such embodiments, a scoring system similar to that used for IHC is used. In some embodiments, a CD79b-positive cancer is a cancer that expresses CD79b according to a reverse-transcriptase PCR (RT-PCR) assay that detects CD79b mRNA. In some embodiments, the RT-PCR is quantitative RT-PCR.

Labeled anti-CD79b antibodies are disclosed. Labels include, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like. In another embodiment, a label is a positron emitter. Positron emitters include but are not limited to ⁶⁸Ga, ¹⁸F, ⁶⁴Cu, ⁸⁶Y, ⁷⁶Br, ⁸⁹Zr, and ¹²⁴I. In a particular embodiment, a positron emitter is ⁸⁹Zr.

### F. Pharmaceutical Formulations

Pharmaceutical formulations of an anti-CD79b antibody or immunoconjugate as described herein are prepared by mixing such antibody or immunoconjugate having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody or immunoconjugate formulations are described in US Patent No. 6,267,958. Aqueous antibody or immunoconjugate formulations include those described in US Patent No. 6,171,586 and WO2006/044908, the latter formulations including a histidine-acetate buffer.

The formulation herein may also contain more than one active ingredient as necessary for the particular indication being treated, preferably those with complementary activities that do not adversely affect each other.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody or immunoconjugate, which matrices are in the form of shaped articles, *e.g.* films, or microcapsules.

The formulations to be used for *in vivo* administration are generally sterile. Sterility may be readily accomplished, e.g., by filtration through sterile filtration membranes.

### G. Therapeutic Methods and Compositions

Any of the anti-CD79b antibodies or immunoconjugates provided herein may be used in methods, e.g., therapeutic methods.

In one aspect, an anti-CD79b antibody or immunoconjugate provided herein is used in a method of inhibiting proliferation of a CD79b-positive cell, the method comprising exposing the cell to the anti-CD79b antibody or immunoconjugate under conditions permissive for binding of the anti-CD79b antibody or immunoconjugate to CD79b on the surface of the cell, thereby inhibiting the proliferation of the cell. In certain embodiments, the method is an *in vitro* or an *in vivo* method In some embodiments, the cell is a B cell. In some embodiments, the cell is a neoplastic B cell, such as a lymphoma cell or a leukemia cell.

Inhibition of cell proliferation in vitro may be assayed using the CellTiter-Glo™ Luminescent Cell Viability Assay, which is commercially available from Promega (Madison, WI). That assay determines the number of viable cells in culture based on quantitation of ATP present, which is an indication of metabolically active cells. *See* Crouch et al. (1993) J. Immunol. Meth. 160:81-88, US Pat. No. 6602677. The assay may be conducted in 96- or 384-well format, making it amenable to automated high-throughput screening (HTS). *See* Cree et al. (1995) AntiCancer Drugs 6:398-404. The assay procedure involves adding a single reagent (CellTiter-Glo^{®} Reagent) directly to cultured cells. This results in cell lysis and generation of a luminescent signal produced by a luciferase reaction. The luminescent signal is proportional to the amount of ATP present, which is directly proportional to the number of viable cells present in culture. Data can be recorded by luminometer or CCD camera imaging device. The luminescence output is expressed as relative light units (RLU).

In another aspect, an anti-CD79b antibody or immunoconjugate for use as a medicament is provided. In further aspects, an anti-CD79b antibody or immunoconjugate for use in a method of treatment is provided. In certain embodiments, an anti-CD79b antibody or immunoconjugate for use in treating CD79b-positive cancer is provided. In certain embodiments, the invention provides an anti-CD79b antibody or immunoconjugate for use in a method of treating an individual having a CD79b-positive cancer, the method comprising administering to the individual an effective amount of the anti-CD79b antibody or immunoconjugate. In one such embodiment, the method further comprises administering to the individual an effective amount of at least one additional therapeutic agent, e.g., as described below.

In a further aspect, the invention provides for the use of an anti-CD79b antibody or immunoconjugate in the manufacture or preparation of a medicament. In one embodiment, the medicament is for treatment of CD79b-positive cancer. In a further embodiment, the medicament is for use in a method of treating CD79b-positive cancer, the method comprising administering to an individual having CD79b-positive cancer an effective amount of the medicament. In one such embodiment, the method further comprises administering to the individual an effective amount of at least one additional therapeutic agent, e.g., as described below.

In a further aspect, the invention discloses a method for treating CD79b-positive cancer. In one embodiment, the method comprises administering to an individual having such CD79b-positive cancer an effective amount of an anti-CD79b antibody or immunoconjugate. In one such embodiment, the method further comprises administering to the individual an effective amount of at least one additional therapeutic agent, as described below.

A CD79b-positive cancer according to any of the above embodiments may be, e.g., lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma). In some embodiments, a CD79b-positive cancer is a cancer that receives an anti-CD79b immunohistochemistry (IHC) or in situ hybridization (ISH) score greater than "0," which corresponds to very weak or no staining in >90% of tumor cells. In another embodiment, a CD79b-positive cancer expresses CD79b at a 1+, 2+ or 3+ level, wherein 1+ corresponds to weak staining in >50% of neoplastic cells, 2+ corresponds to moderate staining in >50% neoplastic cells, and 3+ corresponds to strong staining in >50% of neoplastic cells. In some embodiments, a CD79b-positive cancer is a cancer that expresses CD79b according to a reverse-transcriptase PCR (RT-PCR) assay that detects CD79b mRNA. In some embodiments, the RT-PCR is quantitative RT-PCR.

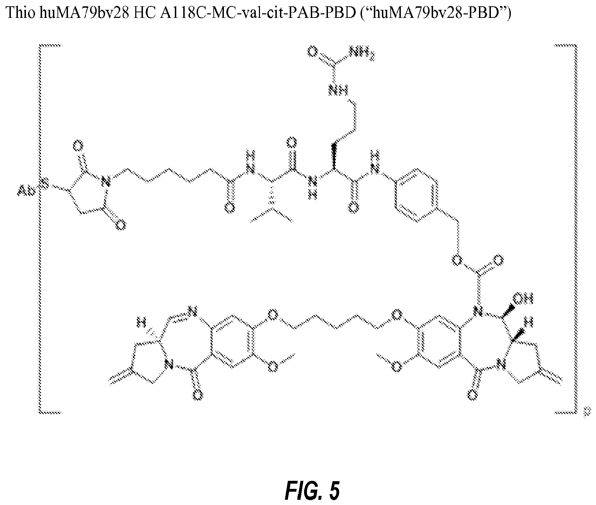

In some embodiments, immunoconjugates comprising a pyrrolobenzodiazepine cytotoxic moiety are particularly useful for treating diffuse large B-cell lymphomas, mantle cell lymphomas, and Burkitt's lymphoma as evidenced, for example, by the xenograft models shown in Examples B, C, D, E, and F. The immunoconjugate for use in treating diffuse large B-cell lymphomas, mantle cell lymphomas, and/or Burkitt's lymphoma, may, in some embodiments, comprise a PBD dimer having the structure: 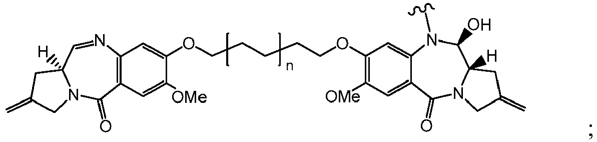 wherein n is 0 or 1. In some embodiments, the PBD dimer is covalently attached to the antibody through a protease cleavable linker, such as, for example, the immunoconjugate shown in Figure 5, which has a val-cit linker.

An "individual" according to any of the above embodiments may be a human.

In a further aspect, the invention provides pharmaceutical formulations comprising any of the anti-CD79b antibodies or immunoconjugate provided herein, e.g., for use in any of the above therapeutic methods. In one embodiment, a pharmaceutical formulation comprises any of the anti-CD79b antibodies or immunoconjugates provided herein and a pharmaceutically acceptable carrier. In another embodiment, a pharmaceutical formulation comprises any of the anti-CD79b antibodies or immunoconjugates provided herein and at least one additional therapeutic agent, e.g., as described below.

Antibodies or immunoconjugates of the invention can be used either alone or in combination with other agents in a therapy. For instance, an antibody or immunoconjugate of the invention may be co-administered with at least one additional therapeutic agent.

In some embodiments, an anti-CD79b immunoconjugate is administered in combination with an anti-CD22 antibody or immunoconjugate. A nonlimiting exemplary anti-CD22 antibody or immunoconjugate comprises the hypervariable regions of 10F4v3, such that the anti-CD22 antibody or immunoconjugate comprises (i) HVR H1 having the sequence of SEQ ID NO: 42, (ii) HVR H2 having the sequence of SEQ ID NO: 43, (iii) HVR H3 having the sequence of SEQ ID NO: 44, (iv) HVR L1 having the sequence of SEQ ID NO: 45, (v) HVR L2 having the sequence of SEQ ID NO: 46, and (vi) HVR L3 having the sequence of SEQ ID NO: 47. In some embodiments, an anti-CD22 antibody or immunoconjugate comprises the heavy chain variable region and light chain variable region of 10F4v3. In some such embodiments, the anti-CD22 antibody or immunoconjugate comprises a heavy chain variable region having the sequence of SEQ ID NO: 48 and a light chain variable region having the sequence of SEQ ID NO: 49. An anti-CD22 immunoconjugate comprises, in some embodiments, a cytotoxic agent selected from an auristatin, a nemorubicin derivative, and a pyrrolobenzodiazepine. In some embodiments, an anti-CD22 immunoconjugate comprises a cytotoxic agent selected from MMAE, PNU-159682, and a PBD dimer having the structure: 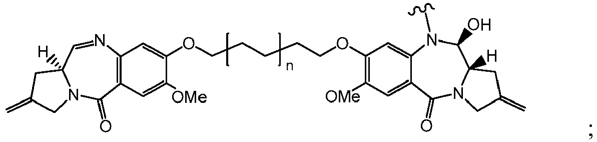 wherein n is 0 or 1. In some embodiments, an anti-CD22 immunoconjugate is selected from a Thio Hu anti-CD22 10F4v3 HC A118C-MC-val-cit-PAB-MMAE, a Thio Hu anti-CD22 10F4v3 HC S400C-MC-val-cit-PAB-MMAE, and a Thio Hu anti-CD22 10F4v3 LC V205C-MC-val-cit-PAB-MMAE immunoconjugate, which are described, e.g., in US 2008/0050310; a Thio Hu anti-CD22 10F4v3 HC A118C-MC-val-cit-PAB-PNU-159682, a Thio Hu anti-CD22 10F4v3 HC A118C-MC-acetal-PNU-159682, a Thio Hu anti-CD22 10F4v3 HC A118C-MC-val-cit-PAB-PBD, a Thio Hu anti-CD22 10F4v3 HC S400C-MC-val-cit-PAB-PNU-159682, a Thio Hu anti-CD22 10F4v3 HC S400C-MC-acetal-PNU-159682, a Thio Hu anti-CD22 10F4v3 HC S400C-MC-val-cit-PAB-PBD, a Thio Hu anti-CD22 10F4v3 LC V205C-MC-val-cit-PAB-PNU-159682, a Thio Hu anti-CD22 10F4v3 LC V205C-MC-acetal-PNU-159682, and a Thio Hu anti-CD22 10F4v3 LC V205C-MC-val-cit-PAB-PBD. The heavy chain and light chain sequences for Thio Hu anti-CD22 10F4v3 HC A118C are shown in SEQ ID NOs: 50 and 51, respectively. The heavy chain and light chain sequences for Thio Hu anti-CD22 10F4v3 HC S400C are shown in SEQ ID NOs: 52 and 51, respectively. The heavy chain and light chain sequences for Thio Hu anti-CD22 10F4v3 LC V205C are shown in SEQ ID NOs: 56 and 53, respectively. Apart from the specific antibody sequence, the structures of the anti-CD22 immunoconjugates are analogous to the structures of the anti-CD79b immunoconjugates described herein, and the anti-CD22 immunoconjugates described in US 2008/0050310. Nonlimiting exemplary immunoconjugates comprising PNU-159682 have the structures: Ab-MC-acetal-PNU-159682 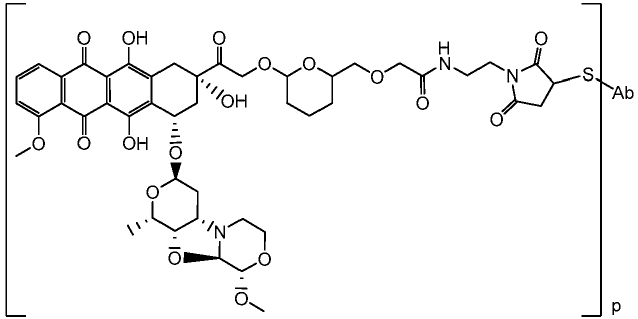 Ab-MC-val-cit-PAB-PNU-159682

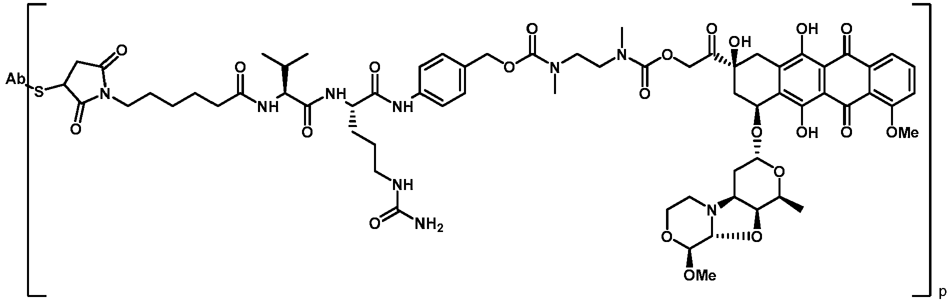

In some embodiments, an anti-CD22 immunoconjugate is administered in combination with an anti-CD20 antibody (either a naked antibody or an ADC). In some embodiments, the anti-CD20 antibody is rituximab (Rituxan^{®}) or 2H7 (Genentech, Inc., South San Francisco, CA). In some embodiments, an anti-CD22 immunoconjugate is administered in combination with an anti-VEGF antibody (e.g, bevicizumab, trade name Avastin^{®}).

Other therapeutic regimens may be combined with the administration of an anti-CD22 immunoconjugate, including, without limitation, radiation therapy and/or bone marrow and peripheral blood transplants, and/or a cytotoxic agent. In some embodiments, a cytotoxic agent is an agent or a combination of agents such as, for example, cyclophosphamide, hydroxydaunorubicin, adriamycin, doxorubincin, vincristine (Oncovin™), prednisolone, CHOP (combination of cyclophosphamide, doxorubicin, vincristine, and prednisolone), CVP (combination of cyclophosphamide, vincristine, and prednisolone), or immunotherapeutics such as anti-CD20 (e.g., rituximab, trade name Rituxan^{®}), anti-VEGF (e.g., bevicizumab, trade name Avastin^{®}), taxanes (such as paclitaxel and docetaxel) and anthracycline antibiotics.

Such combination therapies noted above encompass combined administration (where two or more therapeutic agents are included in the same or separate formulations), and separate administration, in which case, administration of the antibody or immunoconjugate of the invention can occur prior to, simultaneously, and/or following, administration of the additional therapeutic agent and/or adjuvant. Antibodies or immunoconjugates of the invention can also be used in combination with radiation therapy.

An antibody or immunoconjugate of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Antibodies or immunoconjugates of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody or immunoconjugate need not be, but is optionally formulated with one or more agents currently used to prevent or treat the disorder in question. The effective amount of such other agents depends on the amount of antibody or immunoconjugate present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an antibody or immunoconjugate of the invention (when used alone or in combination with one or more other additional therapeutic agents) will depend on the type of disease to be treated, the type of antibody or immunoconjugate, the severity and course of the disease, whether the antibody or immunoconjugate is administered for preventive or therapeutic purposes, previous therapy, the patient's clinical history and response to the antibody or immunoconjugate, and the discretion of the attending physician. The antibody or immunoconjugate is suitably administered to the patient at one time or over a series of treatments. Depending on the type and severity of the disease, about 1 µg/kg to 15 mg/kg (e.g. 0.1mg/kg-10mg/kg of antibody or immunoconjugate can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody or immunoconjugate would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (*e.g.* such that the patient receives from about two to about twenty, or *e.g.* about six doses of the antibody). An initial higher loading dose, followed by one or more lower doses may be administered. However, other dosage regimens may be useful. The progress of this therapy is easily monitored by conventional techniques and assays.

In some embodiments, a lower dose of a huMA79b (such as huMA79bv28) ADC comprising a pyrrolobenzodiazepine (PBD) dimer may be used to achieve the same efficacy as a higher dose of a huMA79b ADC comprising an MMAE moiety.

It is understood that any of the above formulations or therapeutic methods may be carried out using both an immunoconjugate of the invention and an anti-CD79b antibody.

### H. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the disorder and may have a sterile access port (for example the container may be an intravenous solution bag or a vial having a stopper pierceable by a hypodermic injection needle). At least one active agent in the composition is an antibody or immunoconjugate of the invention. The label or package insert indicates that the composition is used for treating the condition of choice. Moreover, the article of manufacture may comprise (a) a first container with a composition contained therein, wherein the composition comprises an antibody or immunoconjugate of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution or dextrose solution. It may further include other materials desirable from a commercial and user standpoint, including other buffers, diluents, filters, needles, and syringes.

### III. EXAMPLES

The following are examples of methods and compositions of the invention. It is understood that various other embodiments may be practiced, given the general description provided above.

### A. Production of Anti-CD79b Antibody Drug Conjugates

Anti-CD79b antibody MA79b and certain variants, including humanized huMA79b graft and humanized variants huMA79bv17, huMA79bv18, huMA79bv28, and huMA79bv32, are described, e.g., in US 8,088,378 B2. Table 2 shows the SEQ ID NOs corresponding to the heavy chain, light chain, and hypervariable regions (HVRs) for each antibody.

**Table 2: Sequences corresponding to MA79b and humanized variants**

| Antibody | HC variable region SEQ ID NO: | LC variable region SEQ ID NO: | HVR H1 SEQ ID NO: | HVR H2 SEQ ID NO: | HVR H3 SEQ ID NO: | HVR L1 SEQ ID NO: | HVR L2 SEQ ID NO: | HVR L3 SEQ ID NO: |
|---|---|---|---|---|---|---|---|---|
| MA79b | 3 | 4 | 15 | 16 | 17 | 18 | 19 | 20 |
| huMA79b graft | 5 | 6 | 15 | 16 | 17 | 18 | 19 | 20 |
| huMA79bv17 | 7 | 8 | 15 | 16 | 17 | 18 | 19 | 20 |
| huMA79bv18 | 9 | 10 | 15 | 16 | 23 | 18 | 19 | 20 |
| huMA79bv28 | 11 | 12 | 21 (identical to 15) | 22 (identical to 16) | 23 | 24 | 25 (identical to 19) | 26 (identical to 20) |
| huMA79bv32 | 13 | 14 | 15 | 16 | 23 | 35 | 19 | 20 |

The heavy chain framework regions for antibodies huMA79bv17, huMA79bv18, huMA79bv28, and huMA79bv32, HC FR1 to FR4, are shown in SEQ ID NOs: 27 to 30, respectively. The light chain framework regions for antibodies huMA79bv17, huMA79bv18, and huMA79bv28, LC FR1 to FR4, are shown in SEQ ID NOs: 31 to 34, respectively. The light chain framework regions for antibody huMA79bv32, LC FR1 to FR4, are shown in SEQ ID NOs: 31, 36, 33, and 34, respectively. The binding affinity of huMA79b was found to be about 0.4 nM using Scatchard analysis. *See, e.g.,* US 8,088,378 B2.

For larger scale antibody production, antibodies were produced in CHO cells. Vectors coding for VL and VH were transfected into CHO cells and IgG was purified from cell culture media by protein A affinity chromatography.

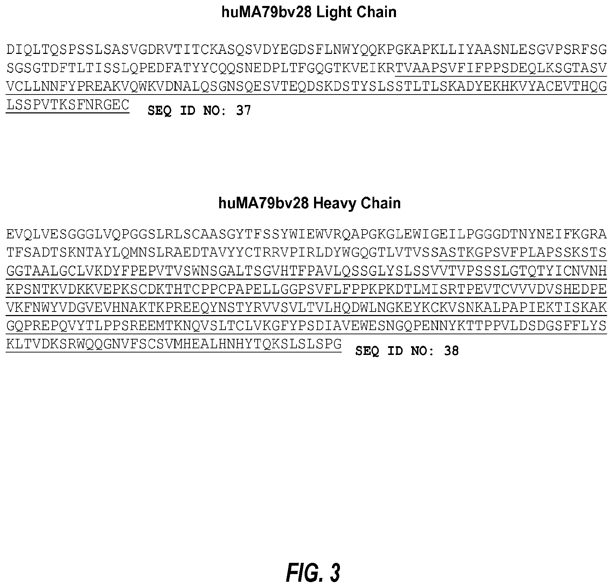

Anti-CD79b antibody-drug conjugates (ADCs) were produced by conjugating Thio huMA79bv28 HC A118C antibodies to certain drug moieties. Thio huMA79bv28 HC A118C is a huMA79bv28 antibody with an A118C mutation in the heavy chain that adds a conjugatable thiol group. *See, e.g.,* US 8,088,378 B2. The amino acid sequence of the heavy chain of Thio huMA79bv28 HC A118C is shown in SEQ ID NO: 39 (*see* Figure 4), and the amino acid sequence of the light chain of Thio huMA79bv28 HC A 118C is shown in SEQ ID NO: 37 (*see* Figure 3). The immunoconjugates were prepared as follows.

### Thio huMA79bv28 HC A118C-MC-val-cit-PAB-PBD ("huMA79bv28-PBD")

Prior to conjugation, the antibody was reduced with dithiothreitol (DTT) to remove blocking groups (e.g. cysteine) from the engineered cysteines of the thio-antibody. This process also reduces the interchain disulfide bonds of the antibody. The reduced antibody was purified to remove the released blocking groups and the interchain disulfides were reoxidized using dehydro-ascorbic acid (dhAA). The intact antibody was then combined with the drug-linker moiety MC-val-cit-PAB-PBD ("val-cit" may also be referred to herein as "vc") to allow conjugation of the drug-linker moiety to the engineered cysteine residues of the antibody. The conjugation reaction was quenched by adding excess N-acetyl-cysteine to react with any free linker-drug moiety, and the ADC was purified. The drug load (average number of drug moieties per antibody) for the ADC was in the range of about 1.6 to about 1.8, as indicated in the Examples below. HuMA79bv28-PBD has the structure shown in Figure 5 (p = drug load).

### Thio huMA79bv28 HC A118C-MC-val-cit-PAB-MMAE ("huMA79bv28-MMAE")

Prior to conjugation, the antibody was reduced with dithiothreitol (DTT) to remove blocking groups (e.g. cysteine) from the engineered cysteines of the thio-antibody. This process also reduces the interchain disulfide bonds of the antibody. The reduced antibody was purified to remove the released blocking groups and the interchain disulfides were reoxidized using dehydro-ascorbic acid (dhAA). The intact antibody was then combined with the drug-linker moiety MC-val-cit-PAB-MMAE ("val-cit" may also be referred to herein as "vc") to allow conjugation of the drug-linker moiety to the engineered cysteine residues of the antibody. The conjugation reaction was quenched by adding excess N-acetyl-cysteine to react with any free linker-drug moiety, and the ADC was purified. The drug load (average number of drug moieties per antibody) for the ADC was determined to be about 2, as indicated in the examples below. Thio huMA79bv28 HC A118C-MC-val-cit-PAB-MMAE is described, e.g., in US 8,088,378 B2.

### B. In vivo anti-tumor activity of humanized anti-CD79b antibody drug conjugates in a WSU-DLCL2 xenograft model

To test the efficacy of Thio huMA79bv28 HC A118C conjugate with PBD ("huMA79bv28-PBD"), the effects of the conjugated antibodies in a mouse xenograft model of WSU-DLCL2 tumors (diffuse large B-cell lymphoma cell line) was examined.

Female CB17 ICR SCID mice (11-12 weeks of age from Charles Rivers Laboratories; Hollister, CA) were each inoculated subcutaneously in the flank with 2 x 10⁷ WSU-DLCL2 cells (DSMZ, German Collection of Microorganisms and Cell Cultures, Braunschweig, Germany). When the xenograft tumors reached an average tumor volume of 150-250 mm³ (referred to as Day 0), the first and only dose of treatment was administered. Tumor volume was calculated based on two dimensions, measured using calipers, and was expressed in mm³ according to the formula: V= 0.5a x b², wherein a and b are the long and the short diameters of the tumor, respectively. To analyze the repeated measurement of tumor volumes from the same animals over time, a mixed modeling approach was used (*see, e.g.,* Pinheiro J, et al. nlme: linear and nonlinear mixed effects models. 2009; R package, version 3.1-96). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 8 mice were treated with a single intravenous (i.v.) dose of 0.5 or 2 mg ADC/kg of Thio huMA79bv28 HC A118C immunoconjugate or control antibody-drug conjugates (control ADCs). One group of mice received 12.86 µg/kg free PBD dimer, SG2057. *See* Hartley et al., Invest. New Drugs, 30: 950-958 (2012); Epub March 8, 2011. The control ADCs bind to a protein that is not expressed on the surface of WSU-DLCL2 cells. Tumors and body weights of mice were measured 1-2 times a week throughout the experiment. Mice were euthanized before tumor volumes reached 3000 mm³ or when tumors showed signs of impending ulceration. All animal protocols were approved by an Institutional Animal Care and Use Committee (IACUC).

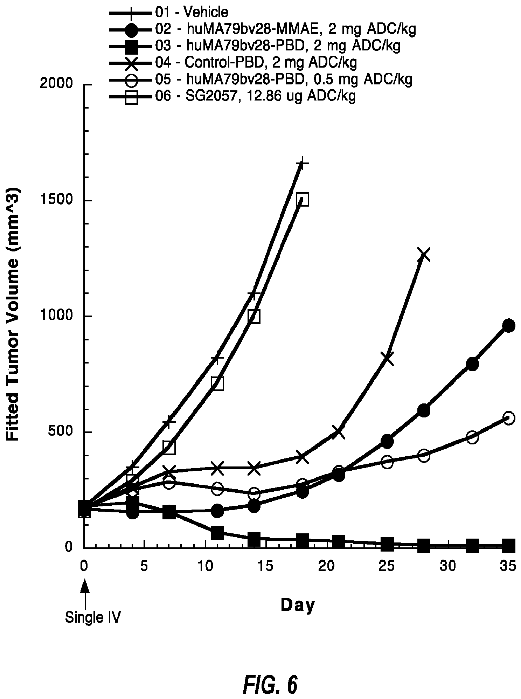

The results of that experiment are shown in Table 3 and Figure 6. Table 3 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 mm³), the drug dose for each group, the antibody dose for each group, and the drug load for each ADC administered.

**Table 3: Anti-CD79b ADC administration to mice with WSU-DLCL2 xenografts**

| **Antibody administered (Treatment)** | **TI** | **PR** | **CR** | **Drug Dose (µg//kg)** | **Ab Dose (mg/kg)** | **Drug Load (Drug /Ab)** |
|---|---|---|---|---|---|---|
| Vehicle* | 8/8 | 0 | 0 | n/a | n/a | n/a |
| huMA79bv28-PBD | 8/8 | 0 | 0 | 3.22 | 0.5 | 1.65 |
| huMA79bv28-PBD | 3/8 | 2 | 6 | 12.86 | 2 | 1.65 |
| Control ADC-MC-val-cit-PAB-PBD ("Control-PBD") | 8/8 | 0 | 0 | 14.03 | 2 | 1.8 |
| Thio huMA79bv28 HC A118C-MC-vc-PAB-MMAE ("huMA79bv28-MMAE") | 8/8 | 0 | 0 | 19.24 | 2 | 2.01 |
| SG2057 | 8/8 | 0 | 0 | 12.86 | n/a | n/a |

| | | | | | | |
|---|---|---|---|---|---|---|
| * Vehicle = 20 mM histidine acetate, pH 5.5, 240 mM sucrose, 0.02% PS20; n/a = not applicable. | | | | | | |

In a 35 day time course with drug conjugates and doses as shown in Table 3, thio huMA79bv28 ADCs conjugated through a protease cleavable linker with PBD ("huMA79bv28-PBD") showed inhibition of tumor growth in SCID mice with WSU-DLCL2 tumors compared to the vehicle and the control ADC ("Control-PBD"). *See* Figure 6.

Furthermore, when administered at 2 mg/kg, huMA79bv28-PBD better inhibited tumor growth than huMA79bv28 conjugated with the auristatin drug MMAE ("huMA79bv28-MMAE"). *See* Figure 6. The PBD free drug SG2057 did not show inhibition of tumor growth when given intravenously at 12.86 µg/kg, which is approximately equivalent to the drug dose of 2 mg/kg of huMA79bv28-PBD. As shown in Table 3, mice receiving 2 mg/kg huMA79bv28-PBD had six complete responses.

In this study, the percent body weight change was determined in each dosage group. The results indicated that administration of the huMA79bv28 ADCs did not cause a significant decrease in body weight during the study.

### C. In vivo anti-tumor activity of humanized anti-CD79b antibody drug conjugates in a Granta-519 xenograft model

To test the efficacy of Thio huMA79bv28 HC A118C conjugates with PBD ("huMA79bv28-PBD"), the effects of the conjugated antibodies in a mouse xenograft model of Granta-519 tumors (human mantle cell lymphoma cell line) was examined.

Female CB17 ICR SCID mice (10-11 weeks of age from Charles Rivers Laboratories; Hollister, CA) were each inoculated subcutaneously in the flank with 2 x 10⁷ Granta-519 cells (DSMZ, German Collection of Microorganisms and Cell Cultures, Braunschweig, Germany). When the xenograft tumors reached an average tumor volume of 150-250 mm³ (referred to as Day 0), the first and only dose of treatment was administered. Tumor volume was calculated based on two dimensions, measured using calipers, and was expressed in mm³ according to the formula: V= 0.5a x b², wherein a and b are the long and the short diameters of the tumor, respectively. To analyze the repeated measurement of tumor volumes from the same animals over time, a mixed modeling approach was used (*see, e.g.,* Pinheiro et al. 2009). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 8 mice were treated with a single intravenous (i.v.) dose of 0.25, 0.5, or 1 mg ADC/kg of huMA79bv28 immunoconjugate or control antibody-drug conjugates (control ADCs). The control ADCs bind to a protein that is not expressed on the surface of Grant-519 cells. One group of mice received 3.22 µg/kg free PBD dimer, SG2057. Tumors and body weights of mice were measured 1-2 times a week throughout the experiment. Mice were euthanized before tumor volumes reached 3000 mm³ or when tumors showed signs of impending ulceration. All animal protocols were approved by an Institutional Animal Care and Use Committee (IACUC).

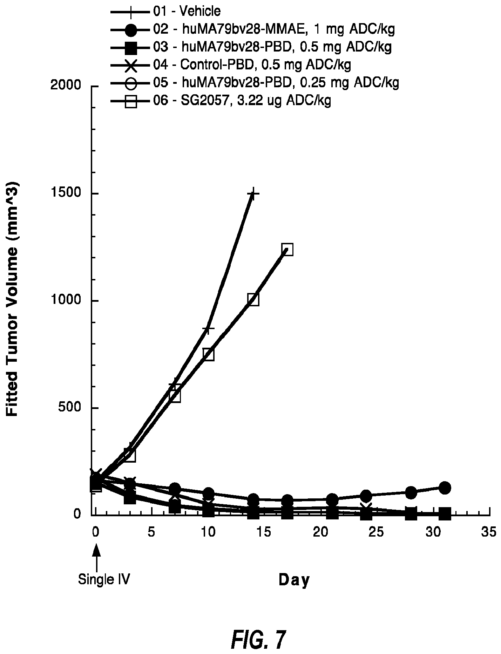

The results of that experiment are shown in Table 4 and Figure 7. Table 4 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 mm³), the drug dose for each group, the antibody dose for each group, and the drug load for each ADC administered.

**Table 4: Anti-CD79b ADC administration to mice with Grant-519 xenografts**

| **Antibody administered (Treatment)** | **TI** | **PR** | **CR** | **Drug Dose-(µg//kg)** | **Ab Dose (mg/kg)** | **Drug Load (Drug** /**Ab**) |
|---|---|---|---|---|---|---|
| Vehicle* | 8/8 | 0 | 0 | n/a | n/a | n/a |
| huMA79bv28-PBD | 0/8 | 0 | 8 | 3.22 | 0.5 | 1.65 |
| huMA79bv28-PBD | 0/8 | 0 | 8 | 1.61 | 0.25 | 1.65 |
| Control-PBD | 3/8 | 3 | 5 | 3.51 | 0.5 | 1.8 |
| huMA79bv28-MMAE | 6/8 | 1 | 2 | 9.62 | 1 | 2.01 |
| SG2057 | 8/8 | 0 | 0 | 3.22 | n/a | n/a |

| | | | | | | |
|---|---|---|---|---|---|---|
| * Vehicle = 20 mM histidine acetate, pH 5.5, 240 mM sucrose, 0.02% PS20; n/a = not applicable. | | | | | | |

In a 31 day time course with the ADCs and doses shown in Table 4, Thio huMA79bv28 conjugated through a protease cleavable linker with PBD ("huMA79bv28-PBD") showed inhibition of tumor growth in SCID mice with Granta-519 tumors compared to the vehicle. However, the control ADC conjugated to PBD ("Control-PBD") also showed anti-tumor activity, indicating that this tumor model is very sensitive to PBD. HuMA79bv28-PBD at a lower dose of 0.25 or 0.5 mg/kg was more effective at inhibiting tumor growth than huMA79bv28-MMAE at 1 mg/kg. The PBD free drug SG2057 did not show inhibition of tumor growth when given intravenously at 3.22 µg/kg, which is approximately equivalent to the drug dose of 0.5 mg/kg of huMA79bv28-PBD.

All 16 mice receiving huMA79bv28-PBD at 0.25 mg/kg or 0.5 mg/kg showed complete response, while only two mice that received 1 mg/kg huMA79bv28-MMAE showed complete response. See Table 4.

In this study, the percent body weight change was determined in each dosage group. The results indicated that administration of the huMA79bv28 ADCs did not cause a significant decrease in body weight during the study.

### D. In vivo anti-tumor activity of humanized anti-CD79b antibody drug conjugates in a SuDHL4-luc xenograft model

To test the efficacy of Thio huMA79bv28 HC A118C conjugates with PBD ("huMA79bv28-PBD"), the effects of the conjugated antibodies in a mouse xenograft model of SuDHL4-luc tumors (diffuse large B-cell lymphoma cell line) was examined.

Female CB17 ICR SCID mice (10-11 weeks of age from Charles Rivers Laboratories; Hollister, CA) were each inoculated subcutaneously in the flank with 2 x 10⁷ SuDHL4-luc cells (obtained from DSMZ, German Collection of Microorganisms and Cell Cultures, Braunschweig, Germany, and engineered at Genentech to stably express a luciferase gene). When the xenograft tumors reached an average tumor volume of 150-250 mm³ (referred to as Day 0), the first and only dose of treatment was administered. Tumor volume was calculated based on two dimensions, measured using calipers, and was expressed in mm³ according to the formula: V= 0.5a x b², wherein a and b are the long and the short diameters of the tumor, respectively. To analyze the repeated measurement of tumor volumes from the same animals over time, a mixed modeling approach was used (*see, e.g.,* Pinheiro et al. 2008). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 7 mice were treated with a single intravenous (i.v.) dose of 1 mg ADC/kg of huMA79bv28 immunoconjugate or control antibody-drug conjugates (control ADCs). The control ADCs bind to a protein that is not expressed on the surface of SuDHL4-luc cells. Tumors and body weights of mice were measured 1-2 times a week throughout the experiment. Mice were euthanized before tumor volumes reached 3000 mm³ or when tumors showed signs of impending ulceration. All animal protocols were approved by an Institutional Animal Care and Use Committee (IACUC).

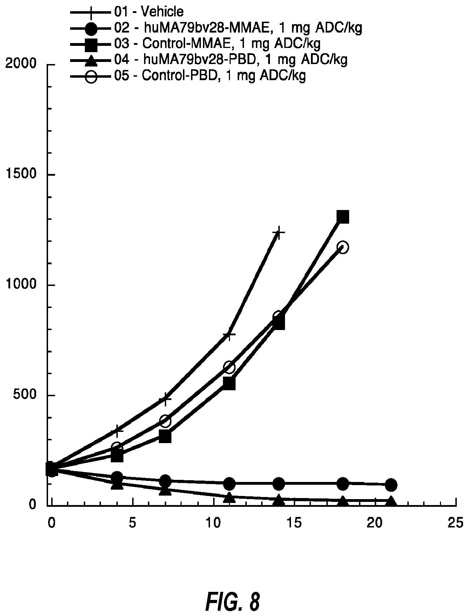

The results of that experiment are shown in Table 5 and Figure 8. Table 5 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 mm³), the drug dose for each group, the antibody dose for each group, and the drug load for each ADC administered.

**Table 5: Anti-CD79b ADC administration to mice with SuDHL4-luc xenografts**

| **Antibody administered (Treatment)** | **TI** | **PR** | **CR** | **Drug Dose (µg//kg)** | **Ab Dose (mg/kg)** | **Drug Load (Drug** /**Ab**) |
|---|---|---|---|---|---|---|
| Vehicle* | 7/7 | 0 | 0 | n/a | n/a | n/a |
| huMA79bv28-PBD | 6/7 | 4 | 3 | 6.43 | 1 | 1.65 |
| Control-PBD | 7/7 | 0 | 0 | 7.02 | 1 | 1.8 |
| huMA79bv28-MMAE | 5/7 | 1 | 3 | 9.62 | 1 | 2.01 |
| Control-ADC-A118C-MC-vc-PAB-MMAE ("Control MMAE") | 7/7 | 0 | 0 | 10.05 | 1 | 2.1 |

| | | | | | | |
|---|---|---|---|---|---|---|
| * Vehicle = 20 mM histidine acetate, pH 5.5, 240 mM sucrose, 0.02% PS20; n/a = not applicable. | | | | | | |

In a 21 day time course with drug conjugates and doses as shown in Table 5, Thio Hu anti-CD79b ADC conjugated through a protease cleavable linker with PBD ("huMA79bv28-PBD") showed inhibition of tumor growth in SCID mice with SuDHL4-luc tumors compared to the vehicle and the control ADC ("Control-PBD"). *See* Figure 8.

Furthermore, 1 mg/kg of huMA79bv28-PBD showed comparable anti-tumor activity to the humanized anti-CD79b thiomab conjugated with auristatin drug MMAE ("huMA79bv28-MMAE"). However, three mice from the group administered huMA79bv28-PBD showed a complete response, and another four mice showed a partial response, in contrast to huMA79bv28-MMAE, which produced three complete responses and one partial response. *See* Table 5.

In this study, the percent body weight change was determined in each dosage group. The results indicated that administration of the huMA79bv28 ADCs did not cause a significant decrease in body weight during the study.

### E. Dose escalation study of huMA79bv28-PBD in a SuDHL4-luc xenograft model

The efficacy of huMA79bv28-PBD at various dose levels in a mouse xenograft model of SuDHL4-luc tumors (diffuse large B-cell lymphoma cell line) was examined.

Female CB17 ICR SCID mice (12-13 weeks of age from Charles Rivers Laboratories; Hollister, CA) were each inoculated subcutaneously in the flank with 2 x 10⁷ SuDHL4-luc cells (obtained from DSMZ, German Collection of Microorganisms and Cell Cultures, Braunschweig, Germany, and engineered at Genentech to stably express a luciferase gene). When the xenograft tumors reached an average tumor volume of 150-300 mm³ (referred to as Day 0), the first and only dose of treatment was administered. Tumor volume was calculated based on two dimensions, measured using calipers, and was expressed in mm³ according to the formula: V= 0.5a x b², wherein a and b are the long and the short diameters of the tumor, respectively. To analyze the repeated measurement of tumor volumes from the same animals over time, a mixed modeling approach was used (*see, e.g.,* Pinheiro et al. 2008). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 8 mice were treated with a single intravenous (i.v.) dose of 0.2, 0.5, 1, or 2 mg ADC/kg of huMA79bv28-PBD or Control-PBD, which binds to a protein that is not expressed on the surface of SuDHL4-luc cells. Tumors and body weights of mice were measured 1-2 times a week throughout the experiment. Mice were euthanized before tumor volumes reached 3000 mm³ or when tumors showed signs of impending ulceration. All animal protocols were approved by an Institutional Animal Care and Use Committee (IACUC).

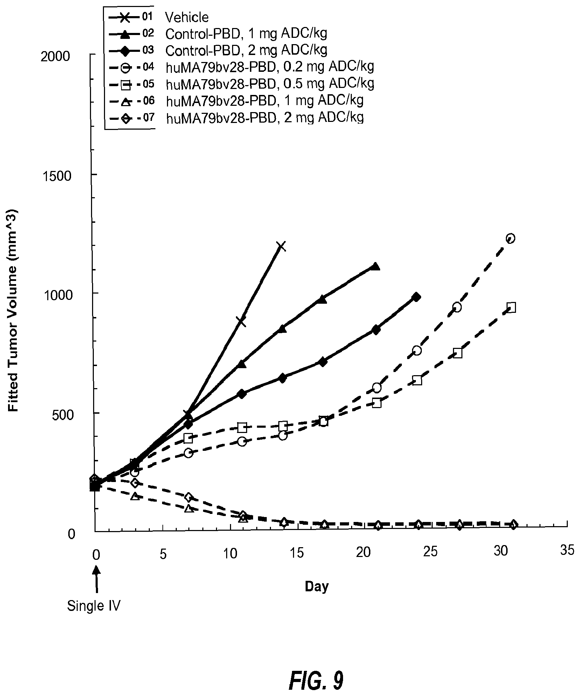

The results of that experiment are shown in Table 6 and Figure 9. Table 7 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 mm³), the drug dose for each group, the antibody dose for each group, and the drug load for each ADC administered.

**Table 6: Anti-CD79a ADC administration to mice with SuDHL4-luc xenografts**

| **Antibody administered (Treatment)** | **TI** | **PR** | **CR** | **Drug Dose (µg//kg)** | **Ab Dose (mg/kg)** | **Drug Load (Drug /Ab)** |
|---|---|---|---|---|---|---|
| Vehicle* | 8/8 | 0 | 0 | n/a | n/a | n/a |
| huMA79bv28-PBD | 8/8 | 1 | 0 | 1.36 | 0.2 | 1.74 |
| huMA79bv28-PBD | 8/8 | 0 | 0 | 3.39 | 0.5 | 1.74 |
| huMA79bv28-PBD | 3/8 | 2 | 5 | 6.78 | 1 | 1.74 |
| huMA79bv28-PBD | 3/8 | 2 | 6 | 13.56 | 2 | 1.74 |
| Control-PBD | 8/8 | 0 | 0 | 7.02 | 1 | 1.8 |
| Control-PBD | 8/8 | 0 | 0 | 14.03 | 2 | 1.8 |

| | | | | | | |
|---|---|---|---|---|---|---|
| * Vehicle = 20 mM histidine acetate, pH 5.5, 240 mM sucrose, 0.02% PS20; n/a = not applicable. | | | | | | |

In a 31 day time course with drug conjugates and doses as shown in Table 6, huMA79bv28-PBD showed dose-dependent inhibition of tumor growth in SCID mice with SuDHL4-luc tumors. When administered at 0.2 mg/kg or higher dose, huMA79bv28-PBD showed clear inhibitory activity compared to vehicle or the control ADC. *See* Figure 9. In addition, a single dose of 1 or 2 mg/kg huMA79bv28-PBD resulted in complete response in 5/8 and 6/8 treated animals, respectively. See Table 6.

In this study, the percent body weight change was determined in each dosage group. The results indicated that administration of huMA79bv28-PBD did not cause a significant decrease in body weight during the study.

### F. Dose escalation study of huMA79bv28-PBD in a BJAB-luc xenograft model

The efficacy of huMA79bv28-PBD at various dose levels in a mouse xenograft model of BJAB-luc tumors (Burkitt's lymphoma cell line) was examined.

Female CB17 ICR SCID mice (8-9 weeks of age from Charles Rivers Laboratories; Hollister, CA) were each inoculated subcutaneously in the flank with 2 x 10⁷ BJAB-luc cells (available, e.g., from Lonza, Basel, Switzerland, and engineered at Genentech to stably express a luciferase gene). When the xenograft tumors reached an average tumor volume of 150-300 mm³ (referred to as Day 0), the first and only dose of treatment was administered. Tumor volume was calculated based on two dimensions, measured using calipers, and was expressed in mm³ according to the formula: V= 0.5a x b², wherein a and b are the long and the short diameters of the tumor, respectively. To analyze the repeated measurement of tumor volumes from the same animals over time, a mixed modeling approach was used (*see, e.g.,* Pinheiro et al. 2008). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 9 mice were treated with a single intravenous (i.v.) dose of 0.05, 0.2, 0.5, or 1 mg ADC/kg of huMA79bv28-PBD or Control-PBD, which binds to a protein that is not expressed on the surface of BJAB-luc cells. Tumors and body weights of mice were measured 1-2 times a week throughout the experiment. Mice were euthanized before tumor volumes reached 3000 mm³ or when tumors showed signs of impending ulceration. All animal protocols were approved by an Institutional Animal Care and Use Committee (IACUC).

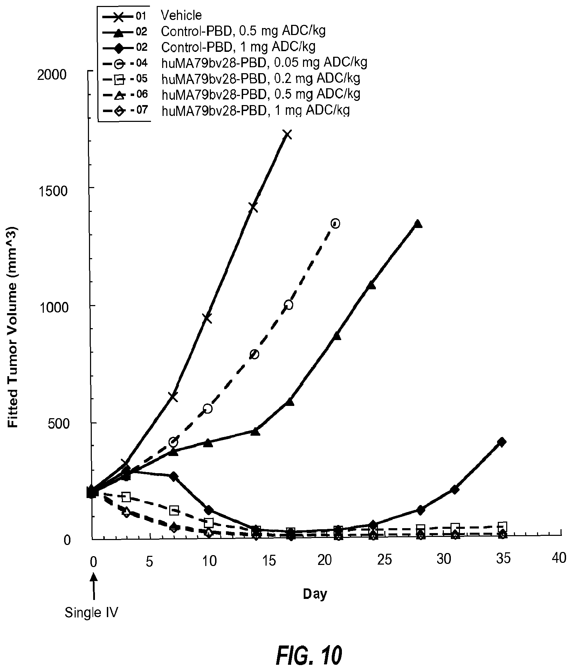

The results of that experiment are shown in Table 7 and Figure 10. Table 7 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 mm³), the drug dose for each group, the antibody dose for each group, and the drug load for each ADC administered.

**Table 7: Anti-CD79a ADC administration to mice with BJAB-luc xenografts**

| **Antibody administered (Treatment)** | **TI** | **PR** | **CR** | **Drug Dose (µg//kg)** | **Ab Dose (mg/kg)** | **Drug Load (Drug /Ab)** |
|---|---|---|---|---|---|---|
| Vehicle* | 9/9 | 0 | 0 | n/a | n/a | n/a |
| huMA79bv28-PBD | 9/9 | 0 | 0 | 0.34 | 0.05 | 1.74 |
| huMA79bv28-PBD | 4/9 | 3 | 5 | 1.36 | 0.2 | 1.74 |
| huMA79bv28-PBD | 0/9 | 0 | 9 | 3.39 | 0.5 | 1.74 |
| huMA79bv28-PBD | 0/9 | 0 | 9 | 6.78 | 1 | 1.74 |
| Control-PBD | 9/9 | 0 | 0 | 3.51 | 0.5 | 1.8 |
| Control-PBD | 9/9 | 4 | 4 | 7.02 | 1 | 1.8 |

| | | | | | | |
|---|---|---|---|---|---|---|
| * Vehicle = 20 mM histidine acetate, pH 5.5, 240 mM sucrose, 0.02% PS20; n/a = not applicable. | | | | | | |

In a 35 day time course with drug conjugates and doses as shown in Table 7, huMA79bv28-PBD showed dose-dependent inhibition of tumor growth in SCID mice with BJAB-luc tumors. When administered at 0.2 mg/kg or higher dose, huMA79bv28-PBD showed clear inhibitory activity compared to vehicle or the control ADC administered at 0.5 mg/kg. *See* Figure 10. In addition, a single dose of 0.5 or 1 mg/kg huMA79bv28-PBD resulted in complete tumor remission in all treated animals. Control-PBD at 1 mg/kg also showed substantial anti-tumor activity, indicating that this model is very sensitive to PBD.

In this study, the percent body weight change was determined in each dosage group. The results indicated that administration of huMA79bv28-PBD did not cause a significant decrease in body weight during the study.

### G. Dose escalation study of huMA79bv28-MMAE in a BJAB-luc xenograft model

The efficacy of huMA79bv28-MMAE at various dose levels in a mouse xenograft model of BJAB-luc tumors (Burkitt's lymphoma cell line) was examined.

Female CB17 ICR SCID mice (13-14 weeks of age from Charles Rivers Laboratories; Hollister, CA) were each inoculated subcutaneously in the flank with 2 x 10⁷ BJAB-luc cells (available, e.g., from Lonza, Basel, Switzerland, and engineered at Genentech to stably express a luciferase gene). When the xenograft tumors reached an average tumor volume of 150-300 mm³ (referred to as Day 0), the first and only dose of treatment was administered. Tumor volume was calculated based on two dimensions, measured using calipers, and was expressed in mm³ according to the formula: V= 0.5a x b², wherein a and b are the long and the short diameters of the tumor, respectively. To analyze the repeated measurement of tumor volumes from the same animals over time, a mixed modeling approach was used (*see, e.g.,* Pinheiro et al. 2008). This approach can address both repeated measurements and modest dropout rates due to non-treatment related removal of animals before the study end. Cubic regression splines were used to fit a non-linear profile to the time courses of log2 tumor volume at each dose level. These non-linear profiles were then related to dose within the mixed model.

Groups of 8 mice were treated with a single intravenous (i.v.) dose of 0.1, 0.5, 1, 2, OR 4 mg ADC/kg of huMA79bv28-MMAE, unconjugated huMA79bv28, or Control-MMAE, which binds to a protein that is not expressed on the surface of BJAB-luc cells. Tumors and body weights of mice were measured 1-2 times a week throughout the experiment. Mice were euthanized before tumor volumes reached 3000 mm³ or when tumors showed signs of impending ulceration. All animal protocols were approved by an Institutional Animal Care and Use Committee (IACUC).

The results of that experiment are shown in Table 8 and Figure 11. Table 8 shows each treatment group, the number of mice with observable tumors at the end of the study ("TI"), the number of mice showing a partial response ("PR"; where the tumor volume at any time after administration dropped below 50% of the tumor volume measured at day 0), the number of mice showing a complete response ("CR"; where the tumor volume at any time after administration dropped to 0 mm³), the drug dose for each group, the antibody dose for each group, and the drug load for each ADC administered.

**Table 8: Anti-CD79a ADC administration to mice with BJAB-luc xenografts**

| **Antibody administered (Treatment)** | **TI** | **PR** | **CR** | **Drug Dose (µg//kg)** | **Ab Dose (mg/kg)** | **Drug Load (Drug /Ab)** |
|---|---|---|---|---|---|---|
| Vehicle* | 8/8 | 0 | 0 | n/a | n/a | n/a |
| huMA79bv28-MMAE | 8/8 | 0 | 0 | 1.77 | 0.1 | 3.7 |
| huMA79bv28-MMAE | 8/8 | 0 | 0 | 8.86 | 0.5 | 3.7 |
| huMA79bv28-MMAE | 8/8 | 4 | 0 | 17.71 | 1 | 3.7 |
| huMA79bv28-MMAE | 1/8 | 1 | 7 | 35.42 | 2 | 3.7 |
| huMA79bv28-MMAE | 0/8 | 0 | 8 | 70.84 | 4 | 3.7 |
| Control-MMAE | 8/8 | 0 | 0 | 57.37 | 4 | 2.9 |
| huMA79bv28 | 8/8 | 0 | 0 | n/a | 4 | n/a |

| | | | | | | |
|---|---|---|---|---|---|---|
| * Vehicle = 20 mM histidine acetate, pH 5.5, 240 mM sucrose, 0.02% PS20; n/a = not applicable. | | | | | | |

In a 42 day time course with drug conjugates and doses as shown in Table 8, huMA79bv28-MMAE showed dose-dependent inhibition of tumor growth in SCID mice with BJAB-luc tumors. In contrast to huMA79bv28-PBD, which showed complete inhibition at 0.2 mg ADC/kg, huMA79bv28-MMAE did not show complete inhibition until a dose of 2 mg ADC/kg.

In this study, the percent body weight change was determined in each dosage group. The results indicated that administration of huMA79bv28-MMAE did not cause a significant decrease in body weight during the study.

**Table of Sequences**

| **SEQ ID NO** | **Description** | **Sequence** |
|---|---|---|
| 1 | humIII variable region sequence | |
| 2 | humκ1 variable region sequence | |
| 3 | MA79b heavy chain variable region | |
| 4 | MA79b light chain variable region | |
| 5 | huMA79b graft heavy chain variable region | |
| 6 | huMA79b graft light chain variable region | |
| 7 | huMA79bv17 heavy chain variable region | |
| 8 | huMA79bv17 light chain variable region | |
| 9 | huMA79bv18 heavy chain variable region | |
| 10 | huMA79bv18 light chain variable region | |
| 11 | huMA79bv28 heavy chain variable region | |
| 12 | huMA79bv28 light chain variable region | |
| 13 | huMA79bv32 heavy chain variable region | |
| 14 | huMA79bv32 light chain variable region | |
| 15 | MA79b HVR H1 | GYTFSSYWIE |
| 16 | MA79b HVR H2 | GEILPGGGDTNYNEIFKG |
| 17 | MA79b HVR H3 | TRRVPVYFDY |
| 18 | MA79b HVR L1 | KASQSVDYDGDSFLN |
| 19 | MA79b HVR L2 | AASNLES |
| 20 | MA79b HVR L3 | QQSNEDPLT |
| 21 | huMA79bv28 HVR H1 | GYTFSSYWIE |
| 22 | huMA79bv28 HVR H2 | GEILPGGGDTNYNEIFKG |
| 23 | huMA79bv28 HVR H3 | TRRVPIRLDY |
| 24 | huMA79bv28 HVR L1 | KASQSVDYEGDSFLN |
| 25 | huMA79bv28 HVR L2 | AASNLES |
| 26 | huMA79bv28 HVR L3 | QQSNEDPLT |
| 27 | huMA79bv28 heavy chain (HC) framework region (FR) 1 | EVQLVESGGGLVQPGGSLRLSCAAS |
| 28 | huMA79bv28 HC FR2 | WVRQAPGKGLEWI |
| 29 | huMA79bv28 HC FR3 | RATFSADTSKNTAYLQMNSLRAEDTAVYYC |
| 30 | huMA79bv28 HC FR4 | WGQGTLVTVSS |
| 31 | huMA79bv28 light chain (LC) FR1 | DIQLTQSPSSLSASVGDRVTIC |
| 32 | huMA79bv28 LC FR2 | WYQQKPGKAPKLLIY |
| 33 | huMA79bv28 LC FR3 | GVPSRFSGSGSGTDFTLTISSLQPEDFATYYC |
| 34 | huMA79bv28 LC FR4 | FGQGTKVEIKR |
| 35 | huMA79bv32 HVR L1 | KASQSVDYSGDSFLN |
| 36 | huMA79bv32 LC FR2 | WYQQKPGKAPKLLFY |
| 37 | huMA79bv28 light chain (Igκ) | |
| 38 | huMA79bv28 heavy chain (IgG1) | |
| 39 | huMA79bv28 | |
| | A118C cysteine engineered heavy chain (IgG1) | |
| 40 | Human CD79b precursor; Acc. No. NP_000617.1; signal sequence = amino acids 1 to 28 | |
| 41 | Human mature CD79b, without signal sequence; amino acids 29 to 229 | |
| 42 | Anti-CD22 10F4v3 HVR H1 | GYEFSRSWMN |
| 43 | Anti-CD22 10F4v3 HVR H2 | GRIYPGDGDTNYSGKFKG |
| 44 | Anti-CD22 10F4v3 HVR H3 | DGSSWDWYFDV |
| 45 | Anti-CD22 10F4v3 HVR L1 | RSSQSIVHSVGNTFLE |
| 46 | Anti-CD22 10F4v3 HVR L2 | KVSNRFS |
| 47 | Anti-CD22 10F4v3 HVR L3 | FQGSQFPYT |
| 48 | Anti-CD22 hu10F4v3 heavy chain variable region | |
| 49 | Anti-CD22 hu10F4v3 light chain variable region | |
| 50 | Anti-CD22 hu10F4v3 A118C cysteine engineered heavy chain (IgG1) | |
| 51 | Anti-CD22 | |
| | hu10F4v3 light chain (Igκ) | |
| 52 | Anti-CD22 hu10F4v3 S400C cysteine engineered heavy chain Fc region (IgG1) | |
| 53 | Anti-CD22 hu10F4v3 V205C cysteine engineered light chain (Igκ) | |
| 56 | Anti-CD22 hu10F4v3 heavy chain Fc region (IgG1) | |
| 54 | huMA79bv28 V205C cysteine engineered light chain (Igκ) | |
| 55 | huMA79bv28 S400C cysteine engineered heavy chain (IgG1) | |

## Claims

1. An immunoconjugate comprising an antibody that binds CD79b covalently attached to a cytotoxic agent, wherein the antibody that binds CD79b comprises (i) HVR-H1 comprising the amino acid sequence of SEQ ID NO: 21, (ii) HVR-H2 comprising the amino acid sequence of SEQ ID NO: 22, (iii) HVR-H3 comprising the amino acid sequence of SEQ ID NO: 23, (iv) HVR-L1 comprising an amino acid sequence selected from SEQ ID NOs: 18, 24, and 35, (v) HVR-L2 comprising the amino acid sequence of SEQ ID NO: 25, and (vi) HVR-L3 comprising the amino acid sequence of SEQ ID NO: 26; and wherein the cytotoxic agent is a pyrrolobenzodiazepine.

2. The immunoconjugate of claim 1, wherein the antibody comprises:
a) a VH sequence having at least 95% sequence identity to the amino acid sequence of SEQ ID NO: 11; or
b) a VL sequence having at least 95% sequence identity to the amino acid sequence of SEQ ID NO: 12; or
c) a VH sequence as in (a) and a VL sequence as in (b); or
d) a VH sequence having an amino acid sequence selected from SEQ ID NOs: 9, 11, and 13; or
e) a VL sequence having an amino acid sequence selected from SEQ ID NOs: 8, 10, 12, and 14; or
f) a VH sequence as in (d) and a VL sequence as in (e); or
g) a VH sequence having the amino acid sequence of SEQ ID NO: 11 and a VL sequence having the amino acid sequence of SEQ ID NO: 12.

3. The immunoconjugate of claim 1 or 2, wherein the antibody is an IgG1, IgG2a or IgG2b antibody.

4. An immunoconjugate of any one of claims 1 to 3, wherein the immunoconjugate has the formula Ab-(L-D)p, wherein:
(a) Ab is the antibody;
(b) L is a linker;
(c) D is the cytotoxic agent; and
(d) p ranges from 1-8.

5. The immunoconjugate of claim 4, wherein D is a pyrrolobenzodiazepine of Formula A: 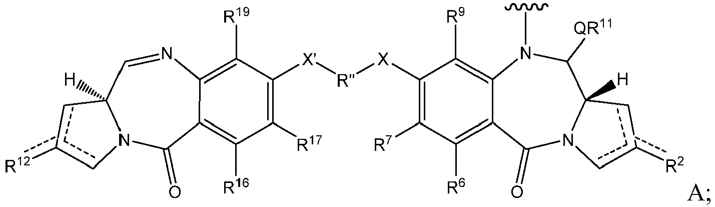 wherein the wavy line indicates the covalent attachement site to the linker;
the dotted lines indicate the optional presence of a double bond between C1 and C2 or C2 and C3;
R² is independently selected from H, OH, =O, =CH₂, CN, R, OR, =CH-R^{D}, =C(R^{D})₂, O-SO₂-R, CO₂R and COR, and optionally further selected from halo or dihalo, wherein R^{D} is independently selected from R, CO₂R, COR, CHO, CO₂H, and halo;
R⁶ and R⁹ are independently selected from H, R, OH, OR, SH, SR, NH₂, NHR, NRR', NO₂, Me₃Sn and halo;
R⁷ is independently selected from H, R, OH, OR, SH, SR, NH₂, NHR, NRR', NO₂, Me₃Sn and halo;
Q is independently selected from O, S and NH;
R¹¹ is either H, or R or, where Q is O, SO₃M, where M is a metal cation;
R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
R¹², R¹⁶, R¹⁹ and R¹⁷ are as defined for R², R⁶, R⁹ and R⁷ respectively;
R" is a C₃₋₁₂ alkylene group, which chain may be interrupted by one or more heteroatoms and/or aromatic rings that are optionally substituted; and
X and X' are independently selected from O, S and N(H).

6. The immunoconjugate of claim 5, wherein D has the structure: 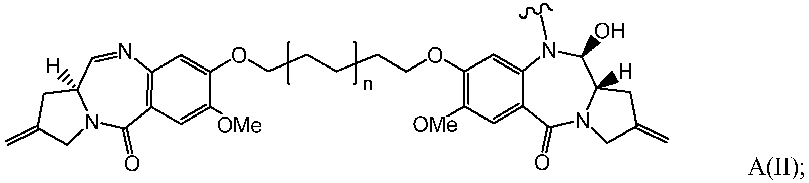 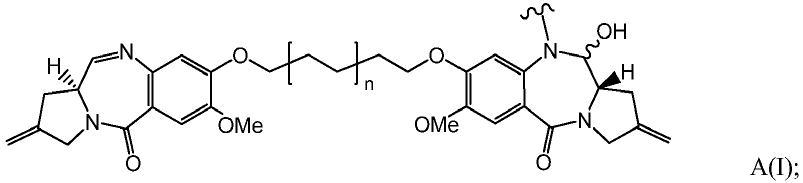 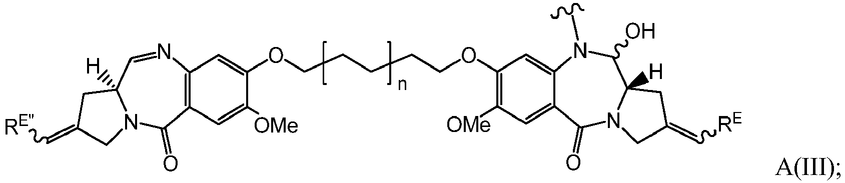 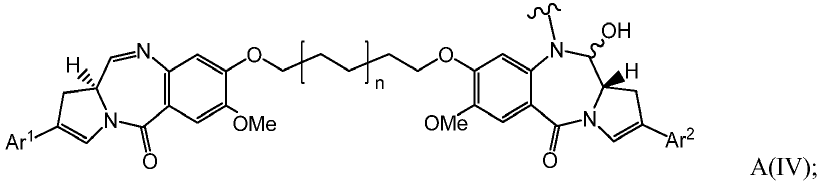 or 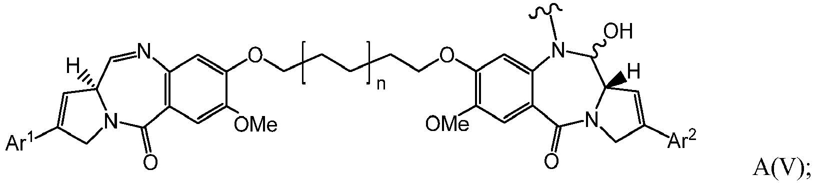 wherein R^{E} and R^{E"} are each independently selected from H or R^{D}, wherein R^{D} is independently selected from R, CO₂R, COR, CHO, CO₂H, and halo;
wherein Ar¹ and Ar² are each independently optionally substituted C₅₋₂₀ aryl; and wherein n is 0 or 1.

7. The immunoconjugate of claim 4, wherein D is a pyrrolobenzodiazepine of Formula B: 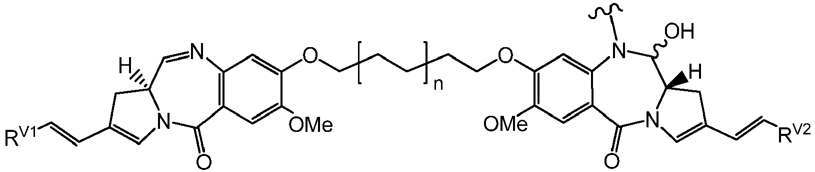 wherein the horizontal wavy line indicates the covalent attachement site to the linker;
R^{V1} and R^{V2} are independently selected from H, methyl, ethyl, phenyl, fluoro-substituted phenyl, and C₅₋₆ heterocyclyl; and
n is 0 or 1.

8. The immunoconjugate of any one of claims 4 to 7, wherein the linker is cleavable by a protease, wherein the linker is optionally a val-cit dipeptide.

9. The immunoconjugate of claim 6 having the formula:

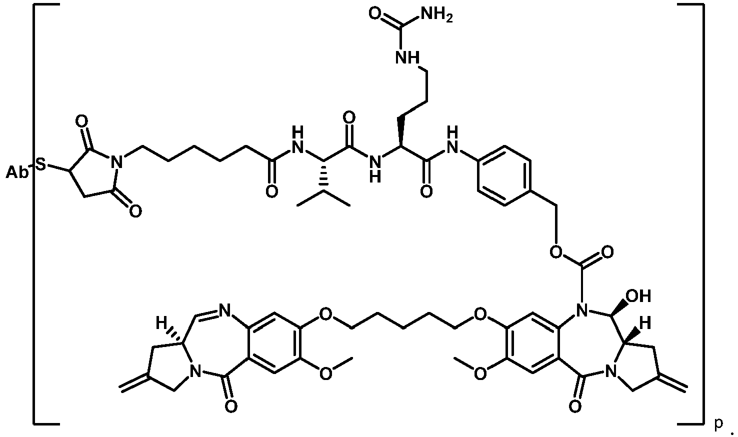

10. The immunoconjugate of claim 9, wherein the antibody comprises a heavy chain of SEQ ID NO: 39 and a light chain of SEQ ID NO: 37, and wherein p ranges from 1 to 3.

11. A pharmaceutical formulation comprising the immunoconjugate of any one of the preceding claims and a pharmaceutically acceptable carrier.

12. The immunoconjugate of any one of claims 1 to 10 for use in a method of treating an individual having a CD79b-positive cancer.

13. The immunoconjugate for use of claim 12, wherein the CD79b-positive cancer is selected from lymphoma, non-Hogkins lymphoma (NHL), aggressive NHL, relapsed aggressive NHL, relapsed indolent NHL, refractory NHL, refractory indolent NHL, chronic lymphocytic leukemia (CLL), small lymphocytic lymphoma, leukemia, hairy cell leukemia (HCL), acute lymphocytic leukemia (ALL), Burkitt's lymphoma, and mantle cell lymphoma.

## Patentansprüche

1. Immunkonjugat, umfassend einen Antikörper, der CD79b bindet, das kovalent an ein zytotoxisches Mittel gebunden ist, wobei der Antikörper, der CD79b bindet, (i) HVR-H1, umfassend die Aminosäuresequenz von Seq.-ID Nr. 21, (ii) HVR-H2, umfassend die Aminosäuresequenz von Seq.-ID Nr. 22, (iii) HVR-H3, umfassend die Aminosäuresequenz von Seq.-ID Nr. 23, (iv) HVR-L1, umfassend eine Aminosäuresequenz, ausgewählt aus den Seq.-ID Nrn. 18, 24 und 35, (v) HVR-L2, umfassend die Aminosäuresequenz von Seq.-ID Nr. 25, und (vi) HVR-L3, umfassend die Aminosäuresequenz von Seq.-ID Nr. 26, umfasst; und wobei das zytotoxische Mittel ein Pyrrolobenzodiazepin ist.

2. Immunkonjugat nach Anspruch 1, wobei der Antikörper Folgendes umfasst:
a) eine VH-Sequenz mit einer Sequenzidentität von zumindest 95 % mit der Aminosäuresequenz von Seq.-ID Nr. 11; oder
b) eine VL-Sequenz mit einer Sequenzidentität von zumindest 95 % mit der Aminosäuresequenz von Seq.-ID Nr. 12; oder
c) eine VH-Sequenz gemäß a) und eine VL-Sequenz gemäß b); oder
d) eine VH-Sequenz mit einer Aminosäuresequenz, die aus den Seq.-ID Nrn. 9, 11 und 13 ausgewählt ist; oder
e) eine VL-Sequenz mit einer Aminosäuresequenz, die aus den Seq.-ID Nrn. 8, 10, 12 oder 14 ausgewählt ist; oder
f) eine VH-Sequenz gemäß d) und eine VL-Sequenz gemäß e); oder
g) eine VH-Sequenz mit der Aminosäuresequenz von Seq.-ID Nr. 11 und eine VL-Sequenz mit der Aminosäuresequenz von Seq.-ID Nr. 12.

3. Immunkonjugat nach Anspruch 1 oder 2, wobei der Antikörper ein IgG1-, IgG2a- oder IgG2b-Antikörper ist.

4. Immunkonjugat nach einem der Ansprüche 1 bis 3, wobei das Immunkonjugat die Formel Ab-(L-D)p aufweist, worin:
(a) Ab der Antikörper ist;
(b) L ein Linker ist;
(c) D das zytotoxische Mittel ist; und
(d) p im Bereich von 1-8 liegt.

5. Immunkonjugat nach Anspruch 4, wobei D ein Pyrrolobenzodiazepin der Formel A ist: 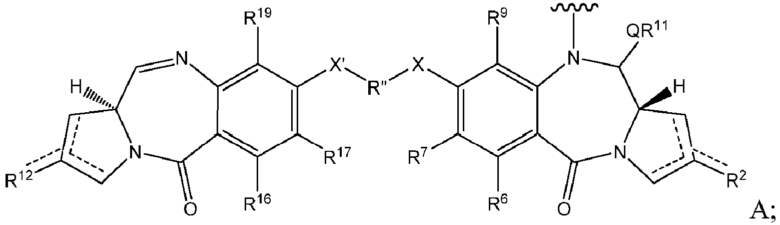 worin die Wellenlinie die Stelle der kovalenten Bindung an den Linker angibt;
die punktierten Linien das optionale Vorhandensein einer Doppelbindung zwischen C1 und C2 oder C2 und C3 angeben;
R² unabhängig aus R, CO₂R, COR, CHO, CO₂H und Halogen ausgewählt ist;
R⁶ und R⁹ jeweils unabhängig aus H, R, OH, OR, SH, SR, NH₂, NHR, NRR', NO₂, Me₃Sn und Halogen ausgewählt sind;
R⁷ unabhängig aus H, R, OH, OR; SH, SR, NH₂, NHR, NRR', NO₂, Me₃Sn und Halogen ausgewählt ist;
Q unabhängig aus O, S und NH ausgewählt ist;
R¹¹ entweder H oder R, oder wenn Q O ist, SO₃M ist, wobei M ein Metallkation ist; R und R' jeweils unabhängig aus gegebenenfalls substituiertem C₁₋₁₂-Alkyl, C₃₋₂₀-Heterocyclyl und C₅₋₂₀-Arylgruppen ausgewählt sind und R und R' in Bezug auf die Gruppe NRR' zusammen mit dem Stickstoffatom, an das sie gebunden sind, gegebenenfalls einen gegebenenfalls substituierten 4-, 5-, 6- oder 7-gliedrigen heterozyklischen Ring bilden;
R¹², R¹⁶, R¹⁹ und R¹⁷ wie für R², R⁶, R⁹ bzw. R⁷ definiert sind;
R" eine C₃₋₁₂-Alkylengruppe ist, deren Kette gegebenenfalls von einem oder mehreren Heteroatomen und/oder aromatischen Ringen unterbrochen ist, die gegebenenfalls substituiert sind; und
X und X' jeweils unabhängig aus O, S und N(H) ausgewählt sind.

6. Immunkonjugat nach Anspruch 5, wobei D die folgende Struktur aufweist: 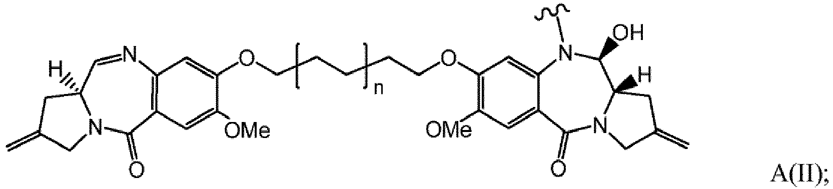 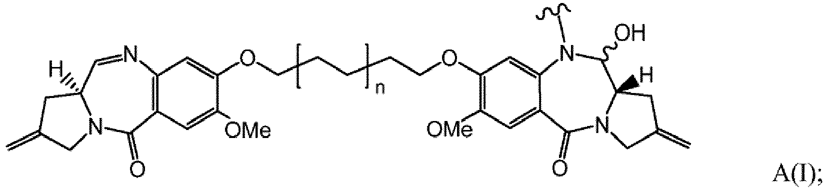 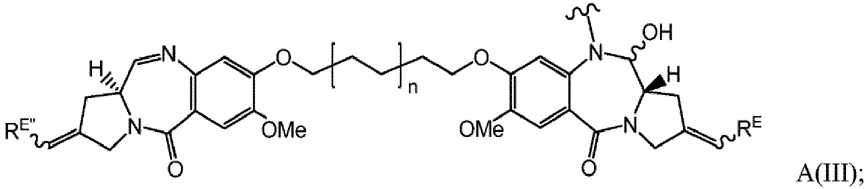 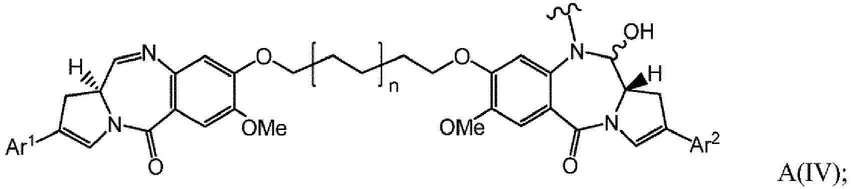 or 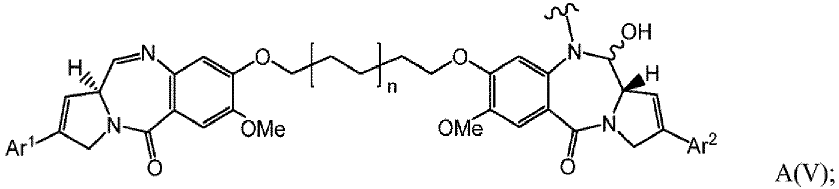
worin R^{E} und R^{E"} jeweils unabhängig aus H oder R^{D} ausgewählt sind, worin R^{D} jeweils unabhängig aus R, CO₂R, COR, CHO, CO₂H und Halogen ausgewählt ist; worin Ar¹ und Ar² jeweils unabhängig gegebenenfalls substituiertes C₅₋₂₀-Aryl sind; und
worin n = 0 oder 1 ist.

7. Immunkonjugat nach Anspruch 4, worin D ein Pyrrolobenzodiazepin der Formel B ist: 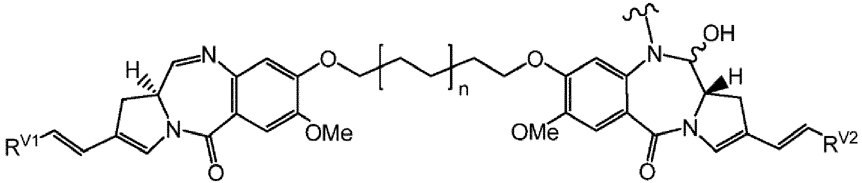 worin die horizontale Wellenlinie die Stelle der kovalenten Bindung mit dem Linker angibt;
R^{V1} und R^{V2} jeweils unabhängig aus H, Methyl, Ethyl, Phenyl, fluorsubstituiertem Phenyl und C₅₋₆-Heterocyclyl ausgewählt sind; und
n = 0 oder 1 ist.

8. Immunkonjugat nach einem der Ansprüche 4 bis 7, wobei der Linker mit einer Protease spaltbar ist, wobei der Linker gegebenenfalls ein Val-Cit-Dipeptid ist.

9. Immunkonjugat nach Anspruch 6 mit der folgenden Formel:

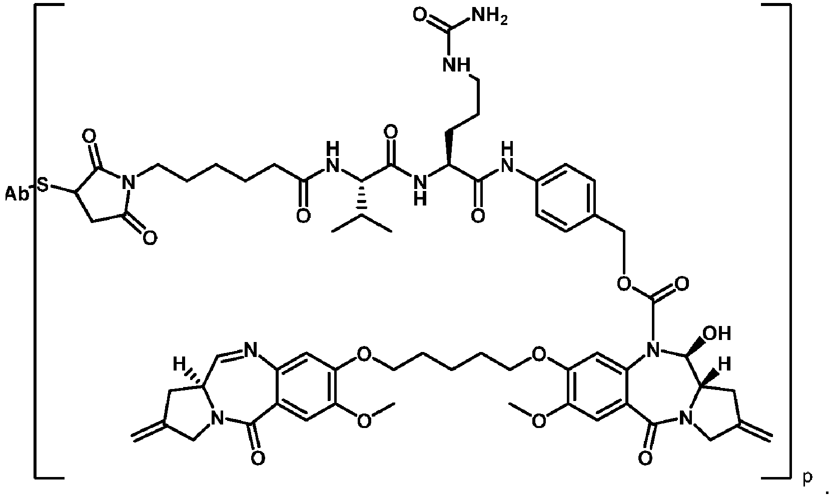

10. Immunkonjugat nach Anspruch 9, wobei der Antikörper eine Schwerkette von Seq.-ID Nr. 39 und eine Leichtkette von Seq.-ID Nr. 37 umfasst, und wobei p im Bereich von 1 bis 3 liegt.

11. Pharmazeutische Zusammensetzung, umfassend ein Immunkonjugat nach einem der vorangegangenen Ansprüche und einen pharmazeutisch annehmbaren Träger.

12. Immunkonjugat nach einem der Ansprüche 1 bis 10 zur Verwendung in einem Verfahren zur Behandlung eines Individuums mit einer CD79b-positiven Krebserkrankung.

13. Immunkonjugat zur Verwendung nach Anspruch 12, wobei die CD79b-positive Krebserkrankung aus einem Lymphom, Non-Hodgkins-Lymphom (NHL), aggressivem NHL, rezidivem aggressiven NHL, rezidivem indolenten NHL, refraktärem NHL, refraktärem indolenten NHL, chronischer lymphatischer Leukämie (CLL), kleiner lymphatischer Leukämie, Leukämie, Haarzellenleukämie (HCL), akuter lymphatischer Leukämie (ALL), Burkitt-Lymphom und Mantelzellenlymphom ausgewählt ist.

## Revendications

1. Immunoconjugué comprenant un anticorps qui se lie à CD79b attaché de façon covalente à un agent cytotoxique, dans lequel l'anticorps qui se lie à CD79b comprend (i) une HVR-H1 comprenant la séquence d'acides aminés de SEQ ID NO: 21, (ii) une HVR-H2 comprenant la séquence d'acides aminés de SEQ ID NO: 22, (iii) une HVR-H3 comprenant la séquence d'acides aminés de SEQ ID NO: 23, (iv) une HVR-L1 comprenant une séquence d'acides aminés sélectionnée parmi SEQ ID NO: 18, 24, et 35, (v) une HVR-L2 comprenant la séquence d'acides aminés de SEQ ID NO: 25, et (vi) une HVR-L3 comprenant la séquence d'acides aminés de SEQ ID NO: 26 ; et dans lequel l'agent cytotoxique est une pyrrolobenzodiazépine.

2. Immunoconjugué selon la revendication 1, dans lequel l'anticorps comprend :
a) une séquence VH présentant au moins 95 % d'identité de séquence avec la séquence d'acides aminés de SEQ ID NO: 11 ; ou
b) une séquence VL présentant au moins 95 % d'identité de séquence avec la séquence d'acides aminés de SEQ ID NO: 12 ; ou
c) une séquence VH telle qu'en (a) et une séquence VL telle qu'en (b) ; ou
d) une séquence VH possédant une séquence d'acides aminés sélectionnée parmi SEQ ID NO: 9, 11, et 13 ; ou
e) une séquence VL possédant une séquence d'acides aminés sélectionnée parmi SEQ ID NO: 8, 10, 12, et 14 ; ou
f) une séquence VH telle qu'en (d) et une séquence VL telle qu'en (e) ; ou
g) une séquence VH possédant la séquence d'acides aminés de SEQ ID NO: 11 et une séquence VL possédant la séquence d'acides aminés de SEQ ID NO: 12.

3. Immunoconjugué selon la revendication 1 ou 2, dans lequel l'anticorps est un anticorps IgG1, IgG2a ou IgG2b.

4. Immunoconjugué selon l'une quelconque des revendications 1 à 3, où l'immunoconjugué possède la formule Ab-(L-D)p, dans laquelle :
(a) Ab est l'anticorps ;
(b) L est un segment de liaison ;
(c) D est l'agent cytotoxique ; et
(d) p est compris entre 1 et 8.

5. Immunoconjugué selon la revendication 4, dans lequel D est une pyrrolobenzodiazépine de Formule A : 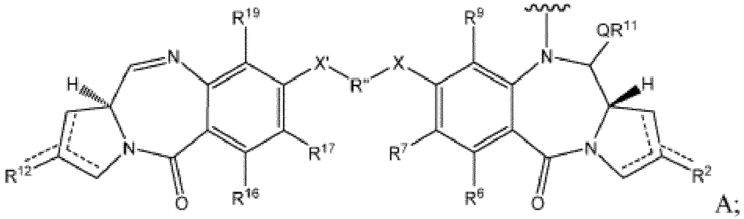 où la ligne ondulée indique le site d'attachement covalent au segment de liaison ;
les lignes en pointillés indiquent la présence facultative d'une double liaison entre C1 et C2 ou C2 et C3 ;
R est indépendamment sélectionné parmi H, OH, =O, =CH₂, CN, R, OR, =CH-R^{D}, =C(R^{D})₂, O-SO₂-R, CO₂R et COR, et facultativement en outre sélectionné parmi halo ou dihalo, où
R^{D} est indépendamment sélectionné parmi R, CO₂R, COR, CHO, CO₂H, et halo ;
R⁶ et R⁹ sont indépendamment sélectionnés parmi H, R, OH, OR, SH, SR, NH₂, NHR, NRR', NO₂, Me₃Sn et halo ;
R⁷ est indépendamment sélectionné parmi H, R, OH, OR, SH, SR, NH₂, NHR, NRR', NO₂, Me₃Sn et halo ;
Q est indépendamment sélectionné parmi O, S et NH ;
R¹¹ est soit H, ou R ou, où Q est O, SO₃M, où M est un cation métallique ;
R et R' sont chacun indépendamment sélectionnés parmi des groupes alkyle en C₁₋₁₂, hétérocyclyle en C₃₋₂₀ et aryle en C₅₋₂₀ facultativement substitués, et facultativement en relation avec le groupe NRR', R et R' ensemble avec l'atome d'azote auquel ils sont attachés forment un cycle hétérocyclique à 4, 5, 6 ou 7 chaînons facultativement substitué ;
R¹², R¹⁶, R¹⁹ et R¹⁷ sont tels que définis pour R², R⁶, R⁹ et R⁷ respectivement ;
R" est un groupe alkylène en C₃₋₁₂, dont la chaîne peut être interrompue par un ou plusieurs hétéroatomes et/ou cycles aromatiques qui sont facultativement substitués ; et
X et X' sont indépendamment sélectionnés parmi O, S et N(H).

6. Immunoconjugué selon la revendication 5, dans lequel D possède la structure : 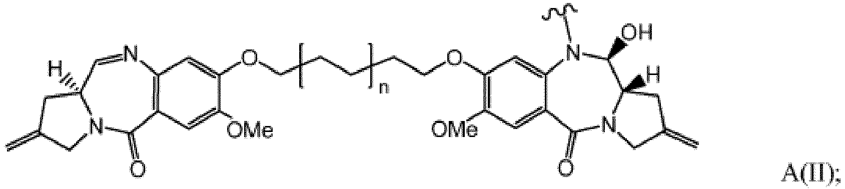 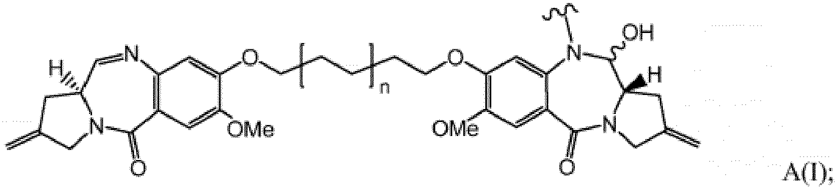 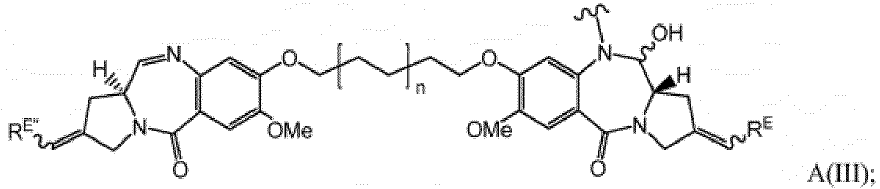 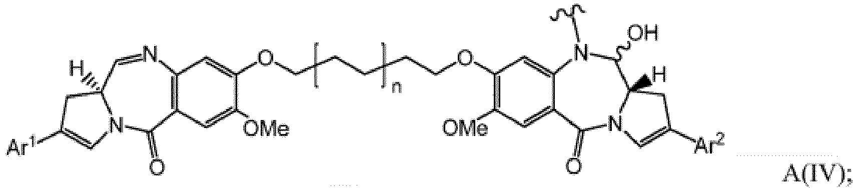 ou 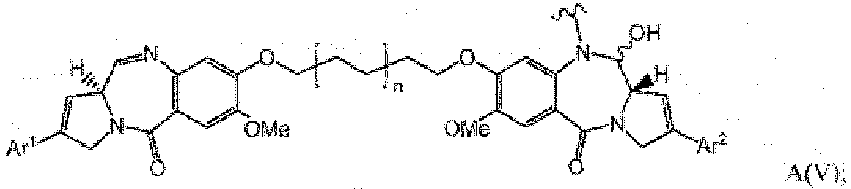
où R^{E} et R^{E"} sont chacun indépendamment sélectionnés parmi H ou R^{D}, où R^{D} est indépendamment sélectionné parmi R, CO₂R, COR, CHO, CO₂H, et halo ;
où Ar¹ et Ar² sont chacun indépendamment un groupe aryle en C₅₋₂₀ facultativement substitué ; et
où n est 0 ou 1.

7. Immunoconjugué selon la revendication 4, dans lequel D est une pyrrolobenzodiazépine de Formule B : 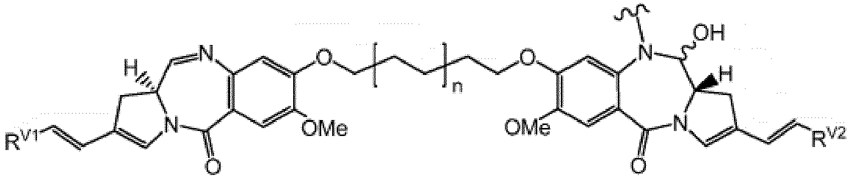 où la ligne ondulée horizontale indique le site d'attachement covalent au segment de liaison ;
R^{V1} et R^{V2} sont indépendamment sélectionnés parmi H, méthyle, éthyle, phényle, phényle fluoro-substitué, et hétérocyclyle en C₅₋₆ ; et
n est 0 ou 1.

8. Immunoconjugué selon l'une quelconque des revendications 4 à 7, dans lequel le segment de liaison peut être clivé par une protéase, où le segment de liaison est facultativement un dipeptide val-cit.

9. Immunoconjugué selon la revendication 6 possédant la formule :

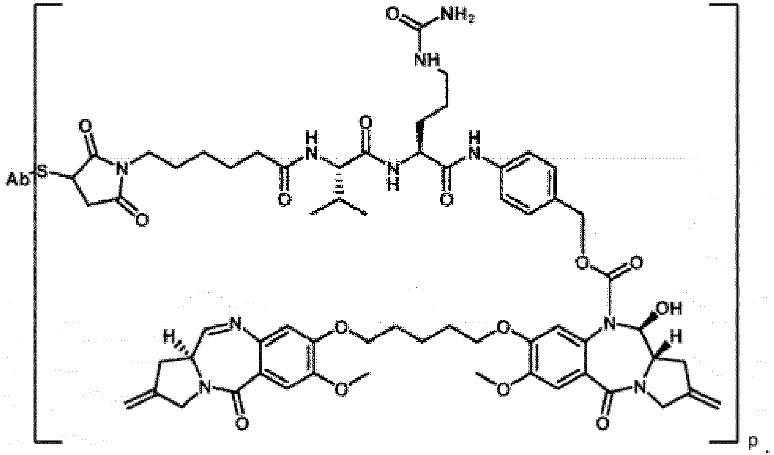

10. Immunoconjugué selon la revendication 9, dans lequel l'anticorps comprend une chaîne lourde de SEQ ID NO: 39 et une chaîne légère de SEQ ID NO: 37, et dans lequel p est compris entre 1 et 3.

11. Formulation pharmaceutique comprenant l'immunoconjugué selon l'une quelconque des revendications précédentes et un véhicule pharmaceutiquement acceptable.

12. Immunoconjugué selon l'une quelconque des revendications 1 à 10 destiné à être utilisé dans un procédé de traitement d'un individu atteint d'un cancer positif pour CD79b.

13. Immunoconjugué destiné à être utilisé selon la revendication 12, dans lequel le cancer positif pour CD79b est sélectionné parmi un lymphome, un lymphome non hodgkinien (LNH), un LNH agressif, un LNH agressif récidivant, un LNH indolent récidivant, un LNH réfractaire, un LNH indolent réfractaire, la leucémie lymphoïde chronique (LLC), un lymphome à petits lymphocytes, la leucémie, la leucémie à tricholeucocytes (LT), la leucémie aiguë lymphoblastique (LAL), un lymphome de Burkitt, et un lymphome à cellules du manteau.
